# EUROPEAN PATENT APPLICATION

(11) **EP 1 035 219 A1**
(43) Date of publication of application: **13.09.2000**
(21) Application number: 99870035.5
(22) Date of filing: 25.02.1999
(51) Int. Cl.: C12Q 1/68, A61K 31/70

(54) **Gastric helicobacter 16 S rDNA sequences from cattle and pigs and their use for detection and typing of Helicobacter strains**

(71) Applicant: UNIVERSITEIT GENT, B-9000 Gent (BE)
(72) Inventor: Ducatelle, Richard, 9700 Oudeaarde (BE); De Groote, Dominique, 9820 Merekbeke (BE); Haesebrouck, Freddy, 9820 Merelbeke (BE); Quint, Wilhelmus, 2631 Nootdorp (NL); Van Doorn, Leendert-Jan, 2986 Ridderkerk (NL)
(74) Representative: De Clercq, Ann

(57) **Abstract**

The present invention relates to new *Helicobacter* 16S rRNA or rDNA polynucleotide sequences useful for detection and typing of *Helicobacter* infected human and animal samples. The present invention relates more particularly to new *"Candidatus* Helicobacter bovis" and *"Candidatus* Helicobacter suis" sequences as defined in SEQ ID NO 1 and 2, sequence variants and fragments thereof. These new *Helicobacter* sequences allow the design of sequence specific probes and primers for detection and typing of the strains they are derived from. Diagnostic methods and kits employing such new bovine and porcine *Helicobacter* sequences are also disclosed.

### Annex to the application documents - subsquently filed sequences listing

SEQUENCE LISTING

<110> Universiteit Gent

<120> New gastric Helicobacter 16 S rDNA sequences from
      cattle and pigs and their use for detection and typing
      of Helicobacter strains

<130> EP.99.UG.01.DUCA

<140> 99870035.5
<141> 1999-02-25

<160> 25

<170> PatentIn Ver. 2.1

<210> 1
<211> 1335
<212> DNA
<213> Helicobacter sp.

<400> 1
acgctggcgg cgtgcctaat acatgcaagt cgaacgatga aatttctagc ttgctaggaa 60
tggattagtg gcgcacgggt gagtaacgca taggaatgtg ccctttagtc tgggatagcc 120
accggaaaca gtgattaata ctagatacgc cctaaggggg aaagaatttc gctaaaggat 180
cagcctatgt cctatcagct agttggtgag gtaaatgctt accaaggcta tgacgggtat 240
ccggcctgag agggtgaacg gacacaccgg aactgagaca cggtccggac tcctacggga 300
ggcagcagta gggaatattg ctcaatgggg gaaaccctga agcagcaacg ccgcgtggag 360
gatgaaggtt ctaggattgt aaactccttt tctgagagaa gataatgacg gtatctcagg 420
aataagcacc ggctaactcc gtgccagcag ccgcggtaat acggagggtg caagcgttac 480
tcggaatcac tgggcgtaaa gagcgtgtag gcggaatgat aagtcaggcg tgaaatcccg 540
tggcttaact gcggaactgc gtttgaaact atcattctgg agtgtgggag aggcaggtgg 600
aactcttggt gtaggggtaa aatccgtaga tatcaagaag aatactcatt gcgaaggcga 660
ccttgctgga catcactgac gctgatgcgc gaaagcgtgg ggagcaaaca ggattagata 720
ccctggtagt ccacgcccta aacgatggat gctaattgtc ggggggcttg tctcctcggt 780
aatgcagcta acgcattaag catcccgcct ggggagtacg atcgcaagat taaaactcaa 840
aggaatagac ggggacccgc acaagcggtg gagcatgtgg tttaattcga agatacgcga 900
agaaccttac ccaggcttga cattgatgga atctacccca aaaggtggag tgccagttta 960
ctggagcctg aaaacaggtg ctgcacggct gtcgtcagct cgtgtcgtga gatgttgggt 1020
taagtcccgc aacgagcgca accctcgtcc ttagttgcca tcagttcggc tgggcactct 1080
aaggagactg ccttcgtaag gaggaggaag gcgaggatga cgtcaagtca tcatggccct 1140
tatgtccggg gctacgcacg tgctacaatg ggatgtacaa agagatgcaa tgtcgtaaga 1200
tggagcaaaa ctcaaaaaca tctcccagtt cggattgtgg tctgcaactc gaccacatga 1260
agcaggaatc gctagtaatc gtgaatcagc catgtcacgg tgaatacgtt cccgggtctt 1320
gtactcacca atcac                                                  1335

<210> 2
<211> 1423
<212> DNA
<213> Helicobacter sp.

<400> 2
tgcaagtcga acgatgaagc ctagcttgct aggttgatta gtggcgcacg ggtgagtaat 60
gcatagatga catgcccttt agtttggaat agccactaga aatggtgatt aataccaaat 120
actaccttac gagggaaaga tttatcgcta aaggattggt ctatgtccta tcagcttgtt 180
ggtgaggtaa aggctcacca aggctatgac gggtatccgg cctgagaggg tgagcggaca 240

cactggaact gagacacggt ccagactcct acgggaggca gcagtaggga atattgctca 300
atgggggaaa ccctgaagca gcaacgccgc gtggaggatg aaggttttag gatcgtaaac 360
tccttttgtt agagaagata atgacggtat ctaacgaata agcaccggct aactccgtgc 420
cagcagccgc ggtaatacgg agggtgcaag cgttactcgg aatcactggg cgtaaagagt 480
gcgtaggcgg ggaggacaag tcaggtgtga aatcctatgg cttaaccata gaactgcatt 540
tgaaactatc cttctggagt gtgggggagg taggtggaat tcttggtgta gggggtaaaa 600
tccgtagaga tcaagaggaa tacccattgc gaaggcgacc tgctggaaca tcactgacgc 660
tgattgcacg aaagcgtggg gagcaaacag gnattagata ccctggtagt ccacgcccta 720
aacgatggat gctagttgtt gggaggcttt gtctttccag taatgcagst acgccttaag 780
catcccgcct ggggagtacg gtcgcaagat taaaactcaa aggaatagac ggggacccgc 840
acaagcggtg gagcatgtgg tttaattcga agttacacga agaaccttac ctaggcttga 900
cattgaagga attccctaga aataggggag tgtctagctt gctagaccct gaaaacaggt 960
gctgcacggc tgtcgtcagc tcgtgtcgtg agatgttggg ttaagtcccg caacgagcgc 1020
aacccttttt cttagttgct aacaggttat gctgcgcact ctaagaagac tgcctgcgta 1080
agcaggagga aggtgaggac gacgtcaagt catcatggcc cttacgccta gggctacaca 1140
cgtgctacaa tggggtgcac aaagagatgc aaagccgcga ggcagagcta atctataaaa 1200
cacctcctag ttcggattgc aggctgcaac tcgcctgcat gaagctggaa tcgctagtaa 1260
tcgcaaatca gctatgttgc ggtgaatacg ttcccgggtc ttgtactcac cgcccgtcac 1320
accatgggag ttgtgtttgc cttaagtcag gatgctaaag cagctactgc ccacggcaca 1380
cacagcgact ggggtgaagt cgtaacaagg taacccgggc ggc 1423

<210> 3
<211> 32
<212> DNA
<213> Helicobacter sp.

<400> 3
acgctggcgg cgtgcctaat acatgcaagt cg 32

<210> 4
<211> 32
<212> DNA
<213> Helicobacter sp.

<400> 4
ggtgagtaca agacccggga acgtattcac cg 32

<210> 5
<211> 24
<212> DNA
<213> Helicobacter sp.

<400> 5
gcagcaacgc cgcgtggagg atga 24

<210> 6
<211> 20
<212> DNA
<213> Helicobacter sp.

<400> 6
acgagctgac gacagccgtg 20

<210> 7
<211> 21
<212> DNA
<213> Helicobacter sp.

<400> 7
agagcgtgta ggcggaatga t                                                21

<210> 8
<211> 22
<212> DNA
<213> Helicobacter sp.

<400> 8
aactgcgttt gaaactatca tt                                               22

<210> 9
<211> 20
<212> DNA
<213> Helicobacter sp.

<400> 9
tgcaagtcga acgatgaagc                                                  20

<210> 10
<211> 27
<212> DNA
<213> Helicobacter sp.

<400> 10
aggctatgac gggtatccgg cctgaga                                          27

<210> 11
<211> 27
<212> DNA
<213> Helicobacter sp.

<400> 11
gccgcccggg ttaccttgtt acgactt                                          27

<210> 12
<211> 21
<212> DNA
<213> Helicobacter sp.

<400> 12
ttgggaggct ttgtctttcc a                                                21

<210> 13
<211> 21
<212> DNA
<213> Helicobacter sp.

<400> 13
gattagctct gcctcgcggc t                                                21

<210> 14
<211> 20
<212> DNA
<213> Helicobacter sp.

<400> 14
cgaggagaca agccccccga                                                20

<210> 15
<211> 1331
<212> DNA
<213> Helicobacter sp.

<400> 15
acgctggcgg cgtgcctaat acatgcaagt cgaacgatga aatttctagc ttgctaggaa 60
tggattagtg gcgcacgggt gagtaacgca taggaatgtg ccctttagtc tgggatagcc 120
accggaaaca gtgattaata ctagatacgc cctaaggggg aaagaatttc gctaaaggat 180
cagcctatgt cctatcagct agttggtgag gtaaatgctt accaaggcta tgacgggtat 240
ccggcctgag agggtgaacg gacacaccgg aactgagaca cggtccggac tcctacggga 300
ggcagcagta gggaatattg ctcaatgggg gaaaccctga agcagcaacg ccgcgtggag 360
gatgaaggtt ctaggattgt aaactccttt tctgagagaa gataatgacg gtatctcagg 420
aataagcacc ggctaactcc gtgccagcag ccgcggtaat acggagggtg caaacgttac 480
tcggaatcac tgggcgtaaa gagcgtgtag gcggaatgat aagtcaggcg tgaaatcccg 540
tggcttaacc gcggaactgc gtttgaaact atcattctgg agtgtgggag aggcaggtgg 600
aactcttggt gtaggggtaa aatccgtaga tatcaagaag aatactcatt gcgaaggcaa 660
cctgctggaa cattactgac gctgatgcgc gaaagcttgg ggagcaaaca ggattagata 720
ccctggtagt ccacgcccta aacgatggat gctaattgtc ggggggcttg tctcctcggt 780
aatgcagcta acgcattaag catcccgcct ggggagtacg atcgcaagat taaaactcaa 840
aggaatagac ggggacccgc acaagcggtg gagcatgtgg tttaattcga agatacgcga 900
agaaccttac ccaggcttga cattgatgga atctacccca aaaggtggag tgccagttta 960
ctggagcttg aaaacaggtg ctgcacggct gtcgtcagct cgtgtcgtga gatgttgggt 1020
taagtcccgc aacgagcgca accctcgtcc ttagttgcca tcagttcggc tgggcactct 1080
aaggagactg ccttcgtaag gaggaggaag gcgaggatga cgtcaagtca tcatggccct 1140
tacgcctggg gctacacacg tgctacaatg ggatgtacaa agagatgcaa tgtcgtaaga 1200
tggagcaaaa ctcaaaaaca tctcccagtt cggattgtgg tctgcaactc gaccacatga 1260
agcaggaatc gctagtaatc gtgaatcagc catgtcacgg tgaatacgtt cccgggtctt 1320
gtactcacca a                                                         1331

<210> 16
<211> 1299
<212> DNA
<213> Helicobacter sp.

<400> 16
acgctggcgg cgtgcctaat acatgcaagt cgaacgatga aatttctagc ttgctaggaa 60
tggattagtg gcgcacgggt gagtaatgca taggaatgtg ccctttagtc tgggatagcc 120
accggaaaca gtgattaata ctagatacgc cctaaggggg aaagaatttc gctaaaggat 180
cagcctatgt cctatcagct agttggtgag gtaaatgctt accaaggcta tgacgggtat 240
ccggcctgag agggtgaacg gacacaccgg aactgagaca cggtccggac tcctacggga 300
ggcagcagta gggaatattg ctcaatgggg gaaaccctga agcagcaacg ccgcgtggag 360
gatgaaggtt ctaggattgt aaactccttt tctgagagaa gataatgacg gtatctcagg 420
aataagcacc ggctaactcc gtgccagcag ccgcggtaat acggagggtg caagcgttac 480
tcggaatcac tgggcgtaaa gagcgtgtag gcggaatgat aagtcaggcg tgaaatcccg 540
tggcttaact gcggaactgc gtttgaaact atcattctgg agtgtgggag aggcaggtgg 600
aactcttggt gtaggggtaa aatccgtaga tatcaagaag aatactcatt gcgaaggcga 660
cctgctggaa catcactgac gctgatgcgc gaaagcgtgg ggagcaaaca ggattagata 720
ccctggtagt ccacgcccta aacgatggat gctaattgtc ggggggcttg tctcctcggt 780
aatgcagcta acgcattaag catcccgcct ggggagtacg atcgcaagat taaaactcaa 840
aggaatagac ggggacccgc acaagcggtg gagcatgtgg tttaattcga agatacgcga 900
agaaccttac ccaggcttga cattgatgga atctacccca aaaggtggag tgccagttta 960
ctggagcctg aaaacaggtg ctgcacggct gtcgtcagct cgtgtcgtga gatgttgggt 1020
taagtcccgc aacgagcgca accctcgtcc ttagttgcca tcagttcggc tgggcactct 1080

aaggagactg ccttcgtaag gaggaggaag gcgaggatga cgtcaagtca tcatggccct 1140
tacgcctggg gctacacacg tgctacaatg ggatgtacaa agagatgcaa tgtcgtaaga 1200
tggagcaaaa ctcaaaaaca tctcccagtt cggattgtgg tctgcaactc gaccacatga 1260
agcaggaatc gctagtaatc gtgaatcagc catgtcacg 1299

<210> 17
<211> 1267
<212> DNA
<213> Helicobacter sp.

<400> 17
aacgatgaaa tttctagctt gctagaagtg gattagtggc gcacgggtga gtaatgcata 60
ggaatgtgcc ctttagtctg ggatagccac cggaaacagt gattaatact agatacgccc 120
taagggggaa agaatttcgc taaaggatca gcctatgtcc tatcagctag ttggtgaggt 180
aaatgcttac caaggctatg acgggtatcc ggcctgagag ggtgaacgga cacaccggaa 240
ctgagacacg gtccggactc ctacgggagg cagcagtagg gaatattgct caatggggga 300
aaccctgaag cagcaacgcc gcgtggagga tgaaggttct aggattgtaa actccttttc 360
tgagagaaga taatgacggt atctcaggaa taagcaccgg ctaactccgt gccagcagcc 420
gcggtaatac ggagggtgca agcgttactc ggaatcactg ggcgtaaaga gcgtgtaggc 480
ggaatgataa gtcaggcgtg aaatcccgtg gcttaactgc ggaactgcgt ttgaaactat 540
cattctggag tgtgggagag gcaggtggaa ctcttggtgt aggggtaaaa tccgtagata 600
tcaagaagaa tactcattgc gaaggcgacc tgctggaaca tcactgacgc tgatgcgcga 660
aagcgtgggg gagcaaacag gattagatac cctggtagtc cacgccctaa acgatggatg 720
ctaattgtcg gggggcttgt ctcctcggta atgcagctaa cgcattaagt atcccgcctg 780
gggagtacga tcgcaagatt aaaactcaaa ggaatagacg gggacccgca caagcggtgg 840
agcatgtggt ttaattcgaa gatacgcgaa gaaccttacc caggcttgac attgatggaa 900
tctaccctaa aaggtggagt gccagtttac tggagcctga aaacaggtgc tgcacggctg 960
tcgtcagctc gtgtcgtgag atgttgggtt aagtcccgca acgagcgcaa ccctcgtcct 1020
tagttgccat cagttcggct gggcactcta aggagactgc cttcgtaagg aggaggaagg 1080
cgaggatgac gtcaagtcat catggccctt acgcctgggg ctacacacgt gctacaatgg 1140
gatgtacaaa gagatgcaat gtcgtaagat ggagcaaaac tcaaaaacat ctcccagttc 1200
ggattgtggt ctgcaactcg accacatgaa gcaggaatcg ctagtaatcg tgaatcagcc 1260
atgtcac 1267

<210> 18
<211> 1236
<212> DNA
<213> Helicobacter sp.

<400> 18
ctagcttgct aggagtggat tagtggcgca cgggtgagta acgcatagga atgtgccctt 60
tagtctggga tagccaccgg aaacagtgat taatactaga tacgccctaa gggggaaaga 120
atttcgctaa aggatcagcc tatgtcctat cagctagttg gtgaggtaaa tgcttaccaa 180
ggctatgacg ggtatccgcc ctgagagggt gaacggacac accggaactg agacacggtc 240
cggactccta cgggaggcag cagtagggaa tattgctcaa tgggggaaac cctgaagcag 300
caacgccgcg tggaggatga aggttctagg attgtaaact ccttttctga gagaagataa 360
tgacggtatc tcaggaataa gcaccggcta actccgtgcc agcagccgcg gtaatacgga 420
gggtgcaagc gttactcgga atcactgggc gtaaagagcg tgtaggcgga atgataagtc 480
aggcgtgaaa tcccgtggct taactgcgga actgcgtttg aaactatcat tctggagtgt 540
gggagaggca ggtggaactc ttggtgtagg ggtaaaatcc gtagatatca agaagaatac 600
tcattgcgaa ggcgacctgc tggaacatca ctgacgctga tgcgcgaaag cgtggggagc 660
aaacaggatt agataccctg gtagtccacg ccctaaacga tggatgctaa ttgtcggggg 720
gcttgtctcc tcggtaatgc agctaacgca ttaagcatcc cgcctgggga gtacgatcgc 780
aagattaaaa ctcaaaggaa tagacgggga cccgcacaag cggtggagca tgtggtttaa 840
ttcgaagata cgcgaagaac cttacccagg cttgacattg atggaatcta ccccaaaagg 900
tggagtgcca gtttactgga gcctgaaaac aggtgctgca cggctgtcgt cagctcgtgt 960
cgtgagatgt tgggttaagt cccgcaacga gcgcaaccct cgtccttagt tgccatcagt 1020

tcggctgggc actctaagga gactgccttc gtaaggagga ggaaggcgag gatgacgtca 1080
agtcatccat ggccccttac gcctggggct acacacgtgc tacaatggga tgtacaaaga 1140
gatgcaatgt cgtaagatgg agcaaaactc aaaaggrtct cccagttcgg attgtggtct 1200
gcaactcgac cacatgaagc agaatcgcta gtaatc 1236

<210> 19
<211> 1194
<212> DNA
<213> Helicobacter sp.

<400> 19
tggcgcacgg gtgagtaacg cataggaatg tgccctttag tctgggatag ccaccggaaa 60
cgatgattaa tactagatac gccctaaggg ggaaagaatt tcgctaaagg atcagcctat 120
gtcctatcag ctagttggtg aggtaaatgc ttaccaaggc tatgacgggt atccgccctg 180
agagggtgaa cggacacacc ggaactgaga cacggtccgg actcctacgg gaggcagcag 240
tagggaatat tgctcaatgg gggaaaccct gaagcagcaa cgccgcgtgg aggatgaagg 300
ttctaggatt gtaaactcct tttctgagag aagataatga cggtatctca ggaataagca 360
ccggctaact ccgtgccagc agccgcggta atacggaggg tgcaagcgtt actcggaatc 420
actgggcgta aagagcgtgt aggcggaatg ataagtcagg cgtgaaatcc cgtggcttaa 480
ctgcggaact gcgtttgaaa ctatcattct ggagtgtggg agaggcaggt ggaactcttg 540
gtgtaggggt aaaatccgta gatatcaaga agaatactca ttgcgaaggc gacctgctgg 600
aacatcactg acgctgatgc gcgaaagcgt ggggagcaaa caggattaga taccctggta 660
gtccacgccc taaacgatgg atgctaattg tcggggggct tgtctcctcg gtaatgcagc 720
taacgcatta agcatcccgc ctggggagta cgatcgcaag attaaaactc aaaggaatag 780
acggggaccc gcacaagcgg tggagcatgt ggtttaattc gaagatacgc gaagaacctt 840
acccaggctt gacattgatg gaatctaccc caaaaggtgg agtgccagtt tactggagcc 900
tgaaaacagg tgctgcacgg ctgtcgtcag ctcgtgtcgt gagatgttgg gttaagtccc 960
gcaacgagcg caaccctcgt ccttagttgc catcagttcg gctgggcact ctaaggagac 1020
tgccttcgta aggaggagga aggcgaggat gacgtcaagt catcatggcc cttacgcctg 1080
gggctacaca cgtgctacaa tgggatgtac aaagagatgc aatgtcgtaa gatggagcaa 1140
aactcaaaaa catctcccag ttcggattgt ggtctgcaac tcgaccacat gaag 1194

<210> 20
<211> 1172
<212> DNA
<213> Helicobacter sp.

<400> 20
gcataggaat gtgcccttta gtctgggata gccaccggaa acagtgatta atactagata 60
cgccctaagg gggaaagaat ttcgctaaag gatcagccta tgtcctatca gctagttggt 120
gaggtaaatg cttaccaagg ctatgacggg tatccggcct gagagggtga acggacacac 180
cggaactgag acacggtccg gactcctacg ggaggcagca gtagggaata ttgctcaatg 240
ggggaaaccc tgaagcagca acgccgcgtg gaggatgaag gttctaggat tgtaaactcc 300
ttttctgaga gaagataatg acggtatctc aggaataagc accggctaac tccgtgccag 360
caaccgcggt aatacggagg gtgcaaacgt tactcggaat cactgggcgt aaagagcgtg 420
taggcggaat gataagtcag gcgtgaaatc ccgtggctta actgcggaac tgcgtttgaa 480
actatcattc tggagtgtgg gagaggcagg tggaactctt ggtgtagggg taaaatccgt 540
agatatcaag aagaatactc attgcgaagg cgacctgctg gaacatcact gacgctgatg 600
cgcgaaagcg tggggagcaa acaggattag ataccctggt agtccacgcc ctaaacgatg 660
gatgctaatt gtcggggggc ttgtctcctc ggtaatcgag ctaacgcatt aagcatcccg 720
cctggggagt acgatcgcaa gattaaaact caaaggaata gacggggacc cgcacaagcg 780
gtggagcatg tggtttaatt cgaagatacg cgaagaacct tacccaggct tgacattgat 840
gcaatctacc ccaaaaggtg gagtgccagt ttactggagc ctgaaaacag gtgctgcacg 900
gctgtcgtca gctcgtgtcg tgagatgttg ggttaagtcc cgcaacgagc gcaaccctcg 960
tccttagttg ccatcagttc ggctgggcac tctaaggaga ctgccttcgt aaggaggagg 1020
aaggcgagga tgacgtcaag tcatcatggc ccttacgcct ggggctacac acgtgctaca 1080
atgggatgta caaagagatg caatgtcgta agatggagca aaactcaaaa acatctccca 1140

gttcggattg tggtctgcaa ctcgaccaca tg                                1172

<210> 21
<211> 1400
<212> DNA
<213> Helicobacter sp.

<400> 21
tgcaagtcga acgatgaagc ctagcttgct aggttgatta gtggcgcacg ggtgagtaat 60
gcatagatga catgcccttt agtttggaat agccactaga aatggtgatt aataccaaat 120
actaccttac gagggaaaga tttatcgcta aaggattggt ctatgtccta tcagcttgtt 180
ggtgaggtaa aggctcacca aggctatgac gggtatccgg cctgagaggg tgagcggaca 240
cactggaact gagacacggt ccagactcct acgggaggca gcagtaggga atattgctca 300
atgggggaaa ccctgaagca gcaacgccgc gtggaggatg aaggttttag gatcgtaaac 360
tccttttgtt agagaagata atgacggtat ctaacgaata agcaccggct aactccgtgc 420
cagcagccgc ggtaatacgg agggtgcaag cgttactcgg aatcactggg cgtaaagagt 480
gcgtaggcgg gaggacaagt caggtgtgaa atcctatggg cttaaccata gaactgcatt 540
tgaaactatc cttctggagt gtgggagagg taggtggaat tcttggtgta gggggtaaaa 600
tccgtagaga tcaagaggga tactcattgc gaaggcgacc tgctggaaca tcactgacgc 660
tgattgcacg aaagcgtggg ggagcaaaca gggattagat accctggtag tccacgccct 720
aaacgattgg atgctagttg ttgggaaggc tttgtctttc cagtaatgca gctaacgcct 780
taagcatccc gcctggggag tacggtcgca agattaaaac tcaaaggaat agacggggac 840
ccgcacaagc ggtggagcat gtggtttaat tcgaggatac acgaagaacc ttacctaggc 900
ttgacattga aggaattccc tagaaatagg ggagtgtcta gcttgctaga ccctgaaaac 960
aggtgctgca cggctgtcgt cagctcgtgt cgtgagatgt tgggttaagt cccgcaacga 1020
gcgcaaccct ttttcttagt tgctaacagg ttatgctgcg cactctaaga agactgcctg 1080
cgtaagcagg aggaaggtga ggacgacgtc aagtcatcat ggcccttacg cctagggcta 1140
cacacgtgct acaatggggt gcacaaagag atgcaaagcc gcgaggcaga gctaatctat 1200
aaaacacctc ctagttcgga ttgcaggctg caactcgcct gcatgaagct ggaatcgcta 1260
gtaatcgcaa atcagctatg ttgcggtgaa tacgttcccg ggtcttgtac tcaccgcccg 1320
tcacaccatg ggagttgtgt ttgccttaag tcaggatgct aaagcagcta ctgcccacgg 1380
cacacacagc gactggggtg                                             1400

<210> 22
<211> 1353
<212> DNA
<213> Helicobacter sp.

<400> 22
gattagtggc gcacgggtga gtaatgcata gatgacatgc cctttagttt ggaatagcca 60
ctagaaatgg tgattaatac caaatactac cttacgaggg aaagatttat cgctaaagga 120
ttggtctatg tcctatcagc ttgttggtga ggtaaaggct caccaaggct atgacgggta 180
tccggcctga gagggtgagc ggacacactg gaactgagac acggtccaga ctcctacggg 240
aggcagcagt agggaatatt gctcaatggg ggaaaccctg aagcagcaac gccgcgtgga 300
ggatgaaggt tttaggatcg taaactcctt ttgttagaga agataatgac ggtatctaac 360
gaataagcac cggctaactc cgtgccagca gccgcggtaa tacggagggt gcaagcgtta 420
ctcggaatca ctgggcgtaa agagtgcgta ggcgggagga caagtcaggt gtgaaatcct 480
atggcttaac catagaactg catttgaaac tatccttctg gagtgtggga gaggtaggtg 540
gaattcttgg tgtaggggta aaatccgtag agatcaagag gaatactcat tgcgaaggcg 600
acctgctgga acatcactga cgctgattgc acgaaagcgt ggggrgcaaa caggdttaga 660
taccctggta gtccacgccc taaacgatgg atgctagttg ttgggaggct ttgtctttcc 720
agtaatgcag ctaacgcctt aagcatcccg cctggggagt acggtcgcaa gattaaaact 780
caaaggaata gacggggacc cgcacaagcg gtggagcatg tggtttaatt cgatgataca 840
cgaagaacct tacctaggct tgacattgaa ggaattcccc tagaaatagg ggagtgtcta 900
gcttgctaga ccctgaaaac aggtgctgca cggctgtcgt cagctcgtgt cgtgagatgt 960
tgggttaagt cccgcaacga gcgcaaccct ttttcttagt tgctaacagg ttatgctgcg 1020

cactctaaga agactgcctg cgtaagcagg aggaaggtga ggacgacgtc aagtcatcat 1080
ggcccttacg cctagggcta cacacgtgct acaatggggt gcacaaagag atgcaaagcc 1140
gcgaggcaga gctaatctat aaaacacctc ctagttcgga ttgcaggctg caactcgcct 1200
gcatgaagct ggaatcgcta gtaatcgcaa atcagctatg ttgcggtgaa tnacgtkccc 1260
gggtcttgta ctcaccgccc gtcacaccat gggagttgtg tttgccttaa gtcaggatgc 1320
taaagcagct actgcccacg cacacacagc gac 1353

<210> 23
<211> 1355
<212> DNA
<213> Helicobacter sp.

<400> 23
gattagtggc gcacgggtga gtaatgcata gatgacatgc cctttagttt ggaatagcca 60
ctagaacatg gtgattaata ccaaatacta ccttacgagg gaaagattta tcgctaaagg 120
attggtctat gtcctatcag cttgttggtg aggtaaaggc tcaccaaggc tatgacgggt 180
atccggcctg agagggtgag cggacacact ggaactgaga cacggtccag actcctacgg 240
gaggcagcag tagggaatat tgctcaatgg gggaaaccct gaagcagcaa cgccgcgtgg 300
aggatgaagg ttttaggatc gtaaactcct tttgttagag aagataatga cggtatctaa 360
cgaataagca ccggctaact ccgtgccagc agccgcggta atacggaggg tgcaagcgtt 420
actcggaatc actgggcgta aagagtgcgt aggcgggagg acaagtcagg tgtgaaatcc 480
tatggcttaa ccatagaact gcatttgaaa ctatccttct ggagtgtggg agaggtaggt 540
ggaattcttg gtgtaggggt aaaatccgta gagatcaaga ggaatactca ttgcgaaggc 600
gacctgctgg aacatcactg acgctgattg cacgaaagcg tggggagcaa acaggattag 660
ataccctggt agtccacgcc ctaaacgatg gatgctagtt gttgggaggc tttgtctttc 720
cagtaatgca nctaacgcct taagcatccc gcctggggag tacggtcgca agattaaaac 780
tcaaaggaat agacggggac ccgcacaagc ggtggagcat gtggtttaat tcgaagttac 840
acgaagaacc ttacctaggc ttgacattga aggaattccc ctagaaatag gggagtgtct 900
agcttgctag accctgaaaa caggtgctgc acggctgtcg tcagctcgtg tcgtgagatg 960
ttgggttaag tcccgcaacg agcgcaaccc ctttttctta gttgctaaca ggtttatgtg 1020
cgcactctaa gaagactgcc tgcgtaagca ggaggaaggt gaggacgacg tcaagtcatc 1080
atggccctta cgcctagggc tacacacgtg ctacaatggg gtgcacaaag agatgcaaag 1140
ccgcgaggca gagctaatct ataaaacacc tcctagttcg gattgcaggc tgcaactcgc 1200
ctgcatgaag ctggaatcgc tagtaatcgc aaaatcagct atgttgcggt gaatacgttc 1260
cccgggtctt gtactcaccg ccccgtcaca ccatgggagt tgtgtttgcc ttaagtcagg 1320
atgctaaagc agctactgcc cacgcacaca cagcg 1355

<210> 24
<211> 1358
<212> DNA
<213> Helicobacter sp.

<400> 24
agtgdhgcac gggtgagtaa tgcatagatg acatgccctt tagtttggaa tagccactag 60
adatggtgat taataccaaa tactacctta cgagggvnag attatcgcta aaggattggt 120
ctatgtccta tcagcttgtt gggtgaggta aaggctcacc aagggctatg acgggtatcc 180
ggcctgagag gktgagcgga cacactggaa ctgagacacg gtccagactc ctacgggagg 240
cagcagtagg gaatattgct caatgggggg aaaccctgaa gcagcaacgc cgcgtggagg 300
atgaaggttt taggatcgka aactcctttt gttagagaag ataatgacgg tatctaacga 360
ataagcaccg gctaactccg tgccagcagc cgcggtaata cggagggtgc aagcgttact 420
cggaatcact gggcgtaaag agtgcgtagg cgggaggaca agtcaggtgt gtgaaatcct 480
atggcttaac catagaactg catttbaaac tatccttctg gagtgtggga gaggtaggtg 540
gaattcttgg tgtaggggta aaatccgtag agatcaagag ggatactcat tggcgaaggc 600
gacctgctgg aacatcactg acgctgattg cacgaaagcg tggggagcaa acaggattag 660
ataccctggt agtccacgcc ctaaacgatg gatgctagtt gttgggaggc tttgtctttc 720
cagtaatgca gctaacgcct taagcatccc gcctggggag tacggtcgca agattaaaac 780
tcaaarggaa tagacgggga cccgcacaag cggtggagca tgtggtttaa ttcgargwta 840

cacgaagaac ccttacctag gccttgacat tgaaggaatt cccctagaaa taggggagtg 900
tctagcttgc tagaccctga aaacaggtgc tgcacggctg tcgtcagctc gtgtcgtgag 960
atgttgggtt aagtcccgca acgagcgcaa ccctttttct tagttgctaa caggttatst 1020
gcgcactcta agaagactgc ctgcgtaagc aggaggaagg tgaggacgac gtcaagtcat 1080
catggccctt acgcctaggg ctacacacgt gctacaatgg ggtgcacaaa gagatgcaaa 1140
gccgcgaggc agagctaatc tataaaacac ctcctagttc ggattgcagg ctgcaactcg 1200
cctgcatgaa gctggaawtc gctagtaaat cgcaaattca gctatgttgc ggtgaatdmg 1260
tkccccgggt cttgtactca ccgccccgtc acacccatgg gagttgtgtt tgccttaagt 1320
caggatgcta aagcagctac tgcccacgca cacacagc 1358

<210> 25
<211> 21
<212> DNA
<213> Helicobacter sp.

<400> 25
aggaattccc tagaaatagg g 21

## Description

The present invention relates to the field of detection and typing of *Helicobacter* infection in clinical samples from humans and other mammals. The present invention relates more particularly to bovine and porcine 16 rDNA polynucleotide sequences as well as their use in diagnostic applications.

In the 19^{th} century, gastric spiral organisms were described for the first time in different animals (Rappin, 1881; Bizzozero, 1893; Salomon, 1896). Salomon observed spiral organisms in the stomachs of dogs, cats and the brown Norwegian rat but not in humans, monkeys, pigs, mice, pigeons, crows and cattle (Salomon, 1896). It was only in 1984 that a renewed interest emerged for similar organisms after the isolation of *Helicobacter pylori* from the human stomach (Marshall & Warren, 1984). The association of *H. pylori* with chronic gastritis, peptic ulceration and gastric cancer (Cover & Blaser, 1992; Blaser et al., 1991; Parsonnet et al., 1991) resulted in intensive research worldwide. Various *Helicobacter* species were isolated from the gastrointestinal tract of different animals. To date, the genus *Helicobacter* consists of 18 different species (On, 1996; Franklin et al., 1996; Mendes et al., 1996; Jalava et al., 1997; Trivett-Moore et al., 1997; Shen et al., 1997) and constitutes together with the genera *Wolinella, Campylobacter* and *Arcobacter,* the epsilon subdivision of the *Proteobacteria,* also known as rRNA superfamily VI (Vandamme et al., 1991).

In 1992, two different groups almost simultaneously reported the presence of helically shaped bacteria in the abomasum of calves and adult cattle based on histological data (Günther & Schulze, 1992; Haringsma & Mouwen, 1992). Both groups described large numbers of spiral-shaped bacteria in the gastric crypts of the pyloric region and considered them as putative *Helicobacter* species. Further indirect evidence of the presence of *Helicobacter-like* organisms in adult cattle and calves was given by serological studies. Seidel et al. (1996) found significant titers of antibodies against H. *pylori* epitopes in the serum of calves after absorption with *Campylobucter jejuni, Wolinella succinogenes, Escherichia coli* and *Proteus mirabilis* strains. One report described a bactericidal activity of bovine serum, colostrum and milk against *H. pylori* (Korhonen et al., 1995). In vitro isolation of these organisms has not been successful so far (Jelinski et al., 1995; Braun et al., 1997) and the taxonomic status of these putative *Helicobacter*-like bacteria is unknown.

The pathogenic role of *H. pylori* led to speculations about the association of bovine *Helicobacter*-like bacteria with abomasal ulcer disease, although no conclusive evidence has been provided to date (Günther & Schulze, 1992 ; Haringsma & Mouwen, 1992). Other bacteria such as *Campylobacter* species and *Clostridium perfringens* have also been studied in association with the occurrence of abomasal lesions (Al Mashat & Taylor, 1980; Mills et al., 1990, Jelinski et al., 1995).

Within the genus *Helicobacter,* a phylogenetic subgroup of morphologically similar bacteria can be distinguished. These bacteria, characterized by their long and tightly coiled (gastrospirillum-like) appearance, have been observed in gastric biopsies of humans, cats, lemurs, dogs, pigs and exotic carnivores (Dent et al., 1987; Lee et al., 1988; O'Rourke et al., 1992; Hänninen et al., 1996; Jalava et al., 1997; Queiroz et al., 1990; Eaton et al., 1993; Jakob et al., 1997). Three species with this morphology (*H*. *felis*, *H. bizzozeronii, H. salomonis)* have been isolated and characterised from gastric samples of cats and dogs (Paster et al., 1991; Hänninen et al., 1996; Jalava et al., 1997).

The observation of gastrospirillum-like organisms in humans was described for the first time in 1987 by Dent et al (Dent et al., 1987). Although initially referred to as "*Gastrospirillum hominis*" (McNulty et al., 1989), this organism was later renamed "*Helicobacter heilmannii*" as 16S rDNA sequence analysis revealed that these human gastrospirilla belonged to the genus *Helicobacter* (O'Rourke et al., 1992; Solnick et al., 1993). From these results, it also became clear that there were at least two different types of "*Helicobacter heilmannii*", referred to as type 1 and type 2. This observation was based on a 3.5 % sequence difference, suggesting that the two sequences represented two different species. The first isolation of a "*Helicobacter heilmannii*" - like bacterium from humans was recently reported by Andersen *et al.* (1996).

In pigs, gastrospirillum-like bacteria were observed in the antral pits and at the mucosal surface of the stomach (Quieroz et al.,1990) and have provisionally been named "*Gastrospirillum suis*" (Mendes et al., 1990). Histopathological studies associated this bacterium with pyloric lymphonodular gastritis (Mendes et al., 1991) and gastric ulcer disease of the pars oesophagea in pigs (Barbosa et al., 1995, Quieroz et al., 1996). Although *in vitro* cultivation of "*Gastrospirillum suis*" has been unsuccessful (Queiroz et al., 1990), *in vivo* cultivation in mice and rats has been reported (Moura et al., 1993; Mendes et al., 1996). In one case, a *Helicobacter* was isolated from the faeces of swine (Seymour et al., 1994) which was later characterised as *Helicobacter pametensis* (Dewhirst et al., 1994). Other members of rRNA superfamily VI, *Campylobacter hyointestinalis* subsp. *Lawsonii* (On et al., 1995), *Arcobacter butzleri* and *Arcobacter cryaerophilus* (Suarez et al., 1997), have also been isolated from the stomach of swine.

As "*Gastrospirillum suis*" remains unculturable, an official species designation is impossible according to the guidelines of the *International Code of Nomenclature of Bacteria* which are stating the necessity of a broad range of phenotypic and phylogenetic data. Murray and Schleifer (1994) anticipated this problem, and proposed a provisional status to record the properties of putative taxa of prokaryotes. This proposal was implemented in 1995 by the International Committee on Systematic Bacteriology by the introduction of the provisional status *Candidatus* for the description of uncultivable organisms based upon genomic data and to a certain extent structural, metabolic, reproductive and environmental characteristics (Murray and Stackebrandt, 1995).

It is an aim of the present invention to provide new *Helicobacter* nucleotide sequences of the 16S rRNA coding gene.

It is also an aim of the present invention to provide new probes and primers for detection of *Helicobacter* species.

It is also an aim of the present invention to provide methods and kits for detection and/or typing of *Helicobacter* species present in cattle and pigs.

It is further an aim of the present invention to provide methods and kits for detection of zoönoses in human samples.

It is also an aim of the present invention to provide new nucleotide sequences for studying and detecting the occurrence of pathogenic *Helicobacter* strains in mammals, more particularly in cattle and pigs.

All the aims of the present invention are met by the following embodiments.

According to one embodiment, the present invention relates to an isolated 16S rDNA *Helicobacter* polynucleic acid sequence selected from any of the following
(a) a sequence represented in any of SEQ ID NO 1 or 2, or the RNA version thereof,
(b) a sequence which hybridizes under stringent conditions to any of the sequences set out in (a).

The term "16S ribosomal polynucleic acid sequences" as used in the present invention refers to 16S rRNA or 16S rDNA polynucleic acid sequences.

According to a first aspect of the present invention, seven abomasal biopsies of adult cattle were sampled from different Belgian and Dutch farms. In all samples the presence of *Helicobacter*-like organisms was demonstrated by biochemical, immunohistochemical and electronmicroscopical data. Bacterial 16S rDNA was amplified from each sample by PCR and sequences were determined either by direct or indirect sequence analysis. Pairwise comparisons revealed all sequences to be more than 99 % homologous. Phylogenetic analysis placed the organism, corresponding to the reference sequence R2XA, within the genus *Helicobacter.* A diagnostic PCR-assay was designed, differentiating the bovine 16S rDNA sequences from those of 15 different *Helicobacter* strains and *Wolinella succinogenes.* These results indicated the corresponding organism to represent a single taxon. The low similarity level towards *H. bilis* (92.8 %), its closest validly named neighbour, strongly suggests that this novel taxon indeed is a novel *Helicobacter* species. An *in situ* hybridisation procedure associated the bovine sequences to the *Helicobacter*-like organisms in the abomasum.

According to a second aspect, the present invention relates to new *Helicobacter* sequences from pigs. Stomachs of five slaughterhouse pigs originating from different Belgian and Dutch farms were selected based on the presence of *"Gastrospirillum suis"* -like bacteria as demonstrated by biochemical, immunohistochemical and electronmicroscopical data. Using broad range primers, bacterial 16S rDNA was amplified by PCR and five *Helicobacter*-like sequences were determined either by direct or indirect sequence analysis. An intersequence homology of 99.7 % was observed, suggesting that the sequences originated from strains belonging to a single species. Phylogenetic analysis of the consensus sequence placed the organism within the genus *Helicobacter,* where it formed a distinct subgroup together with other gastrospirillum-like bacteria *(H. felis, H. bizzozeronii, H. salomonis,* "*H heilmannii*" type 1 and type 2). Diagnostic PCR-primers and a probe were developed, differentiating the porcine sequences from all known *Helicobacters.* These results indicate that the porcine sequences represent a single taxon within the genus *Helicobacter.* The low similarity level towards *H*. *salomonis* (96.6 %), its closest validly named neighbour, strongly suggests that this novel taxon indeed is a novel *Helicobacter* species. *In situ* hybridisation experiments linked the reference sequence to the "*Gastrospirillum suis*"-like bacteria. On the basis of these results, the name "*Candidatus* Helicobacter suis" for this new gastric *Helicobacter* from pigs is proposed.

These sequences are commonly characterized by the fact that they can be used to study and most probably detect pathogenic *Helicobacter* strains in mammals, more particularly in cattle and pigs. Such pathogenic strains cause for instance gastric ulcers and chronic gastritis.

The present invention relates more particularly to an isolated polynucleic acid sequence as defined above represented by any of SEQ ID NO 1 or 2 or 15 to 24.

The present invention also relates to an isolated polynluceic acid sequence as defined above which is more than 92.8%, preferably more than 93.5%, more preferably more than 95% and most preferably more than 97.5% homologous to SEQ ID NO 1. Other preferred ranges of homology include 93, 94, 94.5, 95.5, 96, 96.5, 97, 98, 98.5, 99 or 99.5%.

Sequences which have a homology of more than 92.8% to SEQ ID NO 1 are considered to belong to the same group of organisms as the one where SEQ ID NO 1 has been derived from.

According to the present invention, the homologies of SEQ ID NO 1 were calculated by means of the GENESCAN program (Applied Maths bvba, Risquons-toutstraat 38, B-8511 Kortrijk, Belgium).

The term "homology" refers to a sequence identity as calculated by the above-given program.

SEQ ID NO 2 is 99.5% homologous to the closest found sequence. Sequences of more than 99.5% homology compared to SEQ ID NO 2 are also within the scope of the present invention.

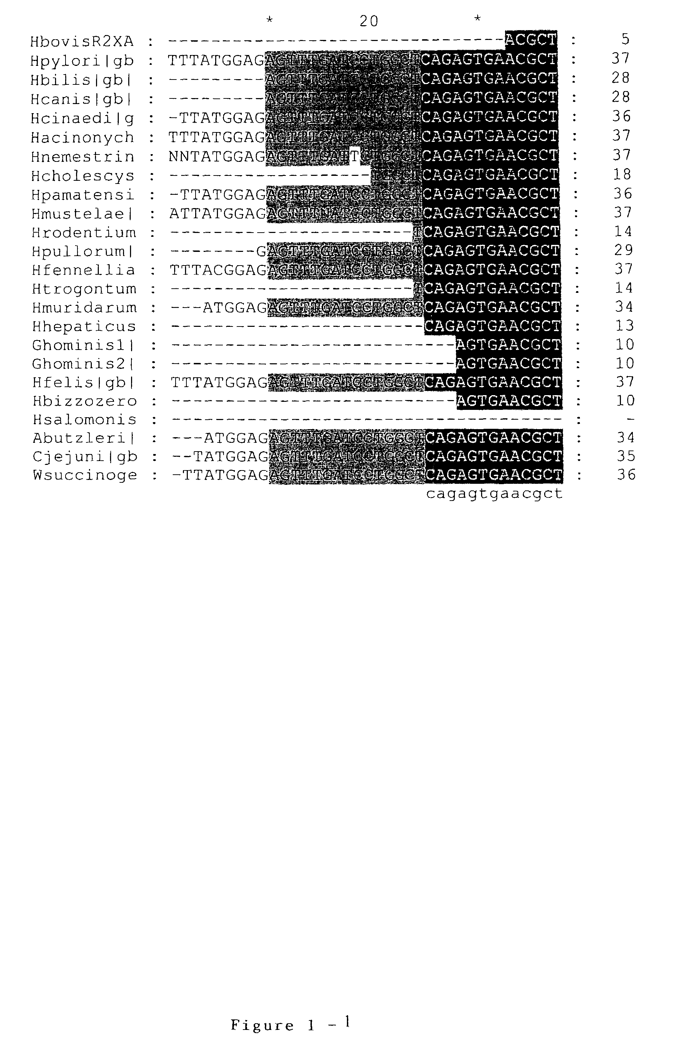

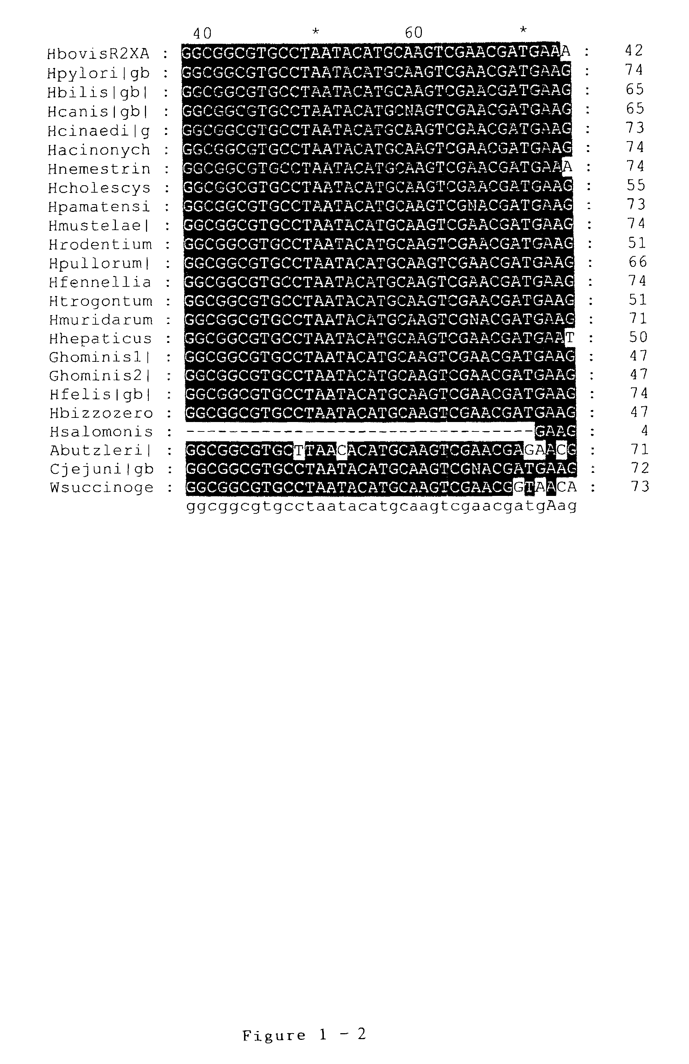

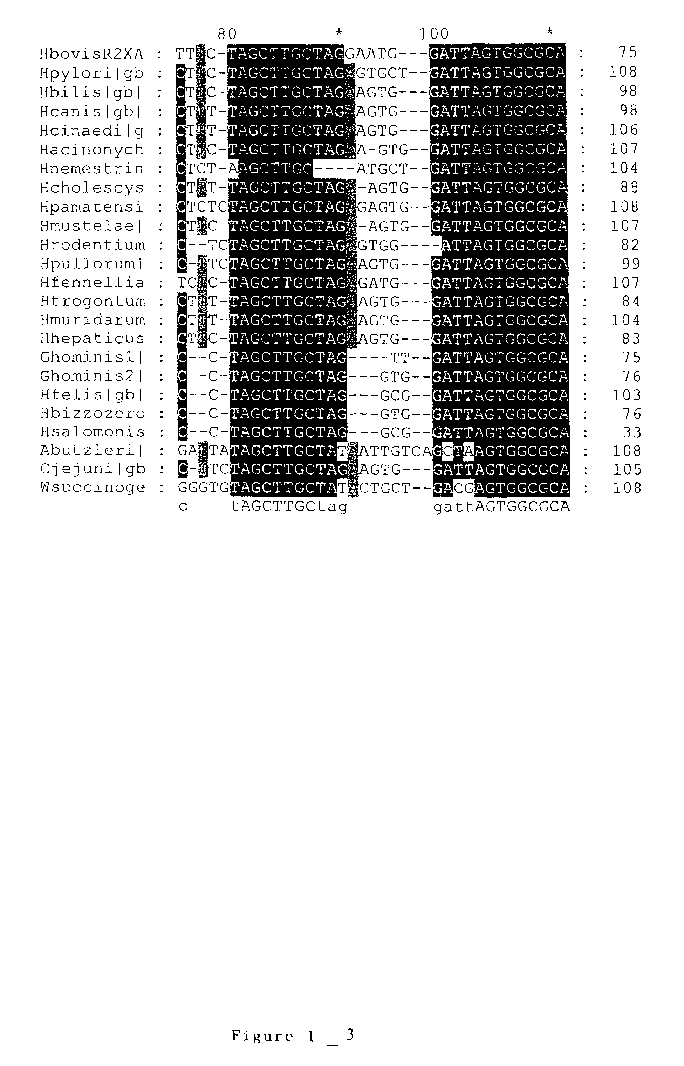

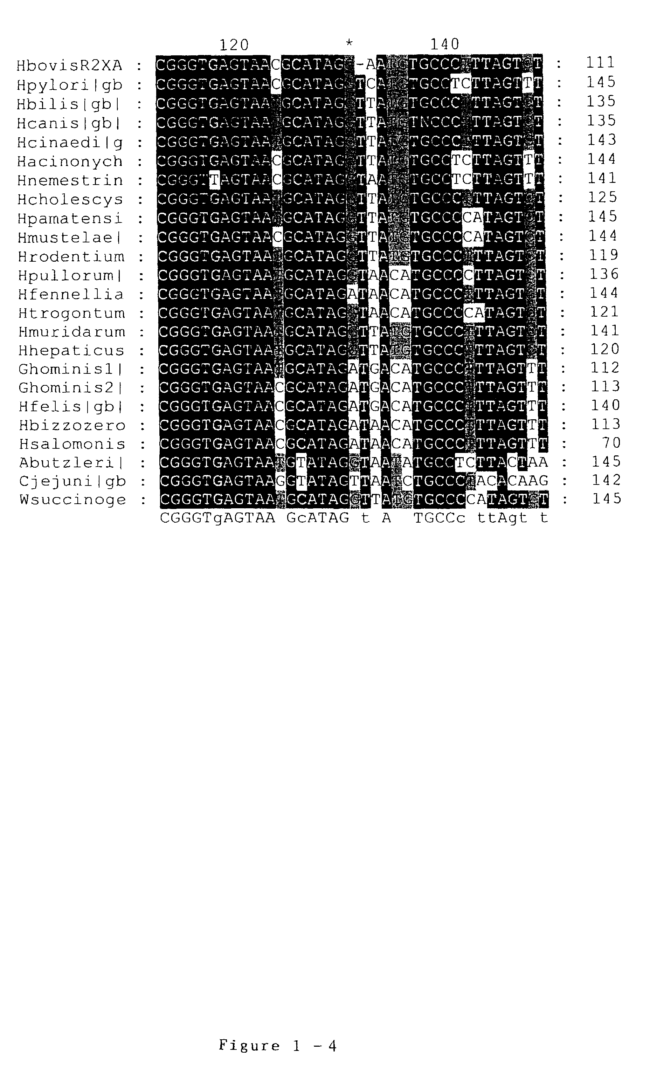

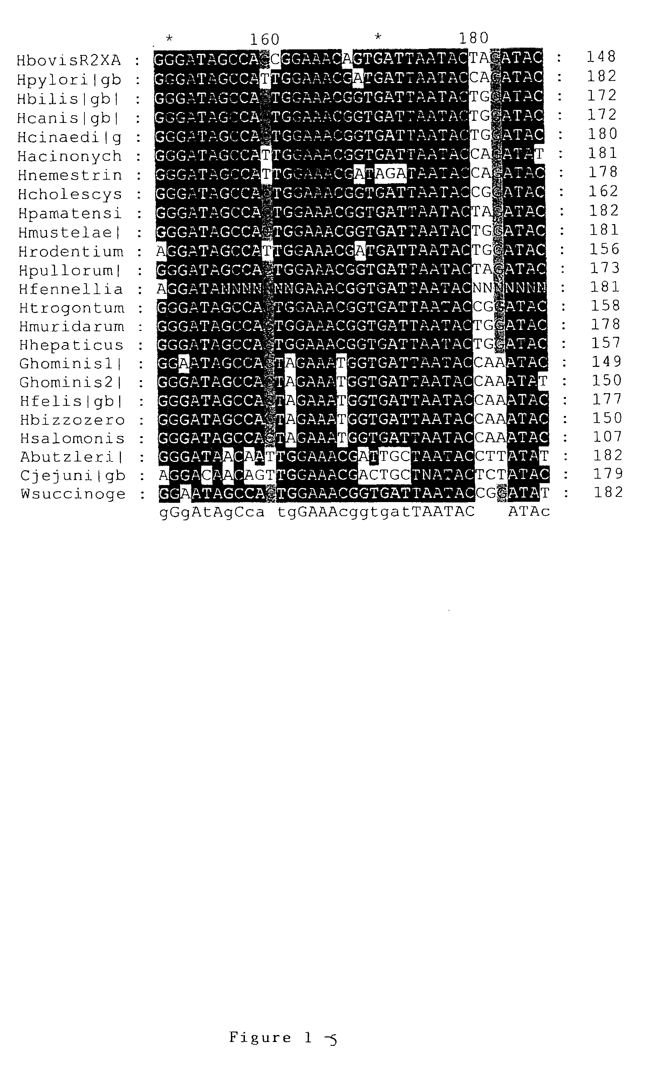

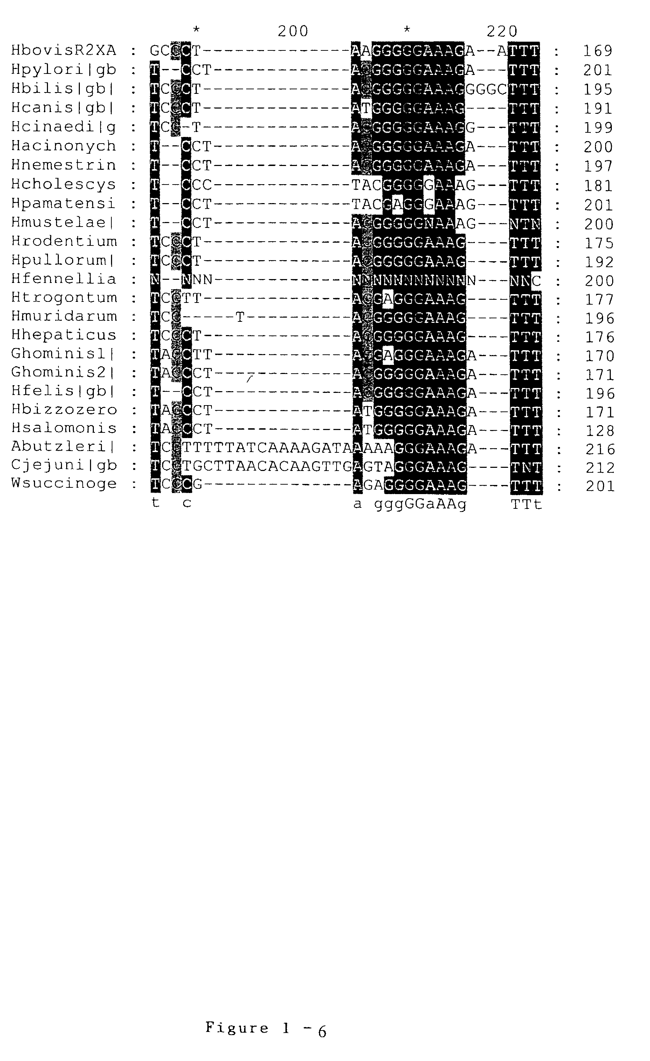

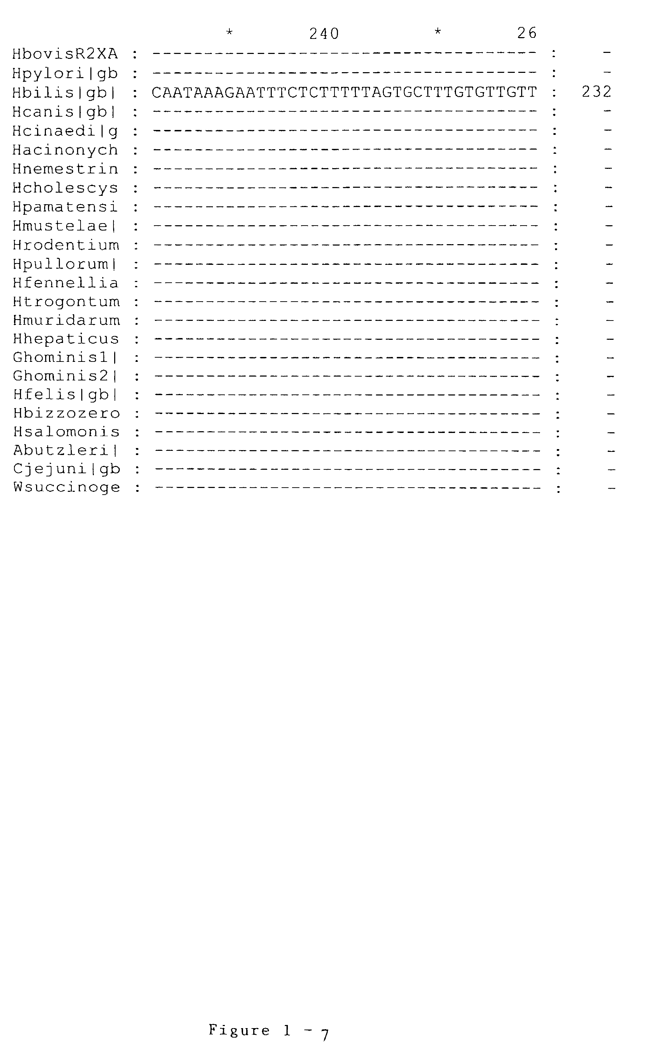

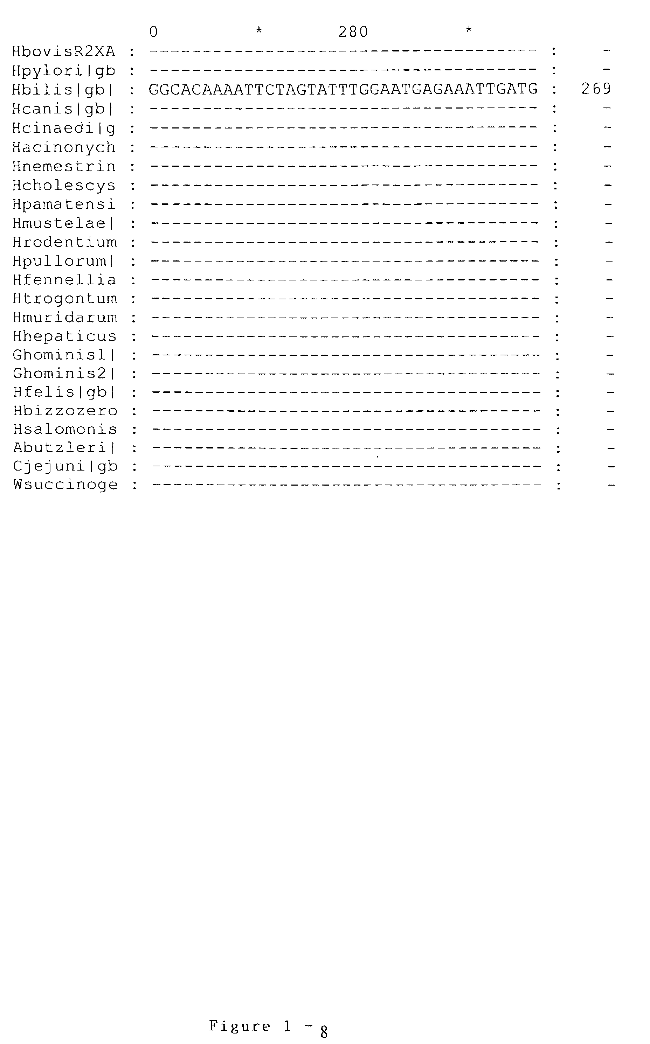

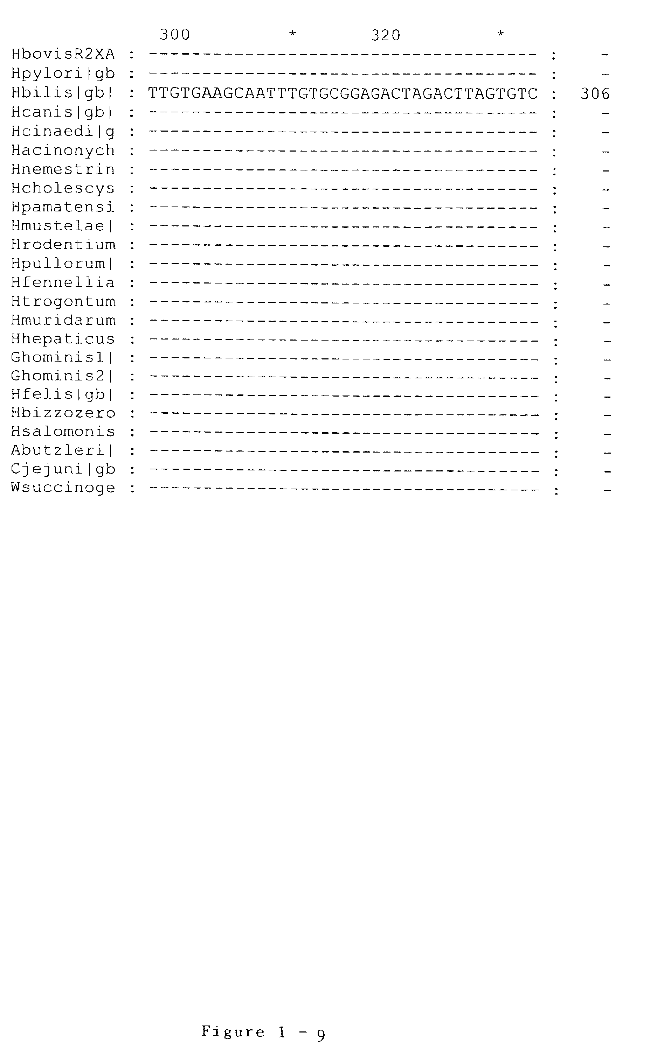

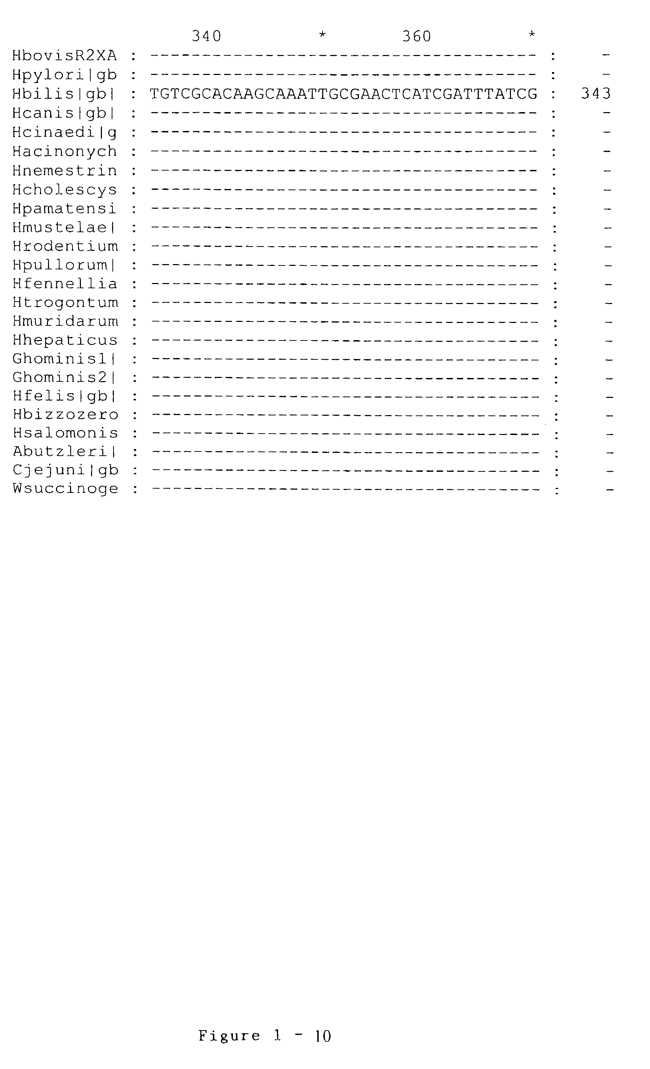

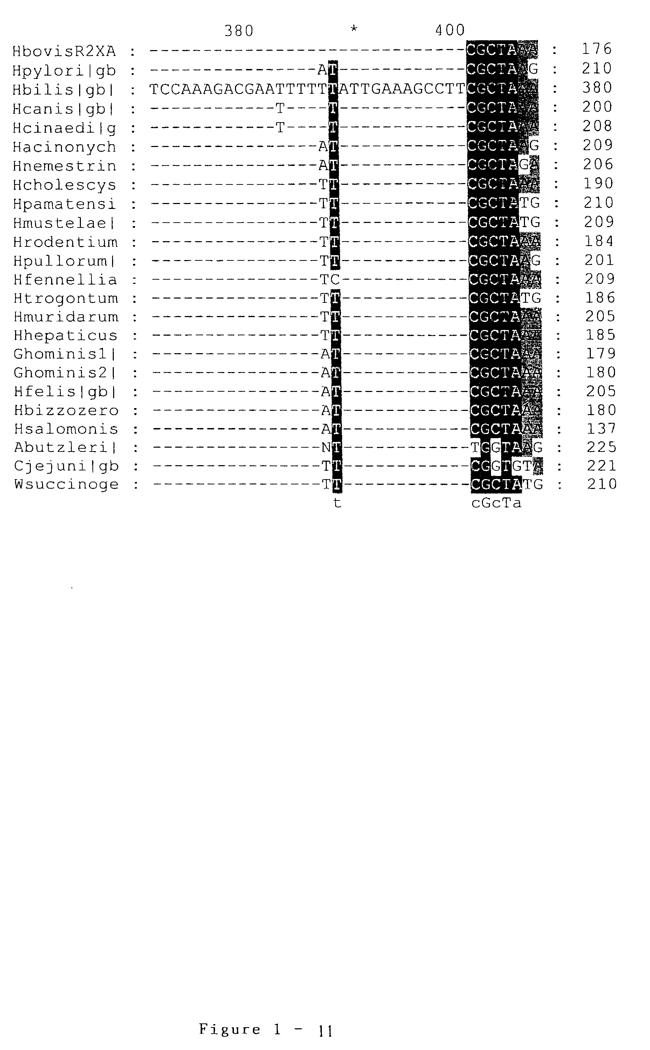

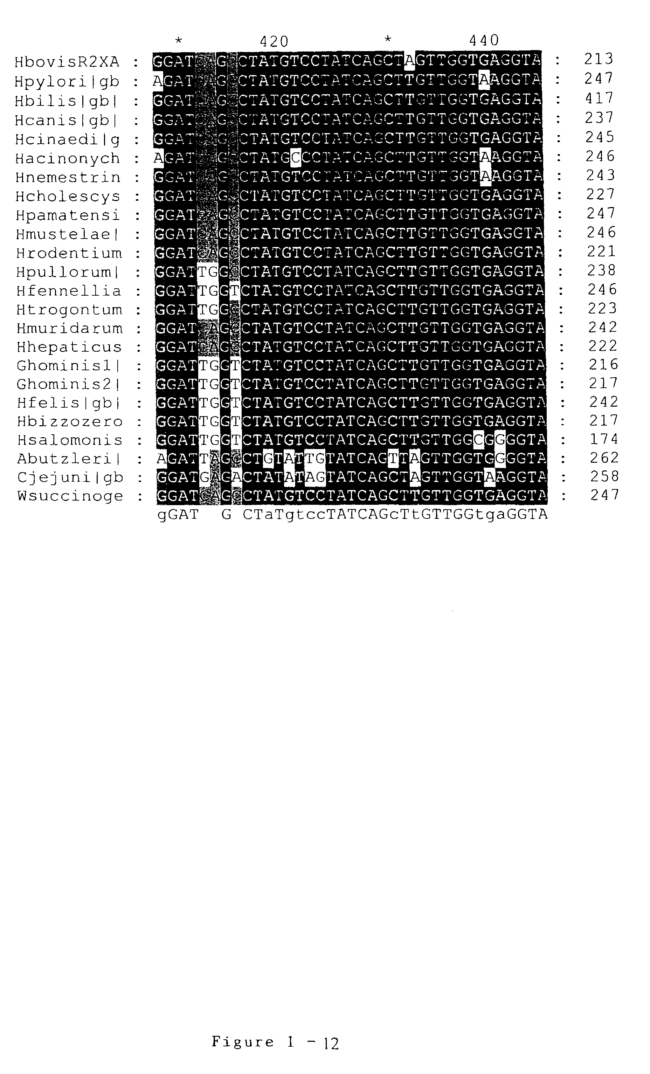

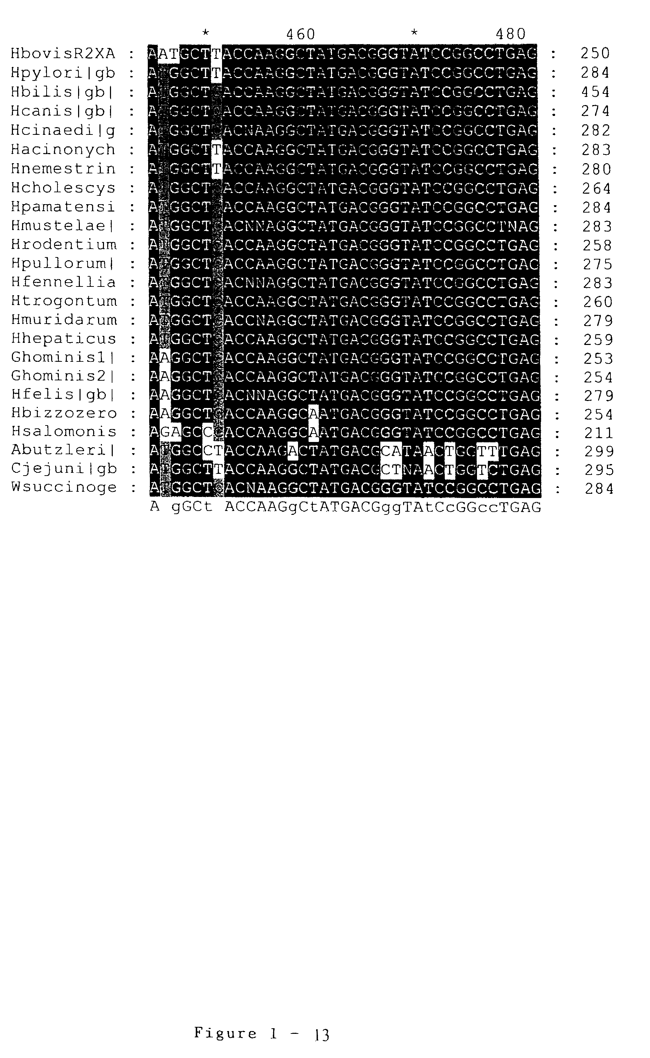

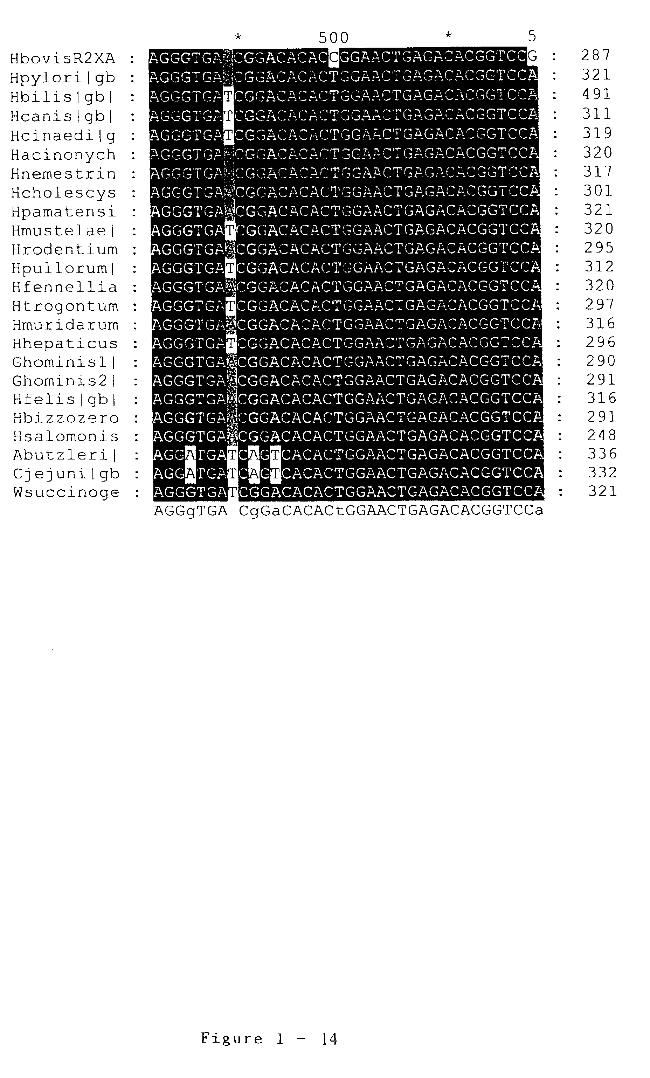

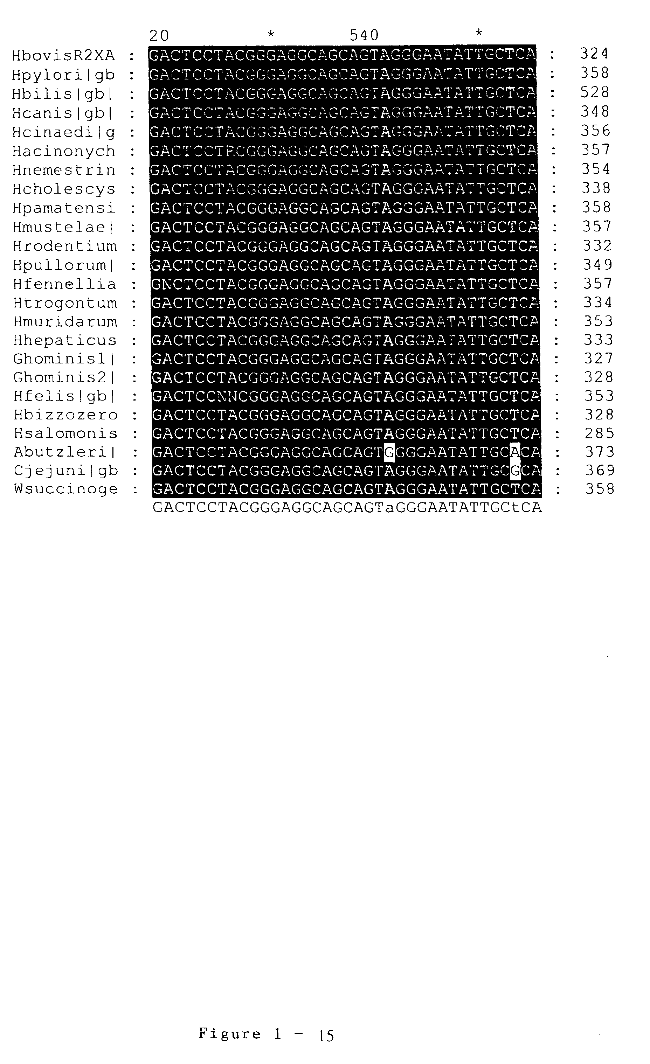

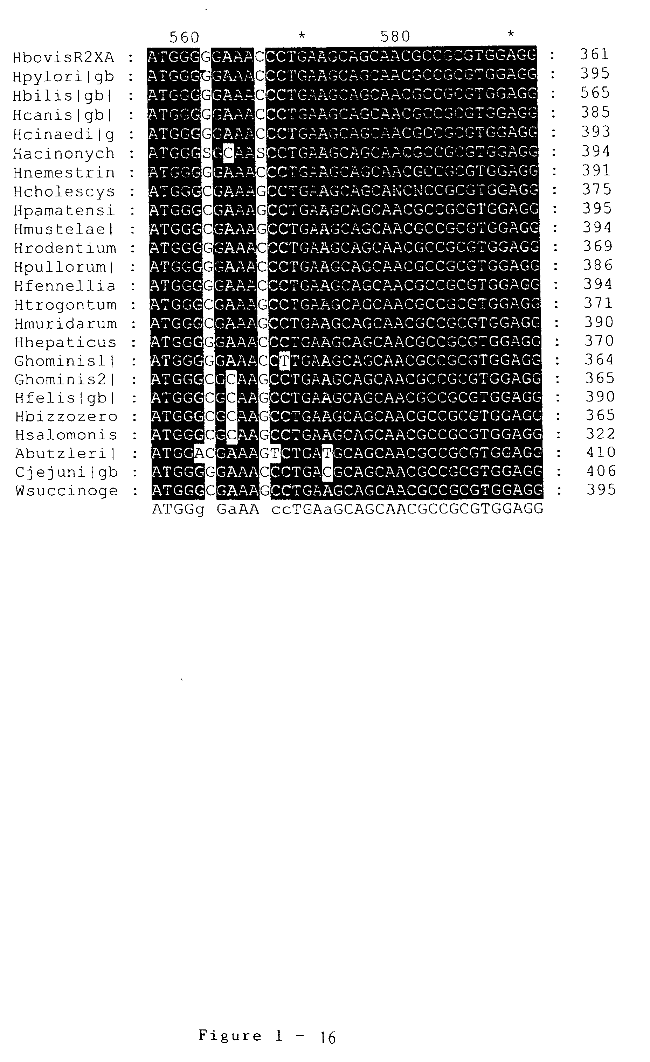

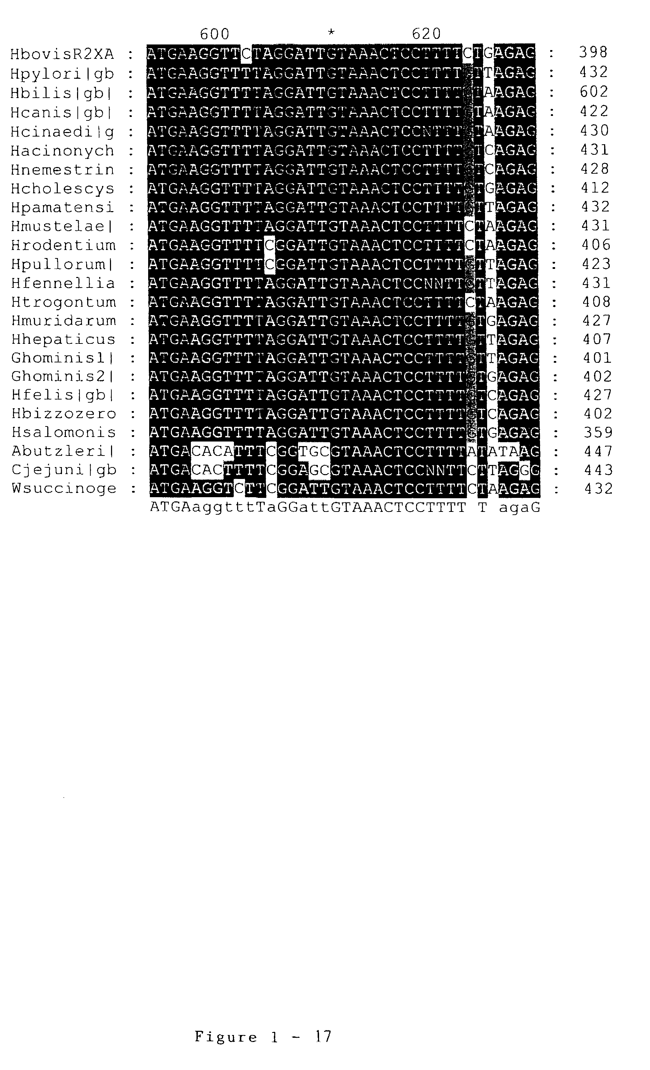

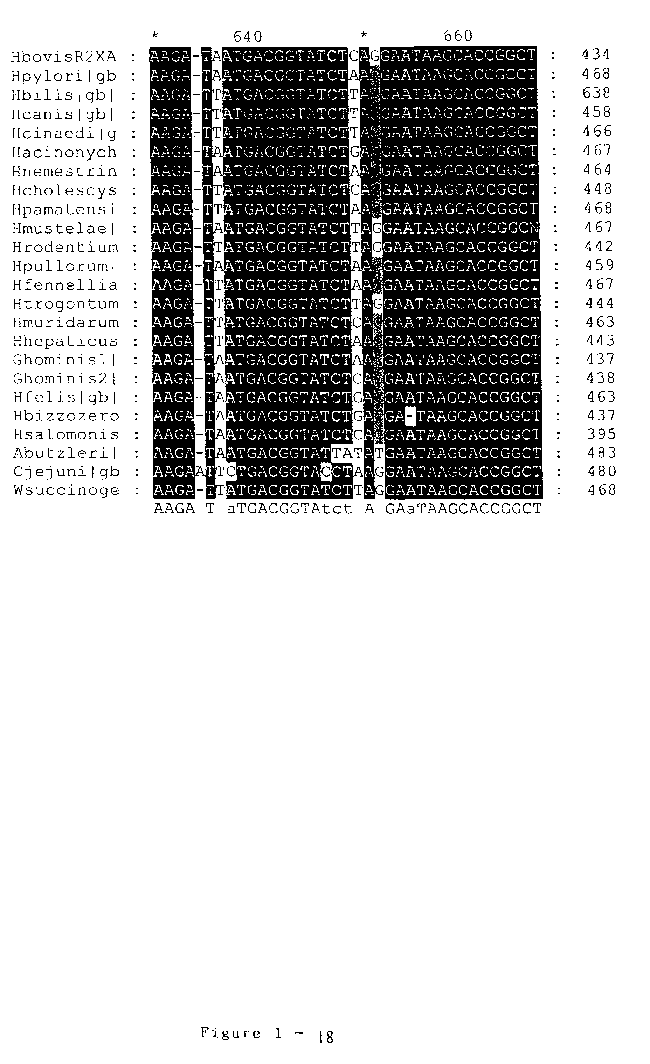

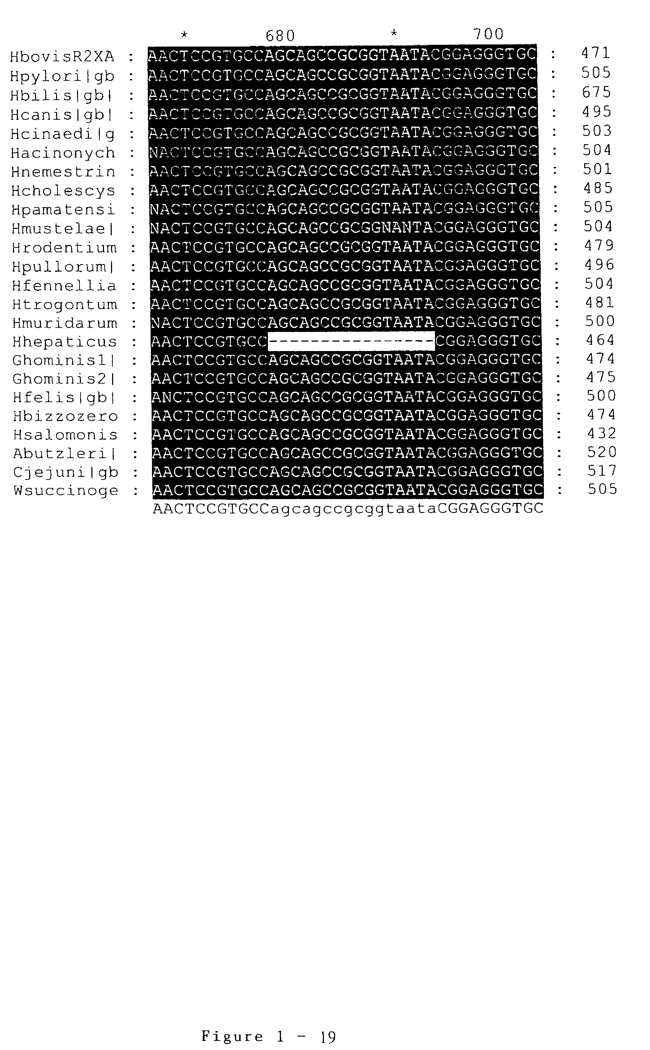

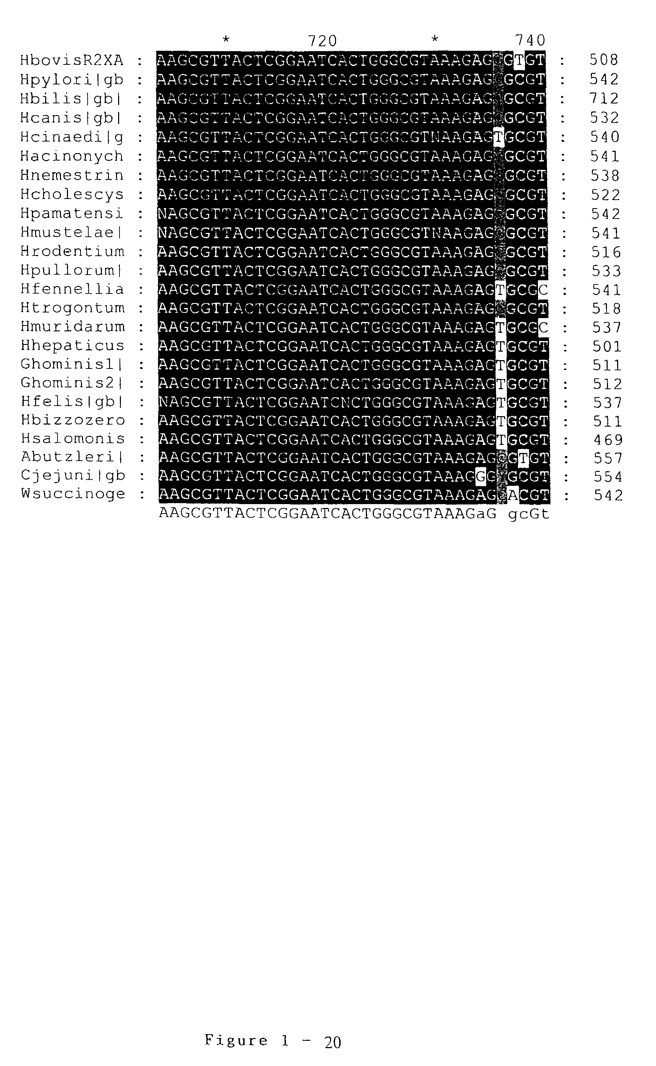

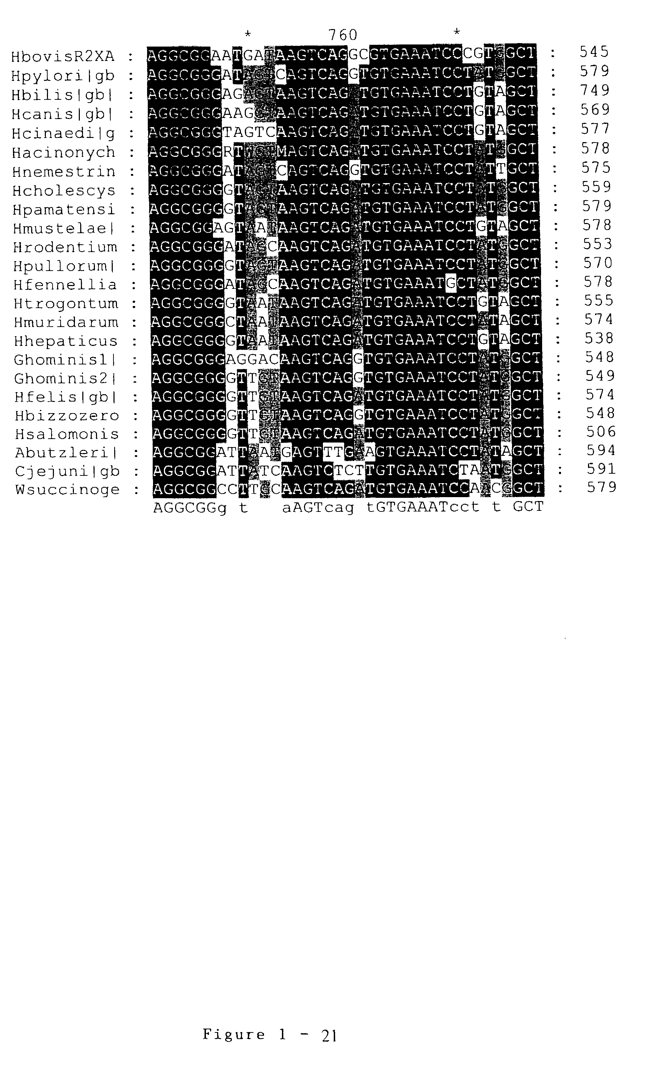

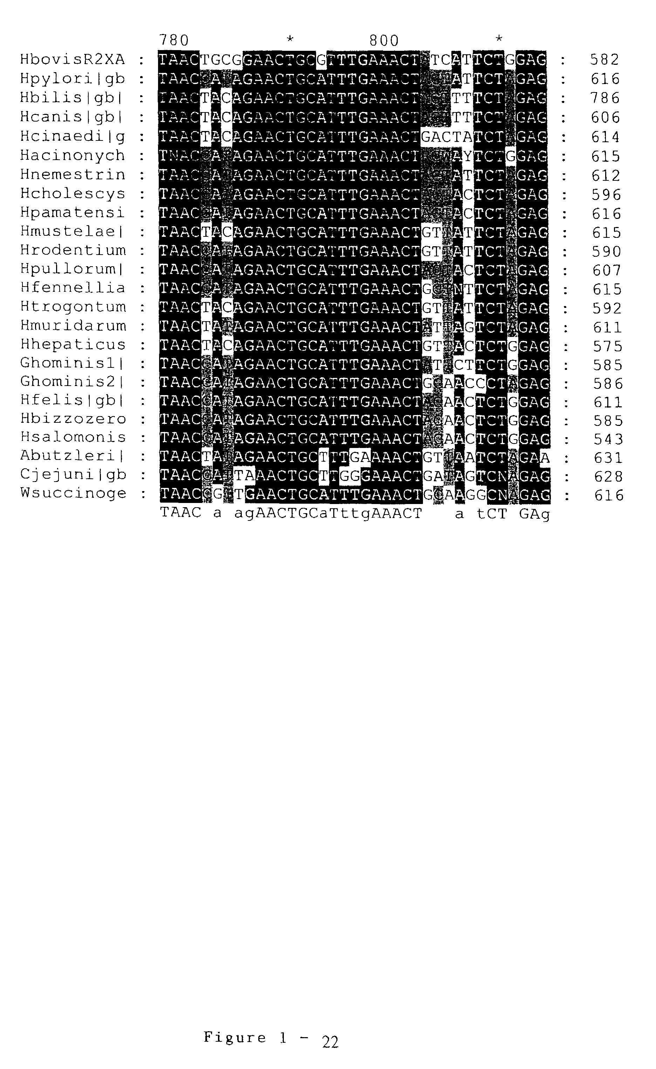

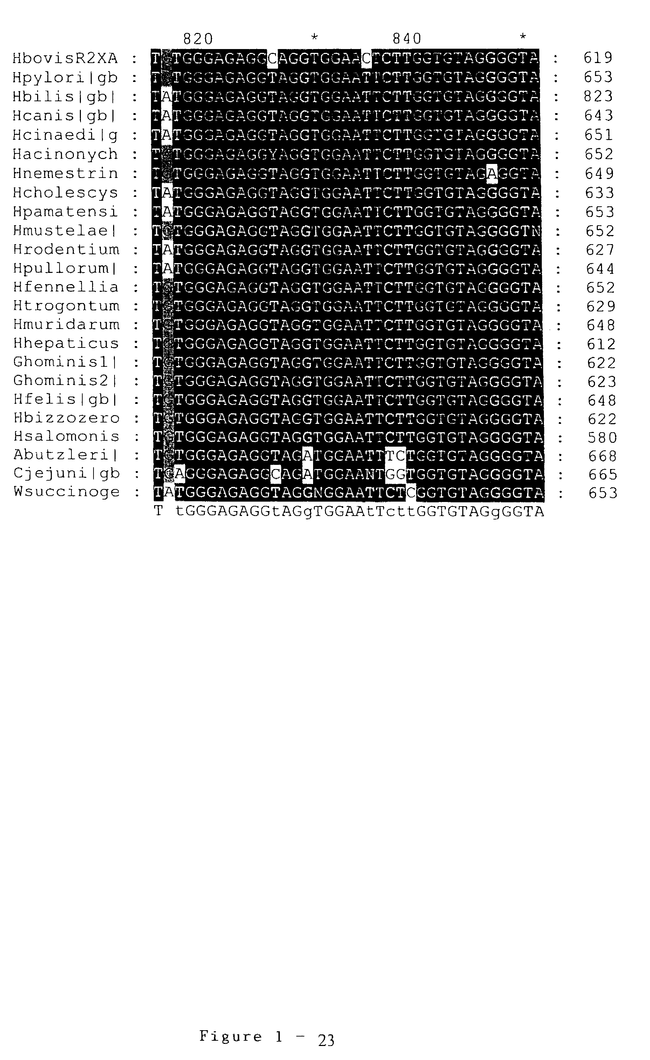

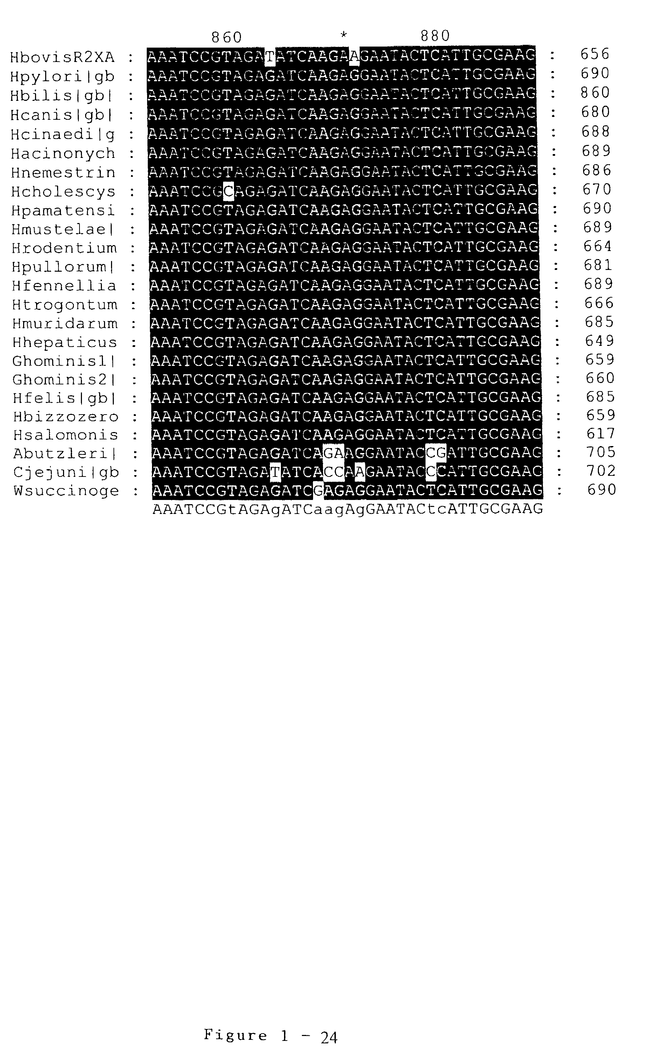

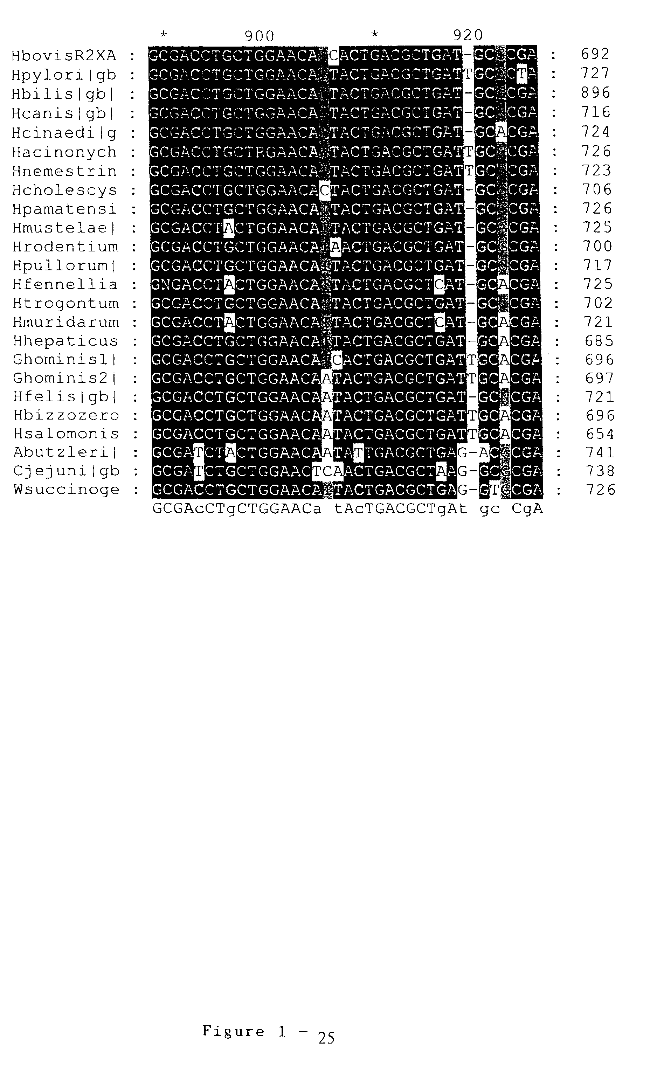

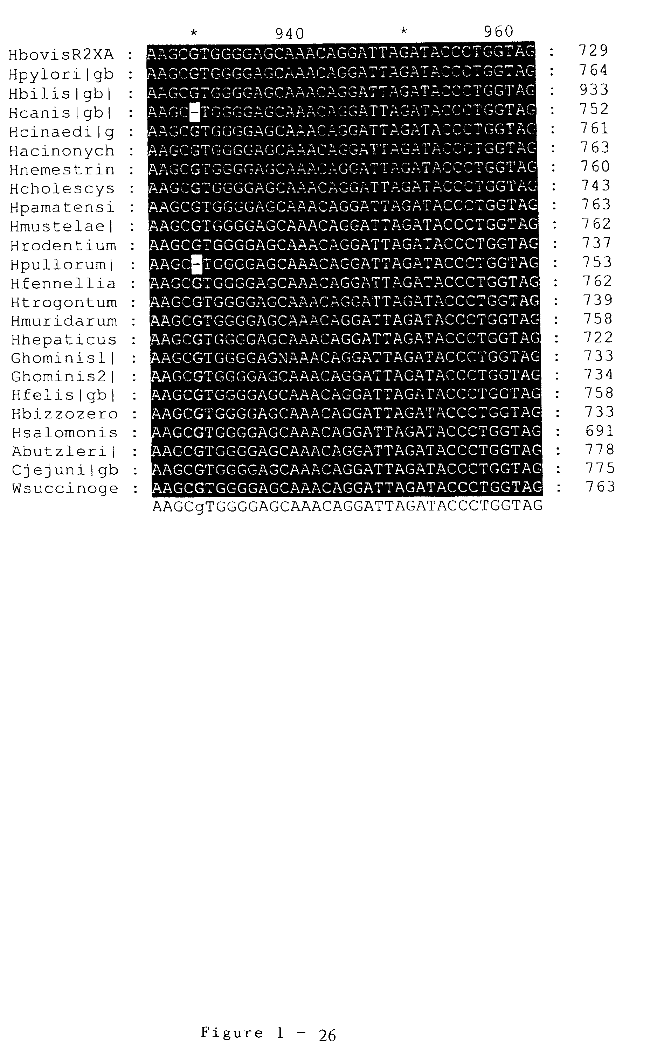

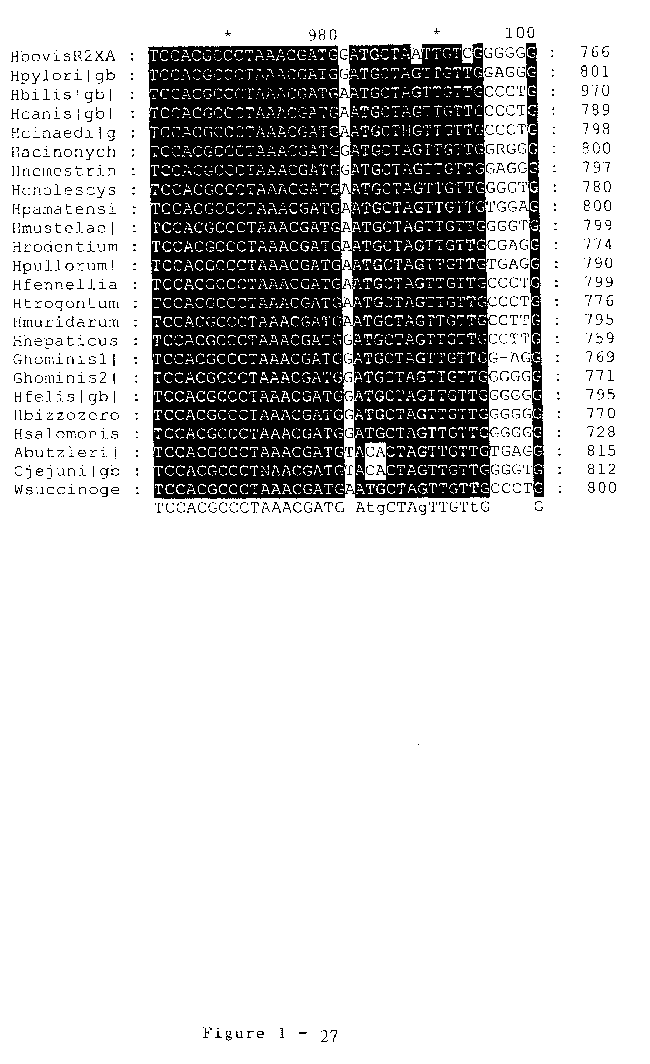

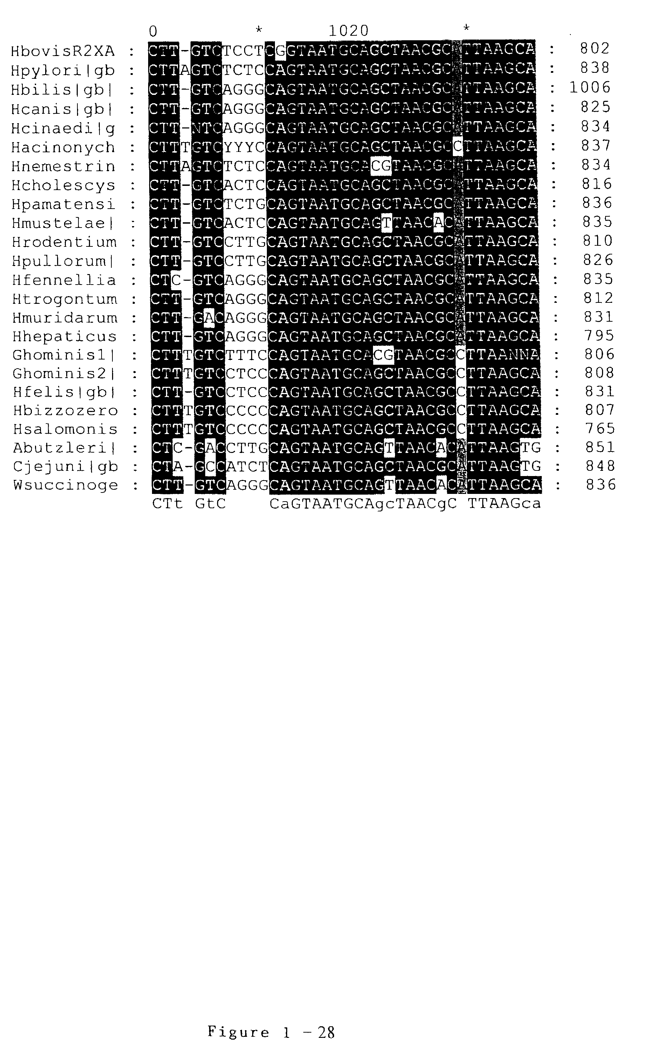

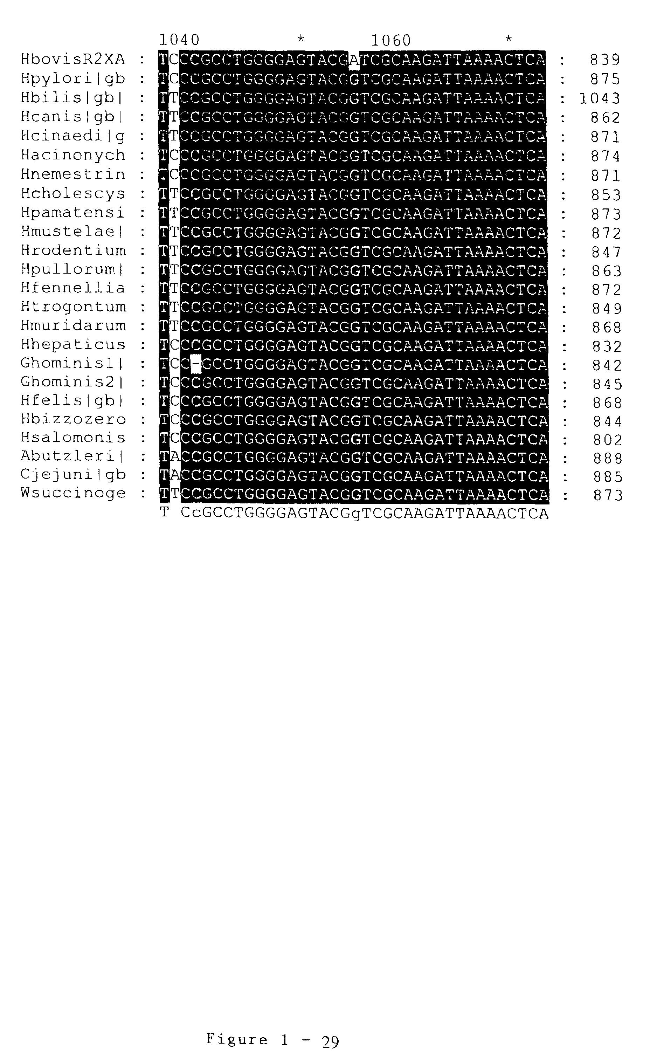

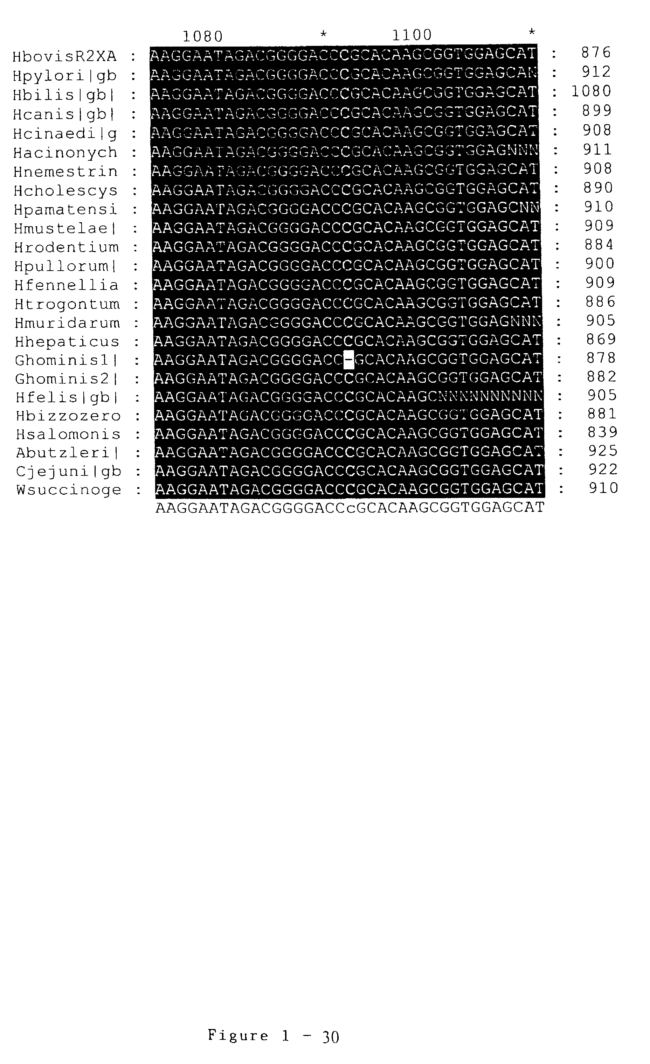

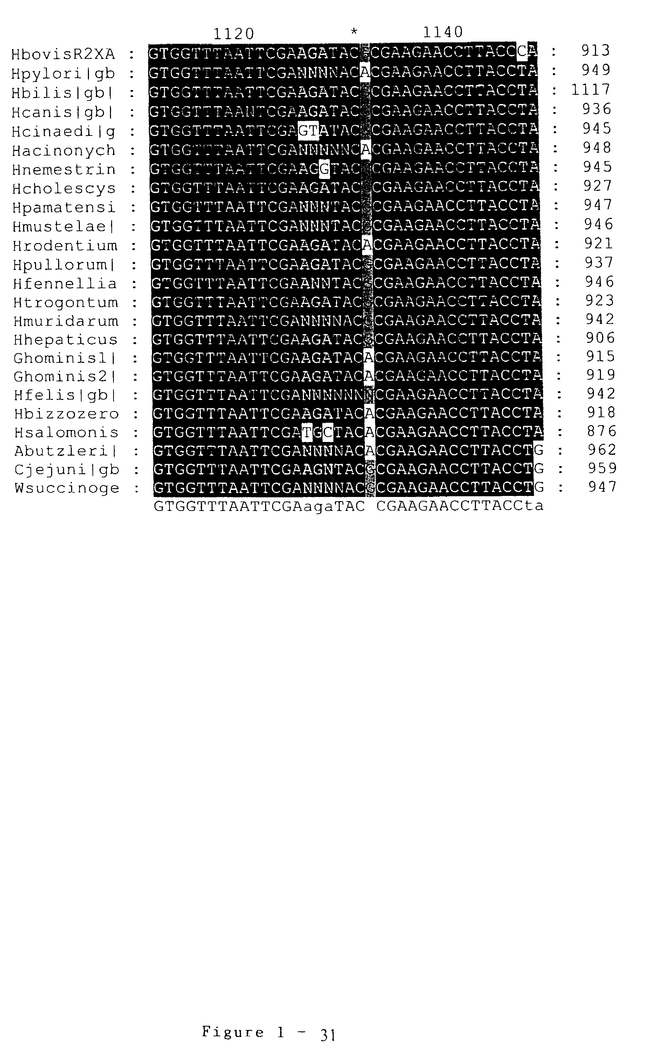

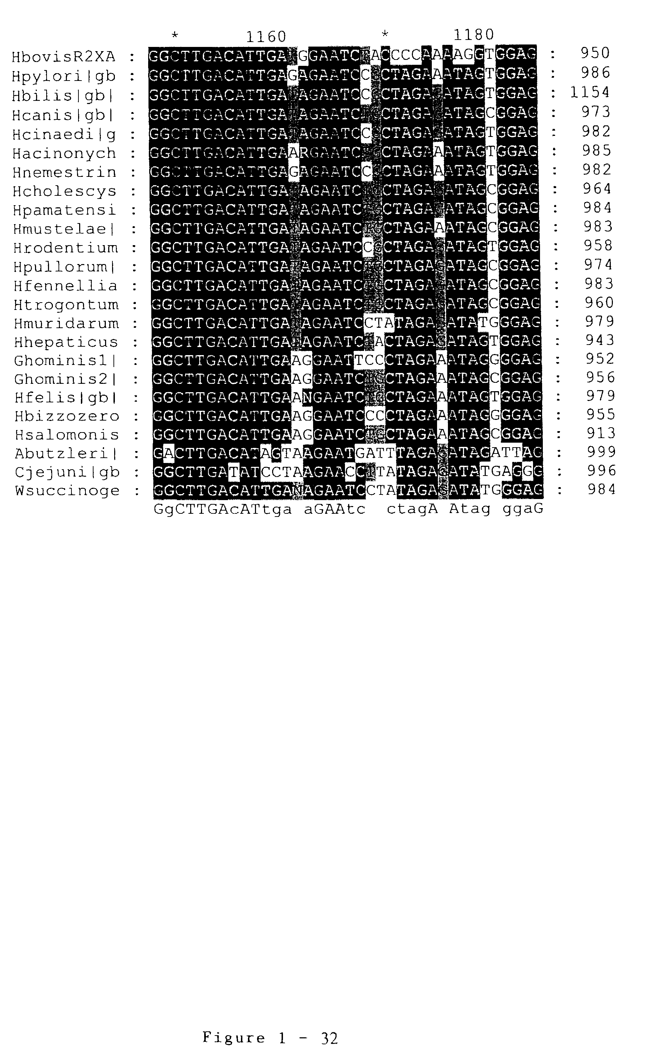

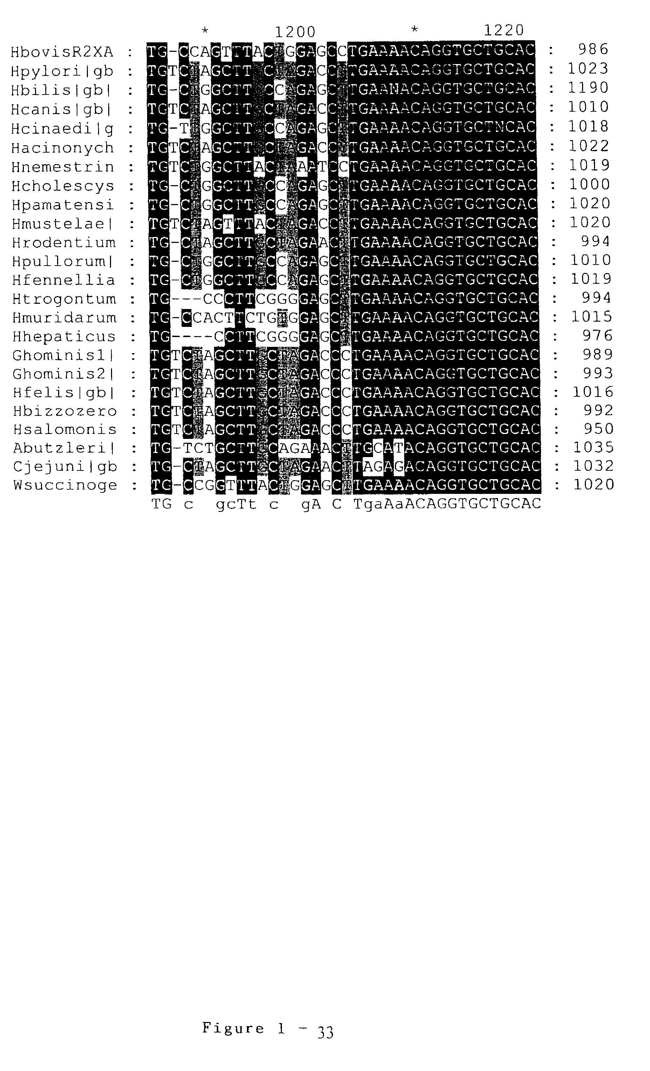

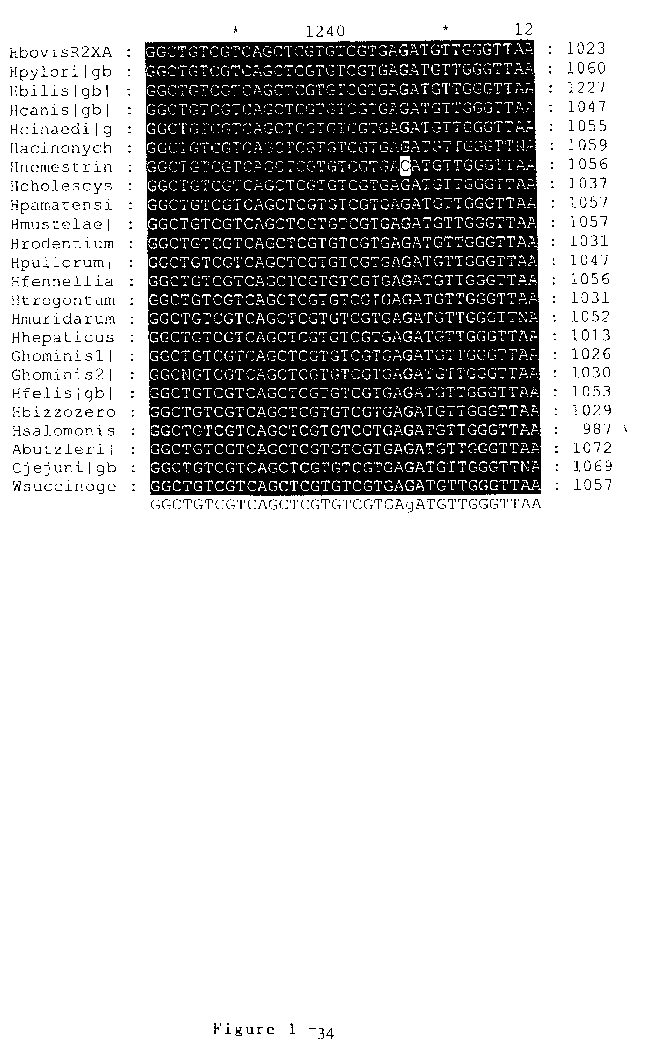

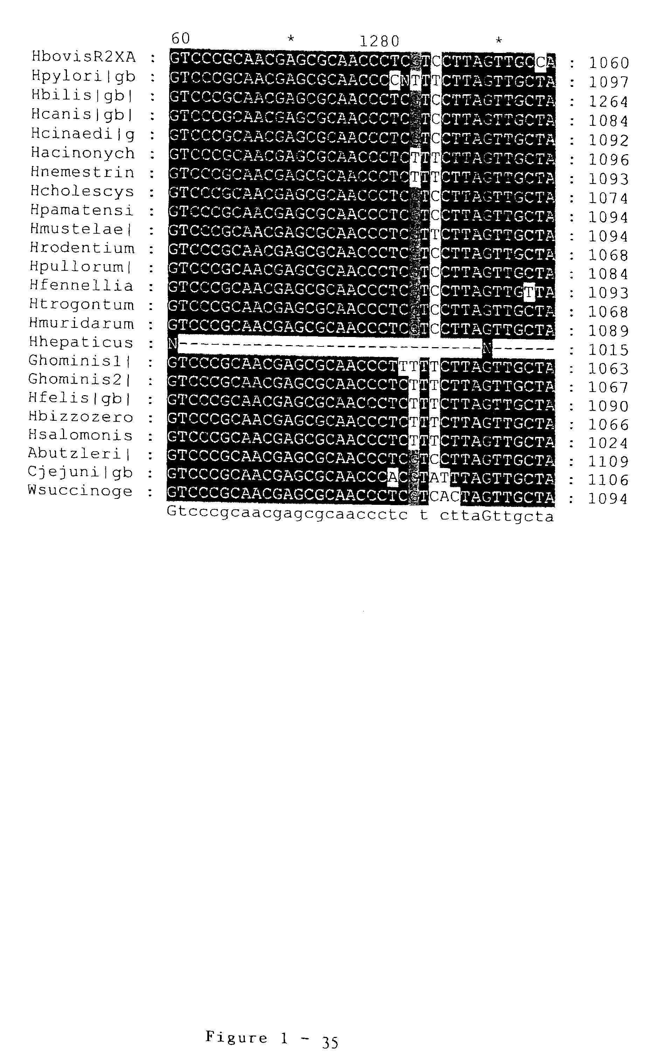

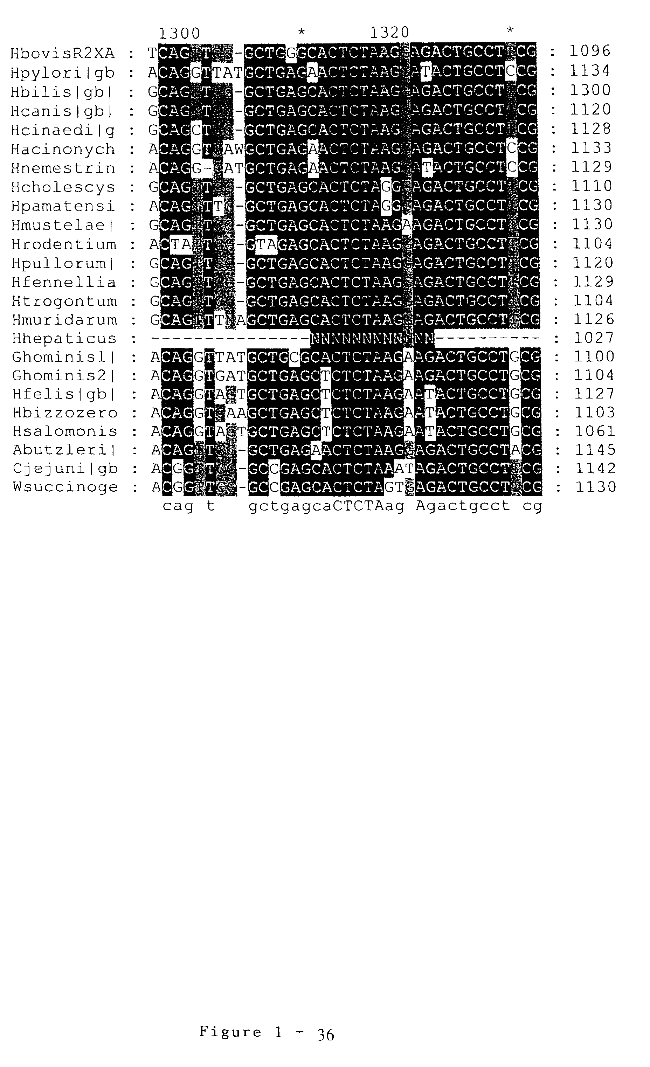

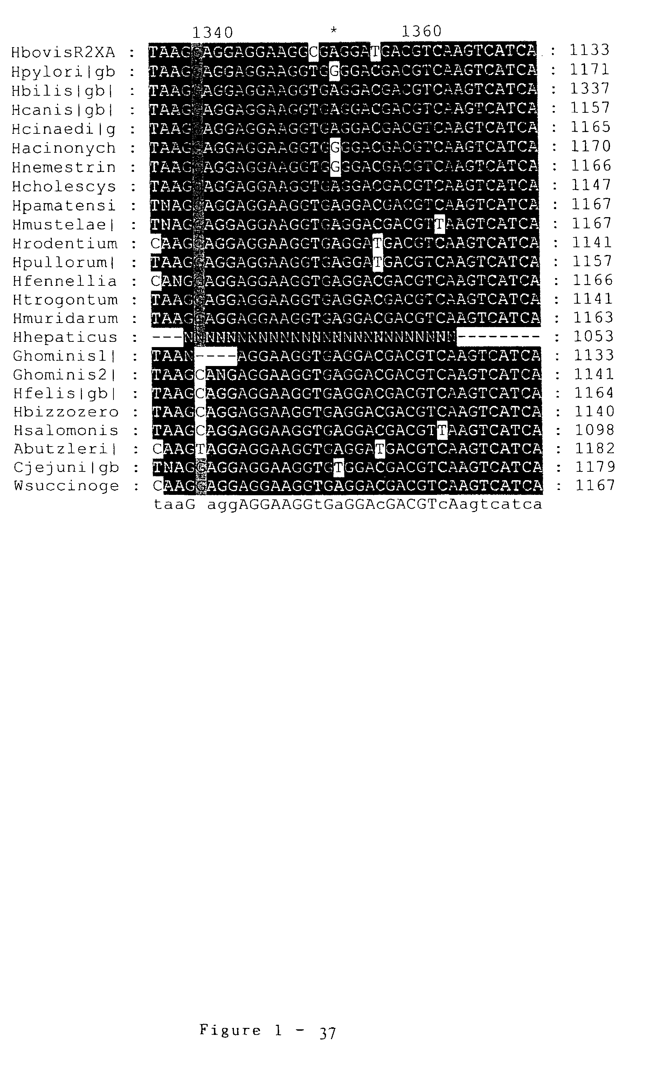

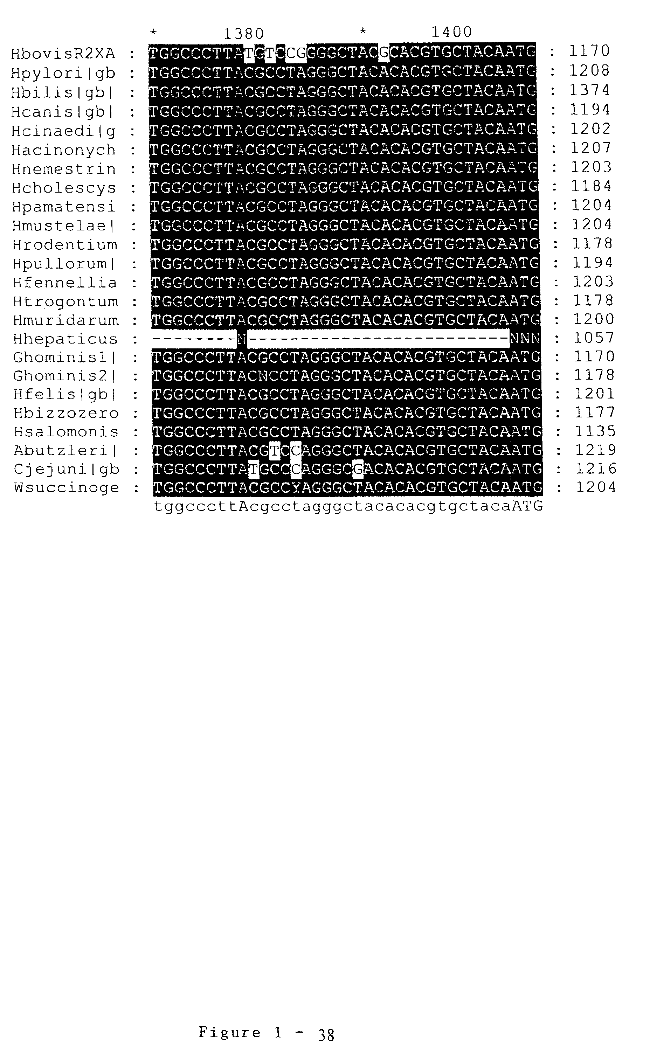

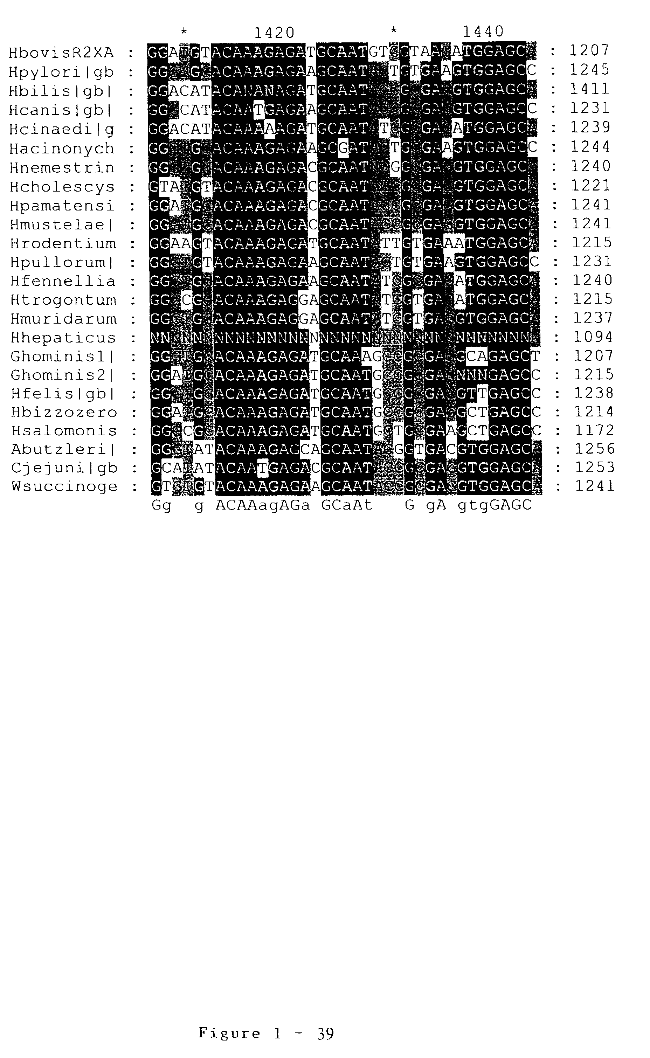

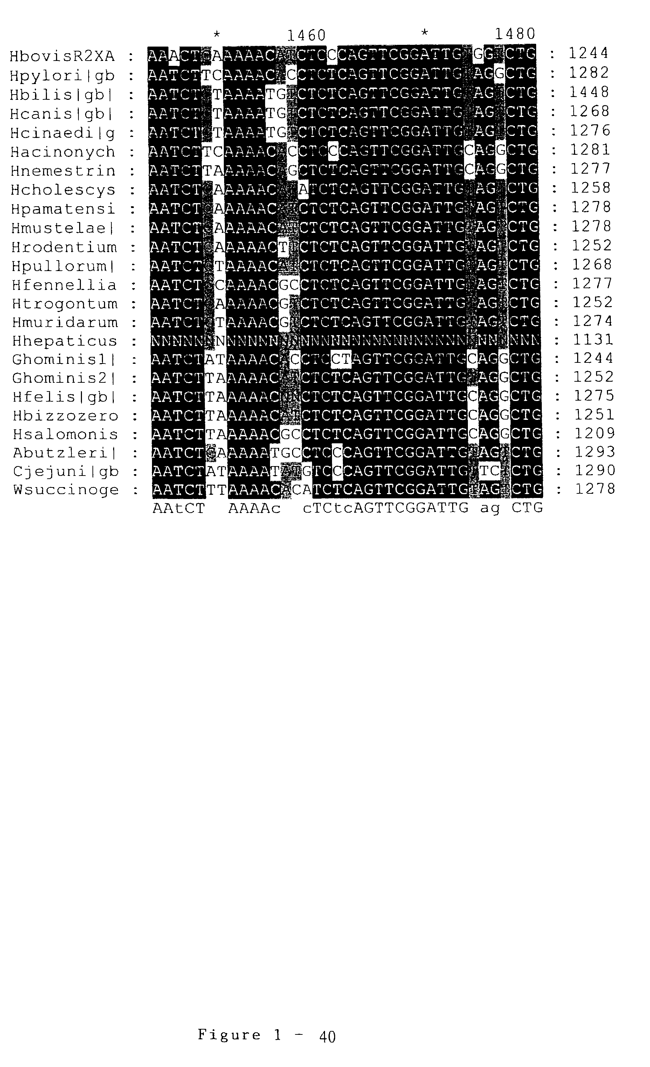

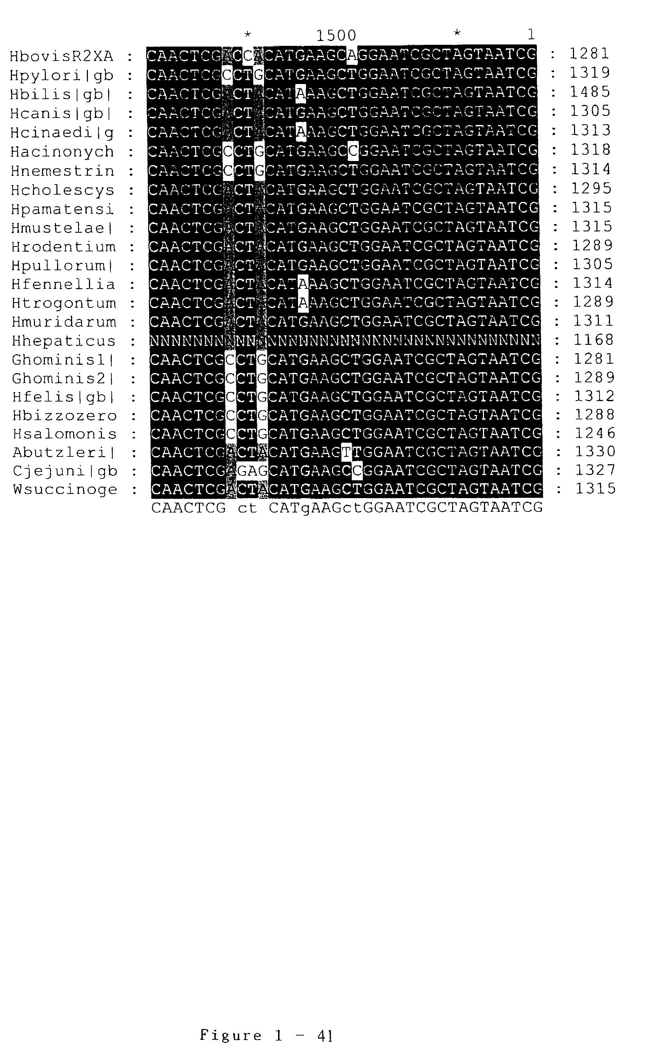

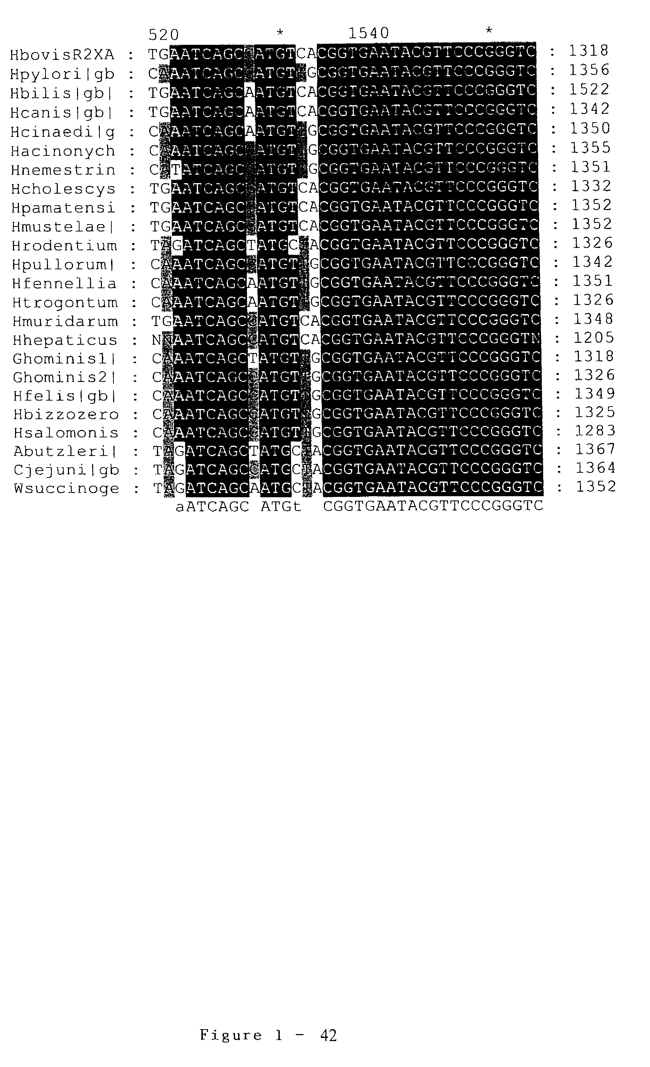

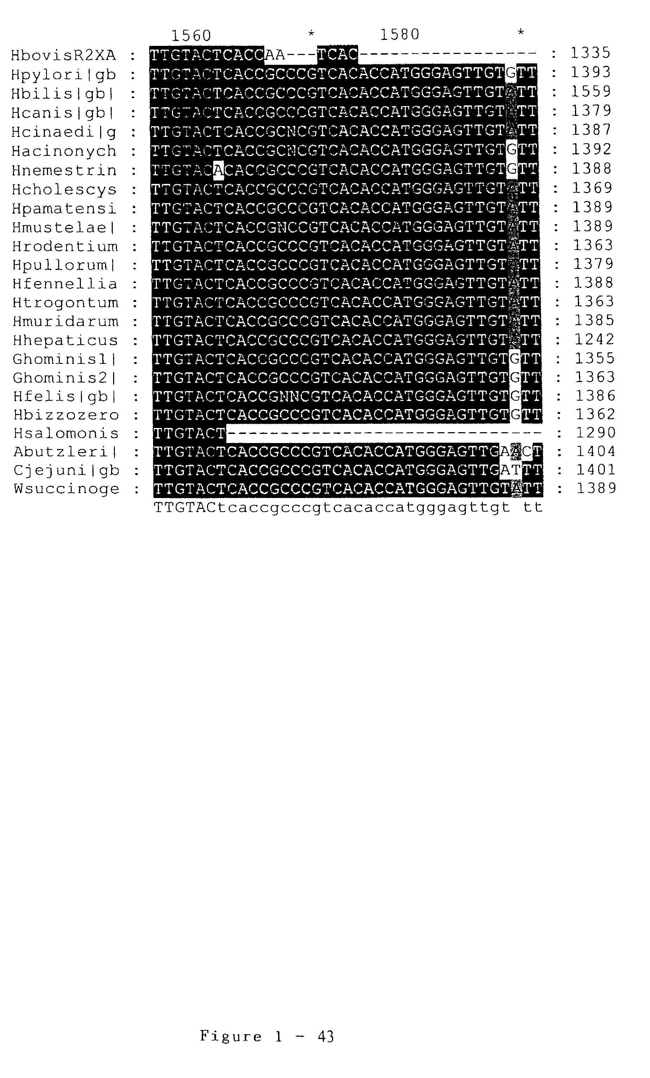

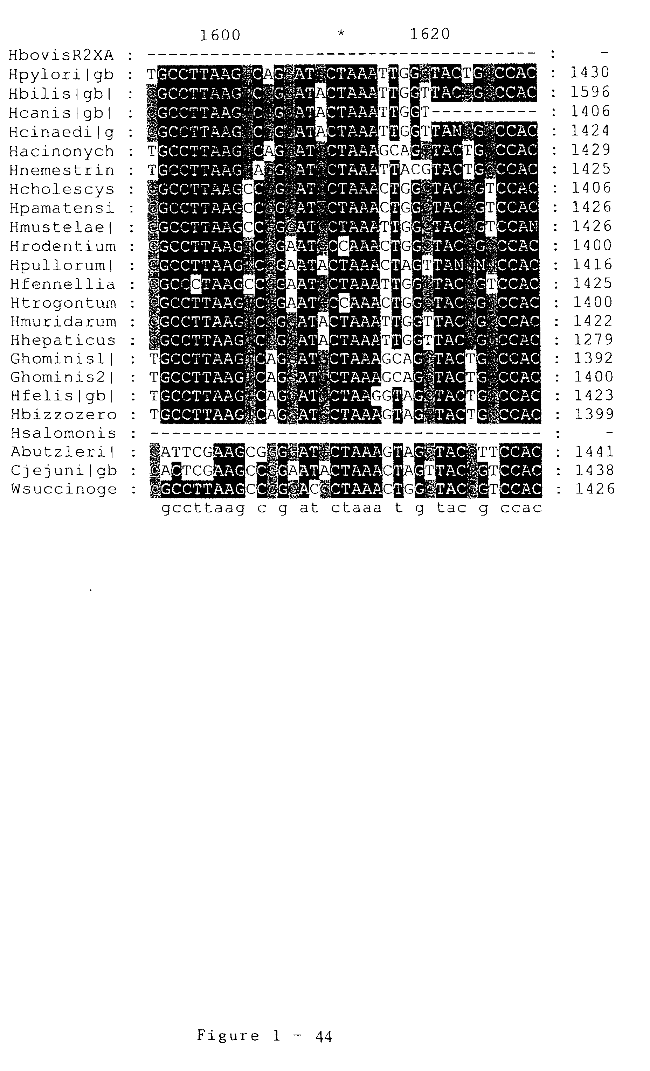

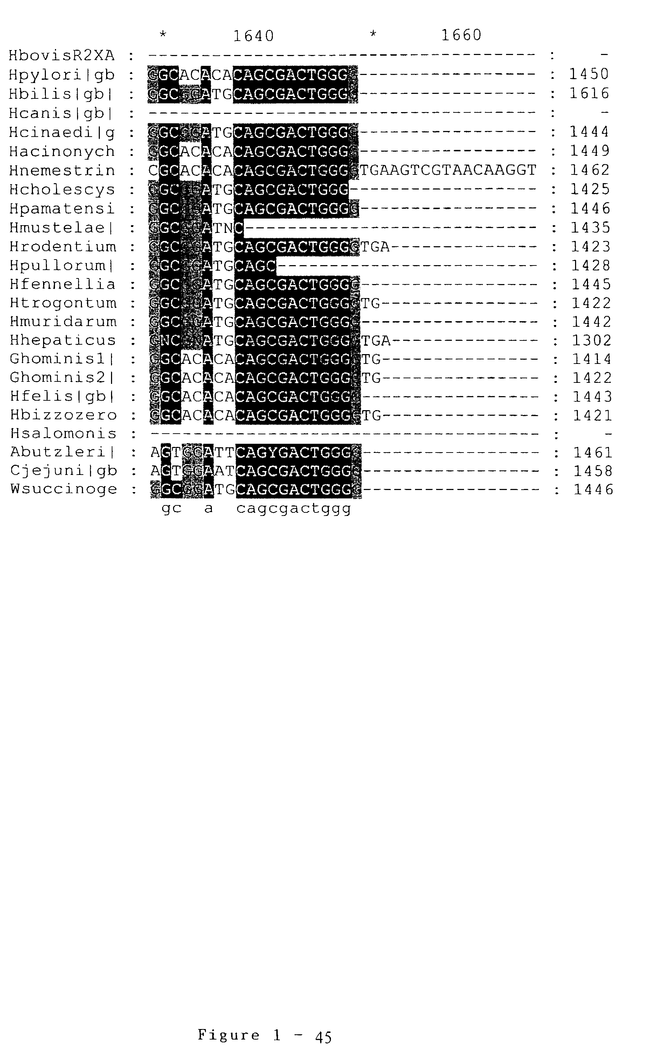

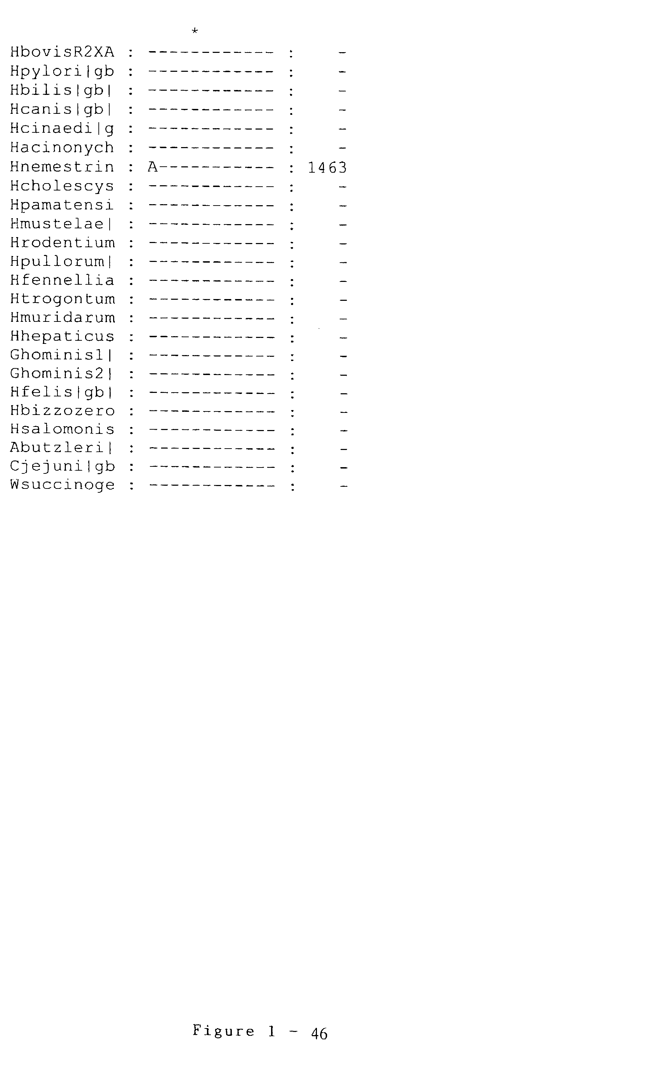

Preferred sequences according to the present invention are set out in Figures 1, 2, 4 and 5: SEQ ID NO 1 to 2 and 15 to 24. Also unique parts and fragments of these sequences are part of the present invention. Preferred unique parts are set out in Table 2.

Since SEQ ID NO 2 shows 96.6% homology to its closest found validly named neighbour, the use of sequences of more than 96.6% homology to SEQ ID NO 1 for identification or typing of *Helicobacter* species is also within the scope of the present invention. Preferably sequences of more than 97%, 97.5%, 98%, 98.5%, 99% or 99.5% homology to SEQ ID NO 2 are used for this goal.

According to another embodiment, the present invention relates to a part of an isolated polynucleic acid as defined above, more particularly part or a fragment of SEQ ID NO 1 or 2, wherein said part is unique to the polynucleic acid sequence it is derived from.

According to the present invention, the term "unique" implies that at least one nucleotide of the fragment or part is different from a nucleotide present at the same nucleotide position in a known 16S rRNA sequence or the corresponding gene. Such a nucleotide can be deduced theoretically by looking at an alignment of the new sequences of this invention with other closely related *Helicobacter* 16S rDNA gene nucleotide sequences (see Figures 1, 2, 4 and 5). Said type of nucleotides are unique to the sequence they are derived from. These fragments are thus not part of any known 16S rRNA or gene sequence encoding the same. The fragments according to this embodiment of the present invention may be of any length between 10 to the maximum number of nucleotides of SEQ ID NO 1 or 2 or its variants. Preferred lengths are 10, 15, 20, 25, 30, 35, 40, 45, 50, 55, 60, etc. nucleotides.

According to another embodiment, the present invention relates to a probe which specifically hybridizes to a polynucleic acid sequence as defined above.

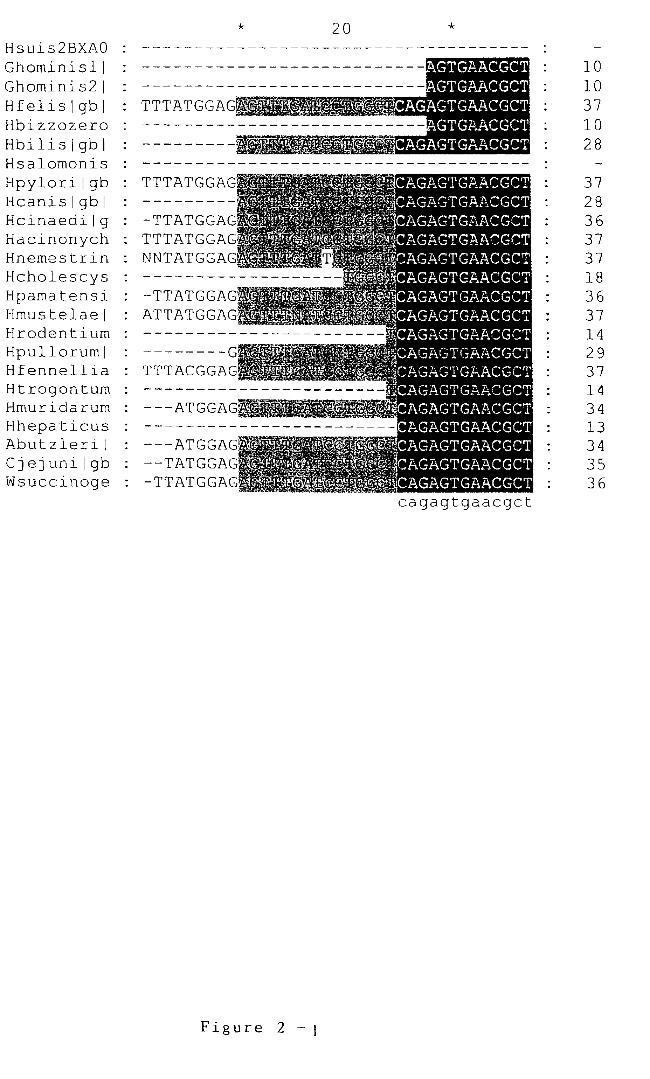

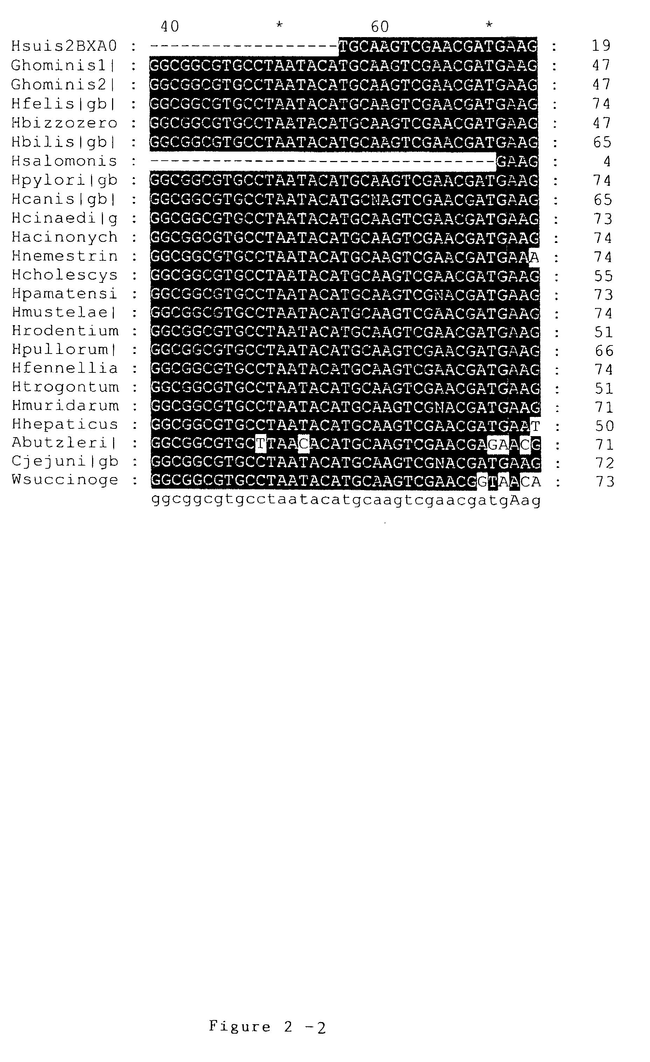

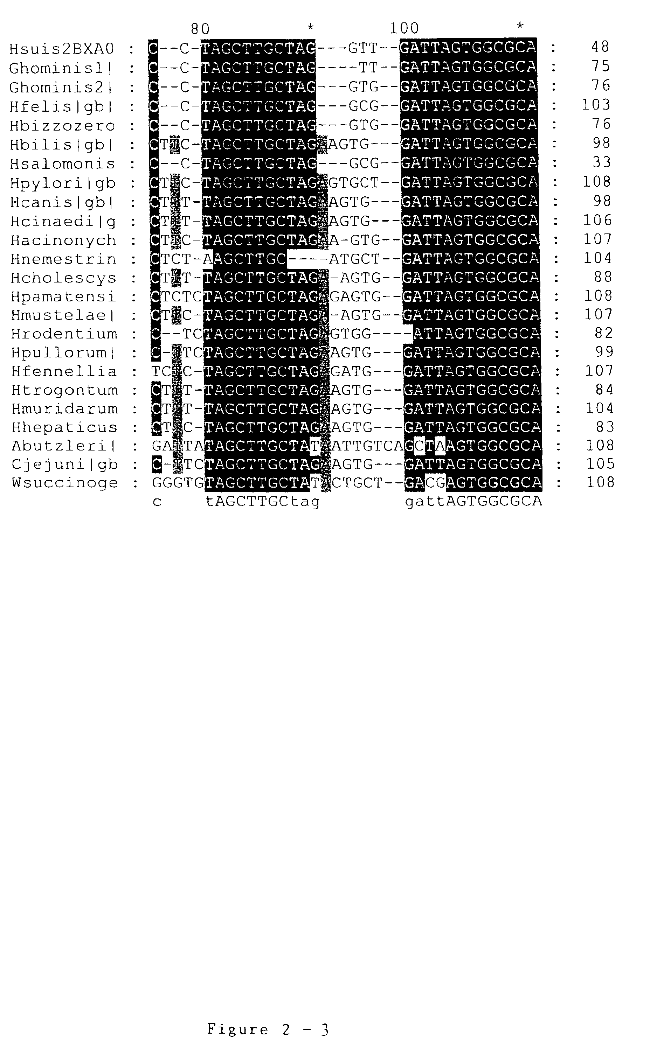

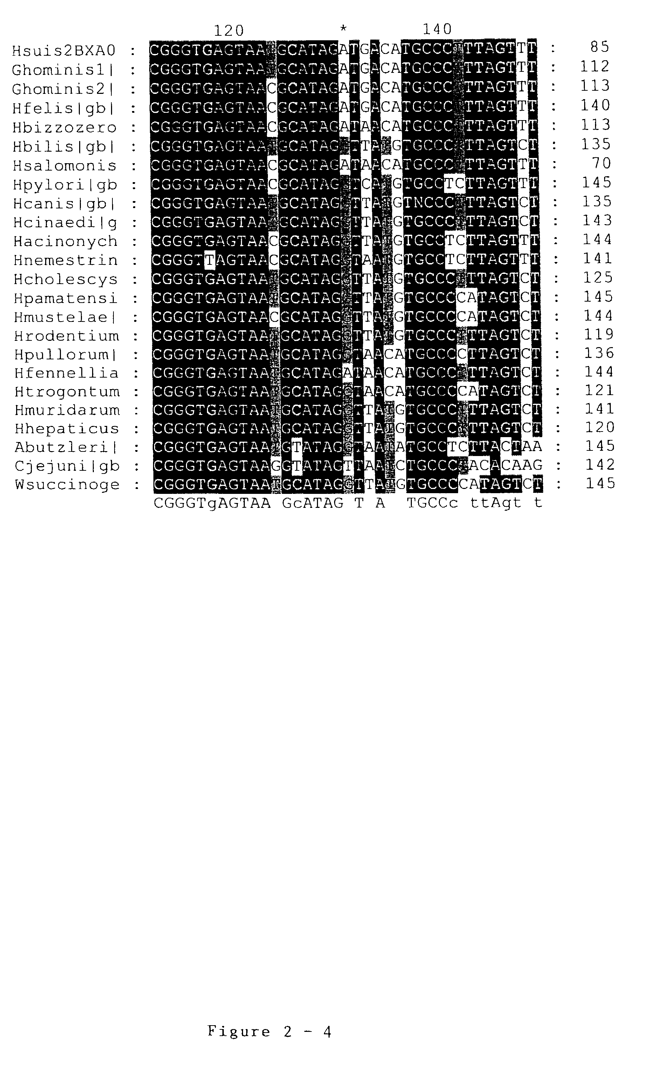

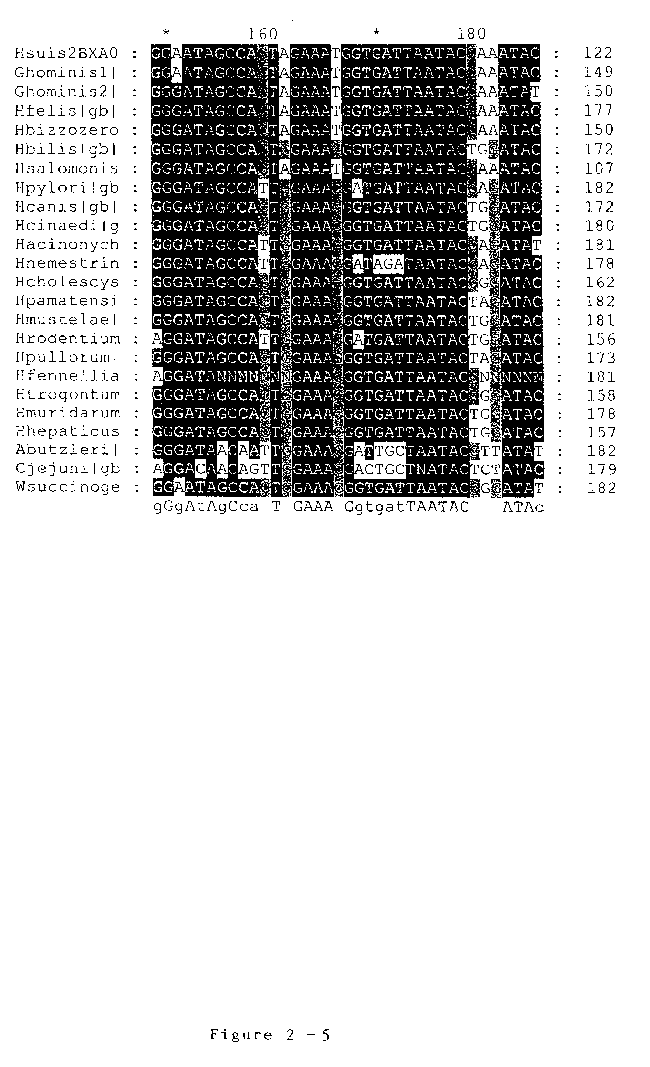

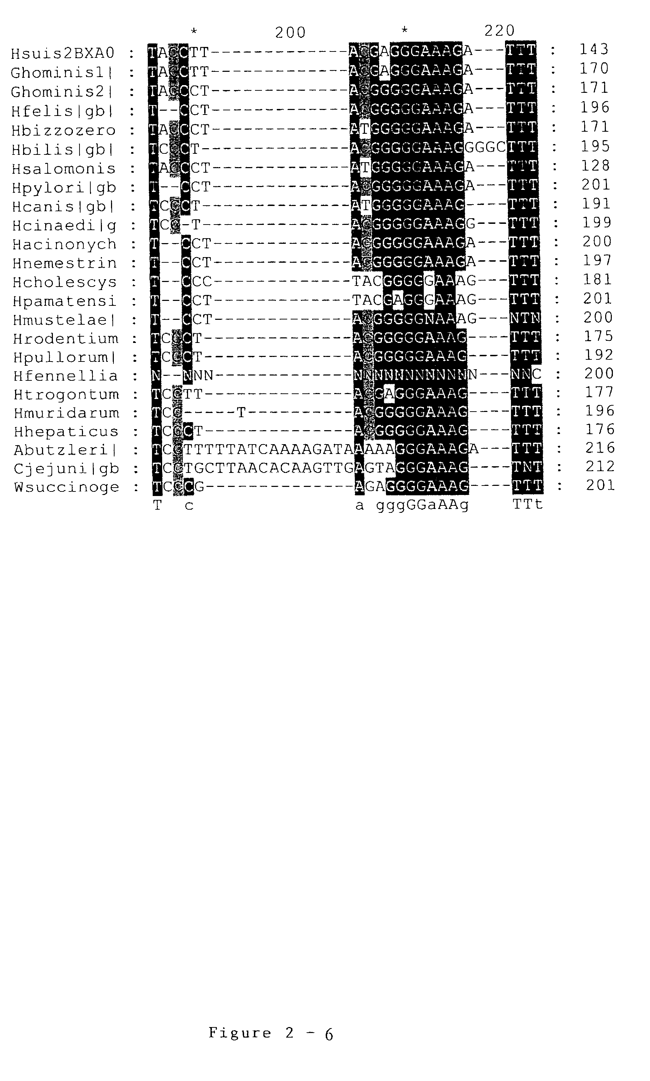

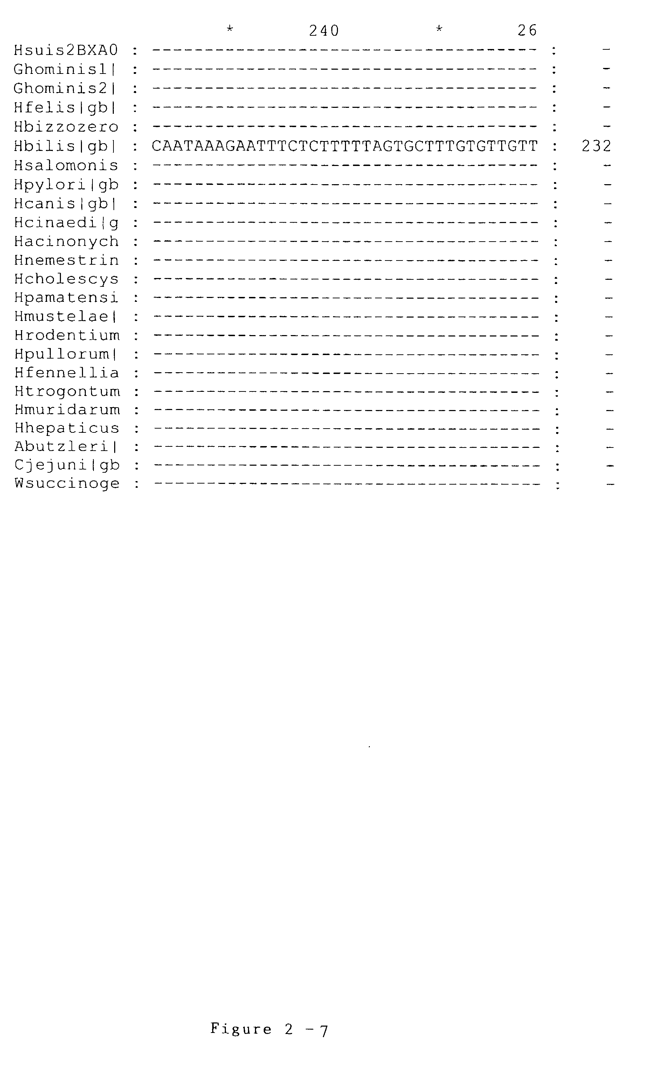

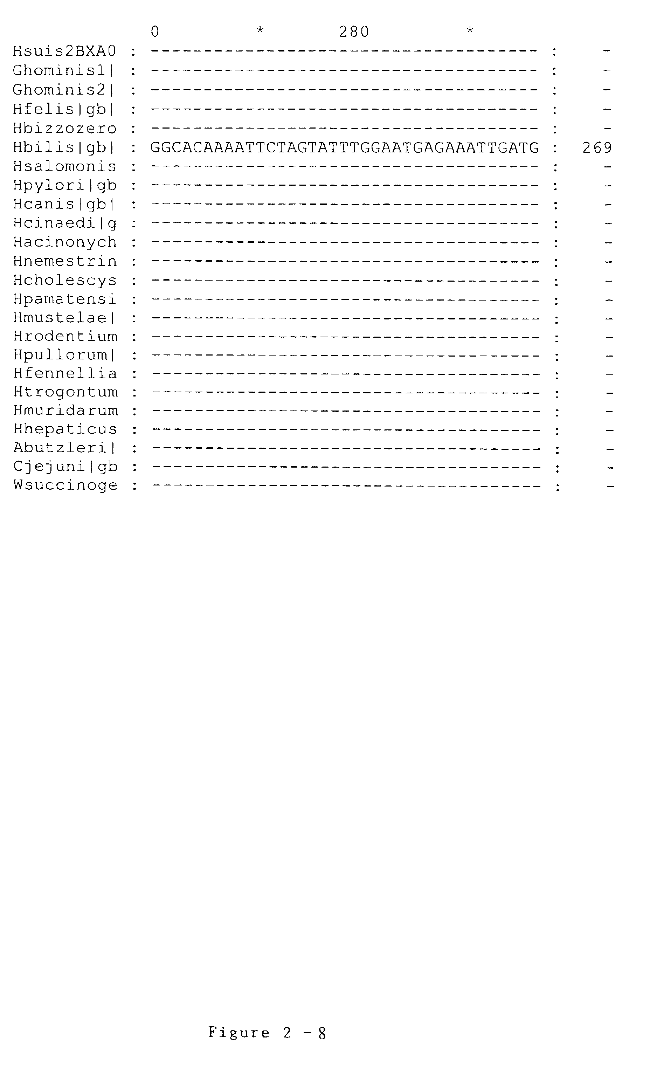

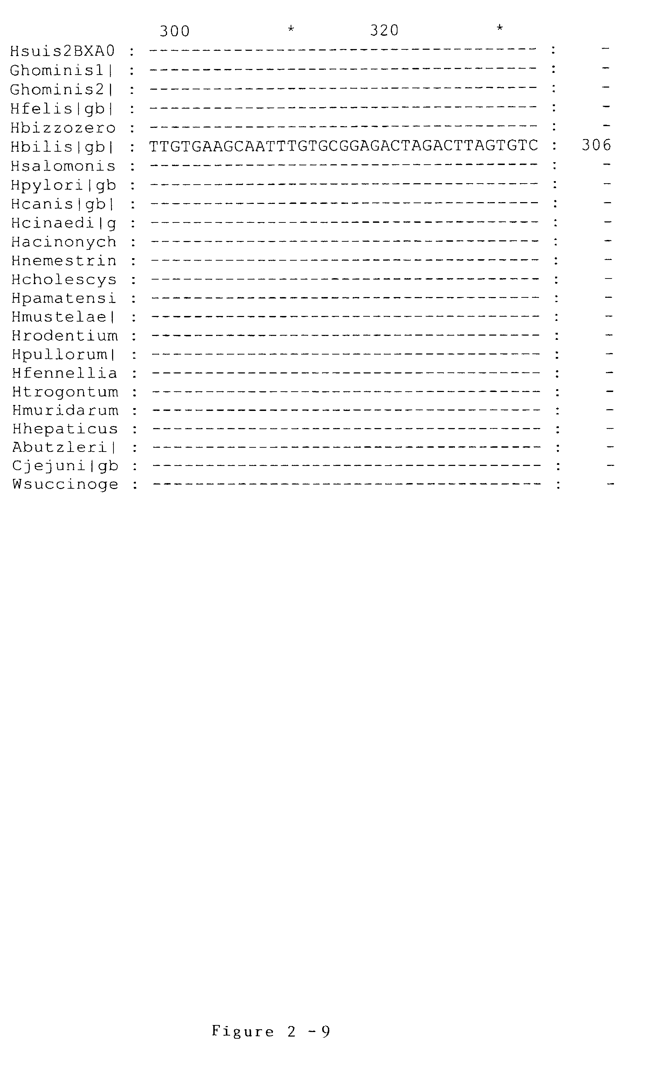

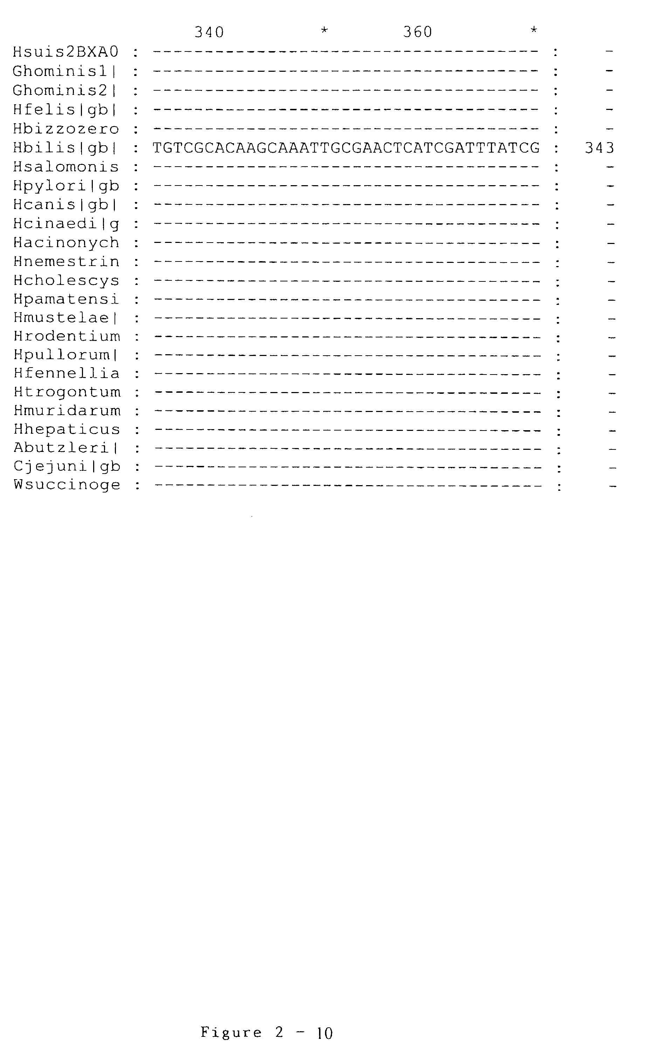

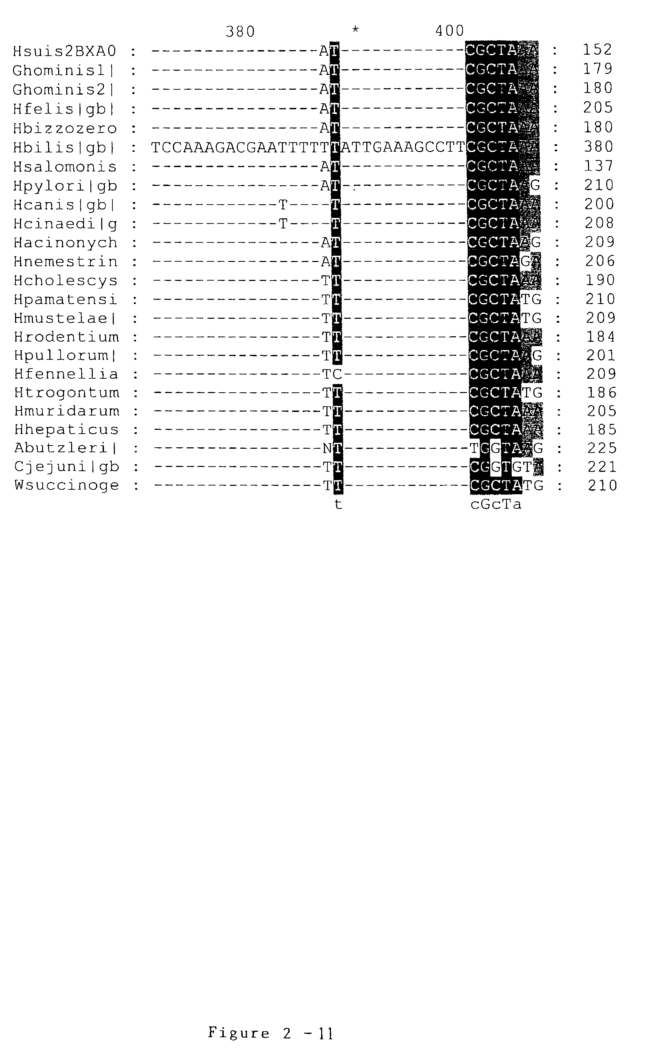

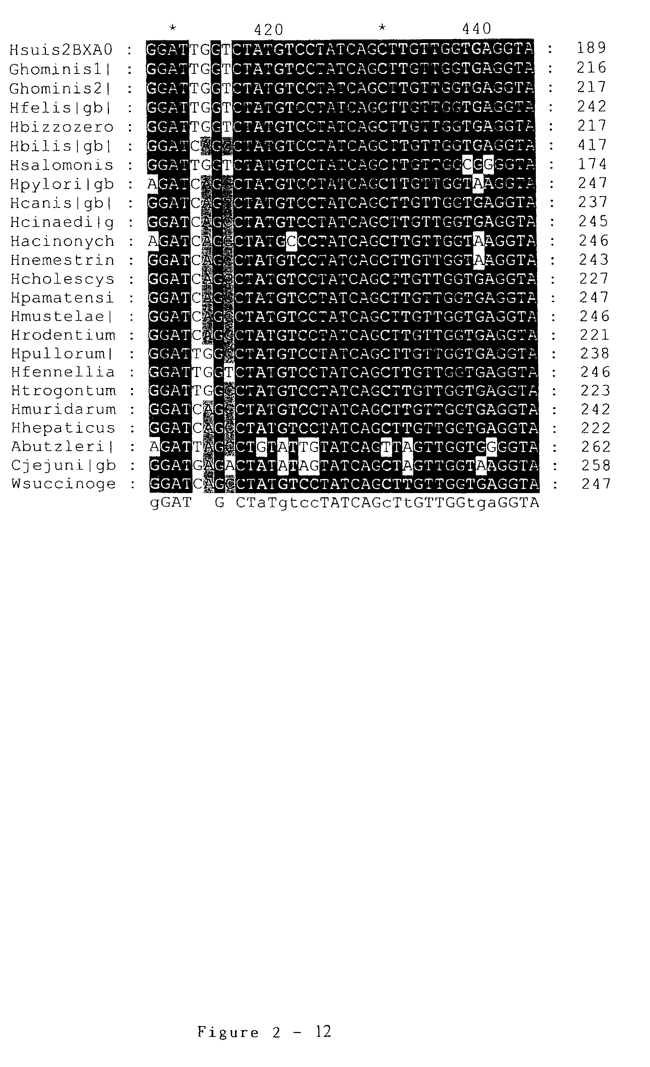

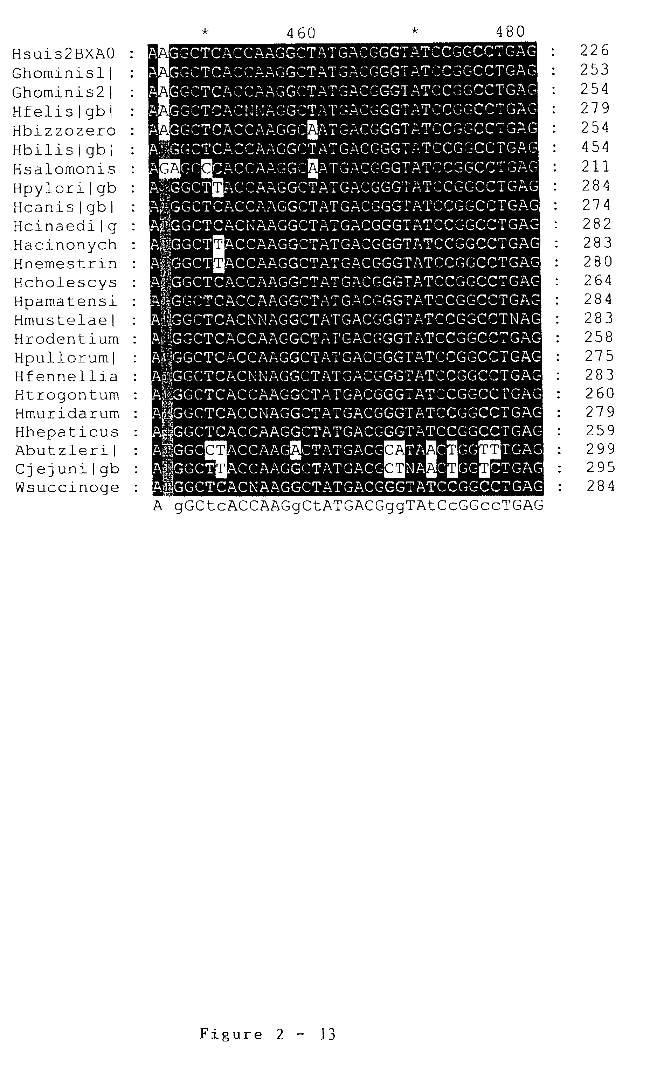

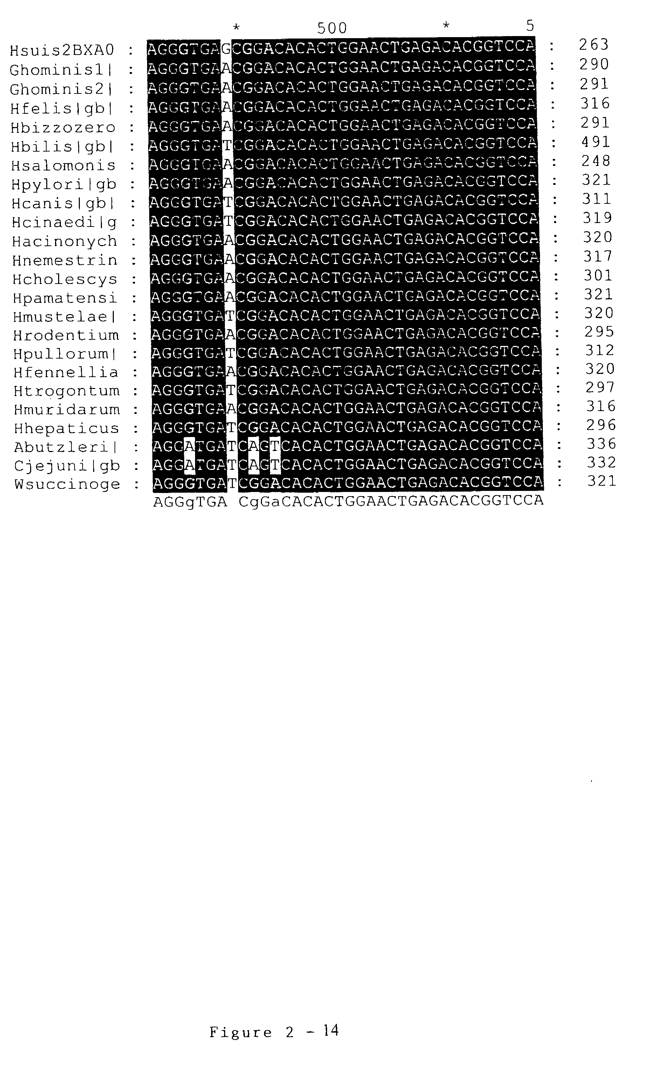

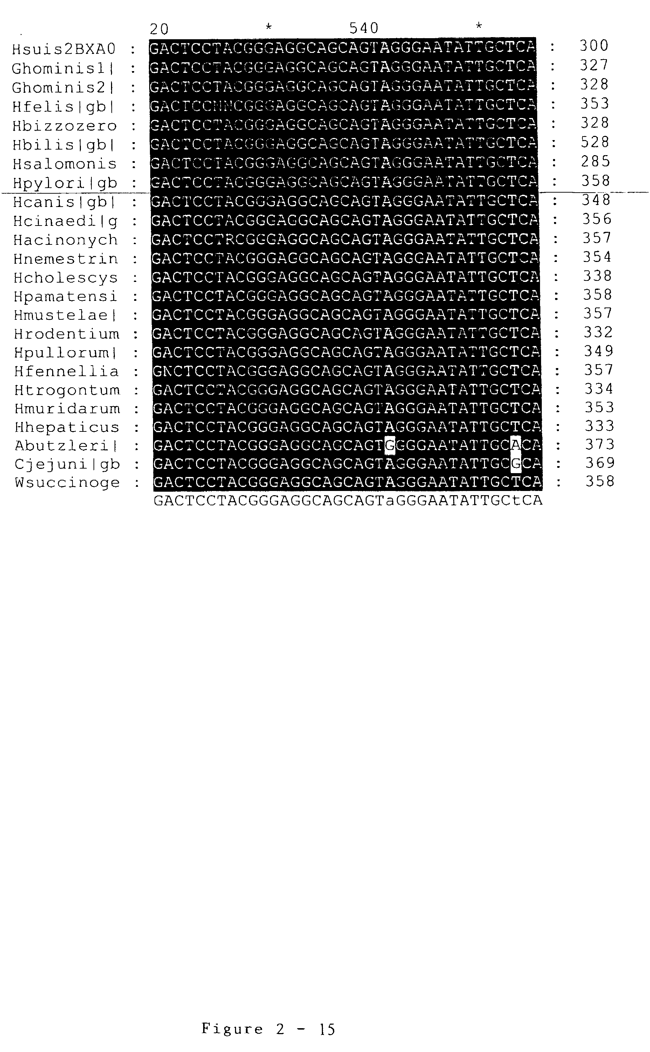

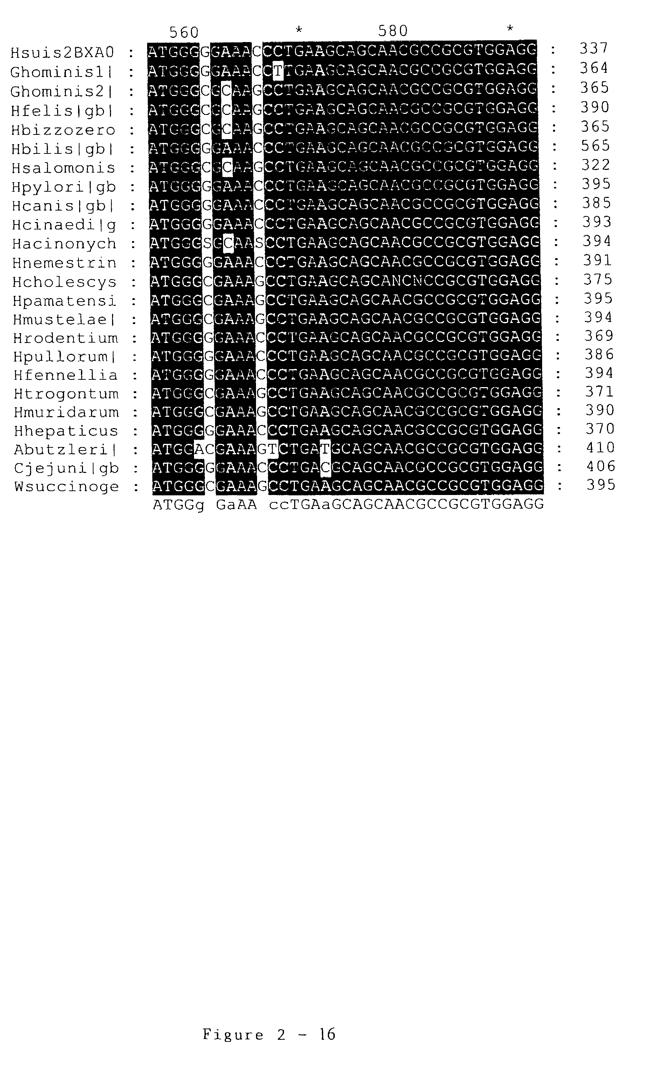

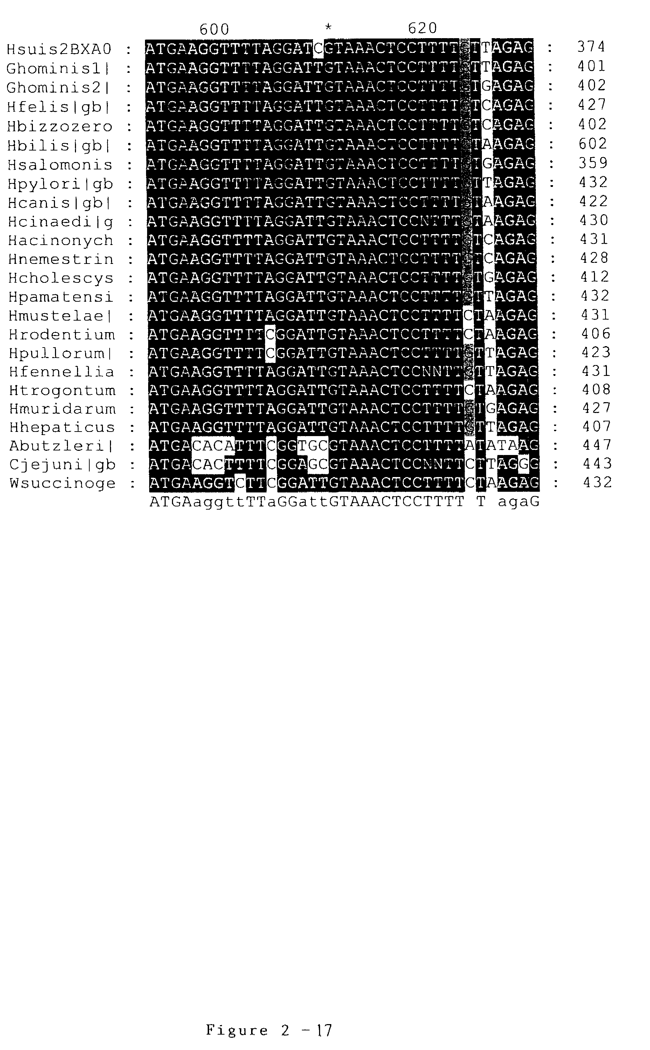

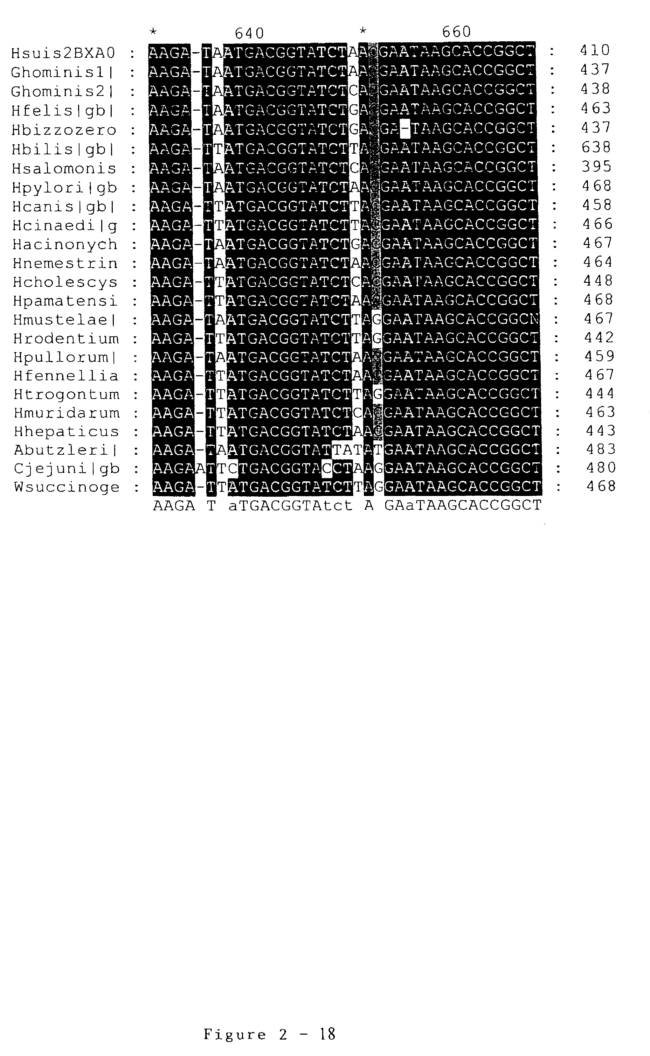

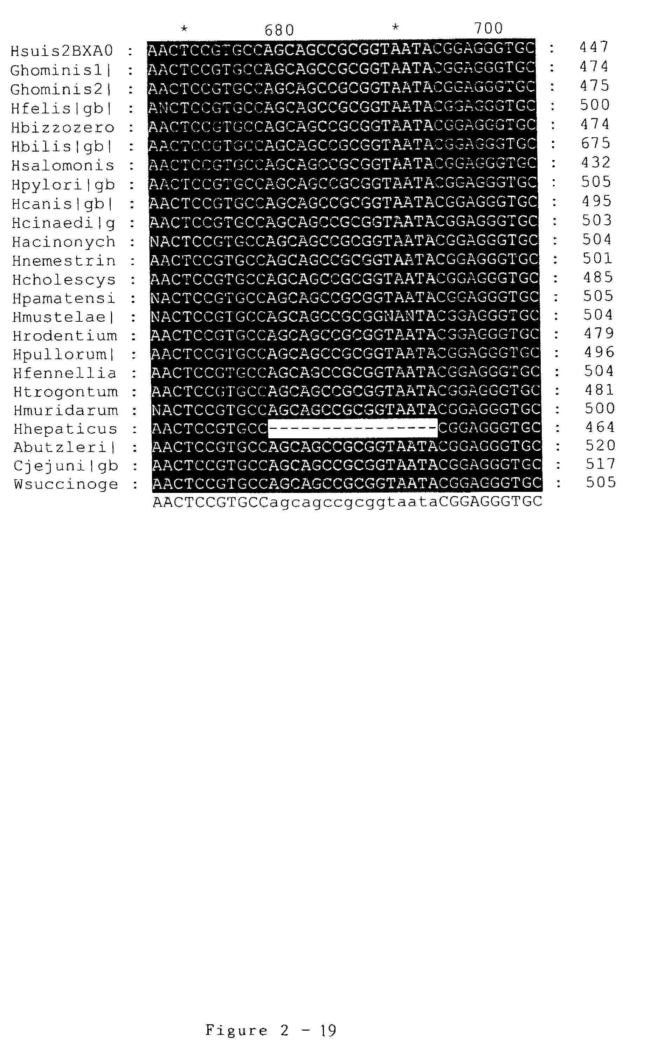

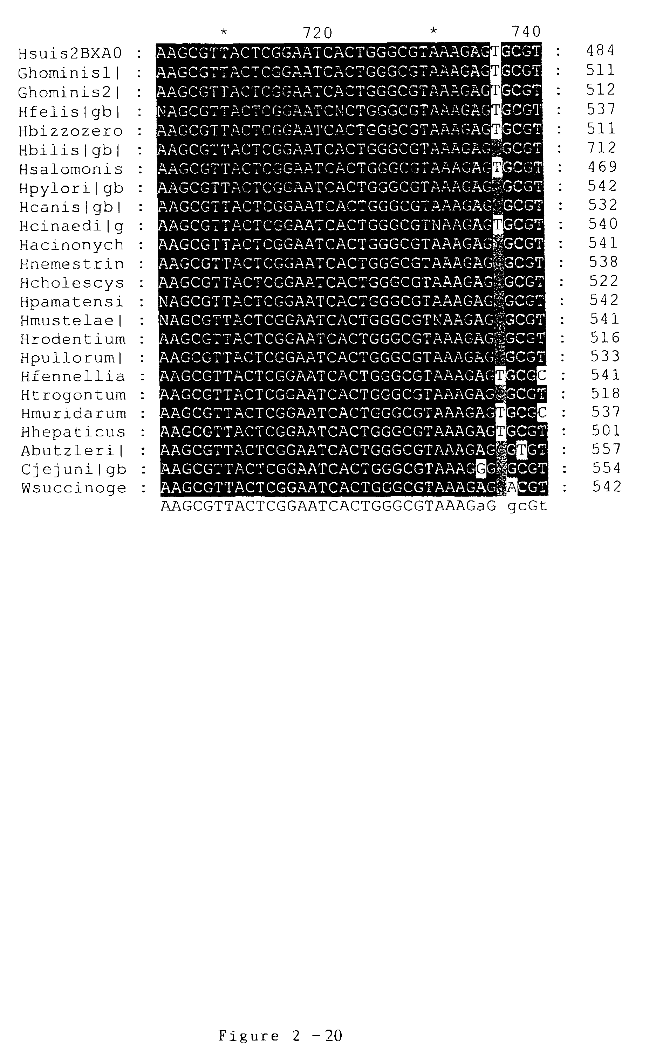

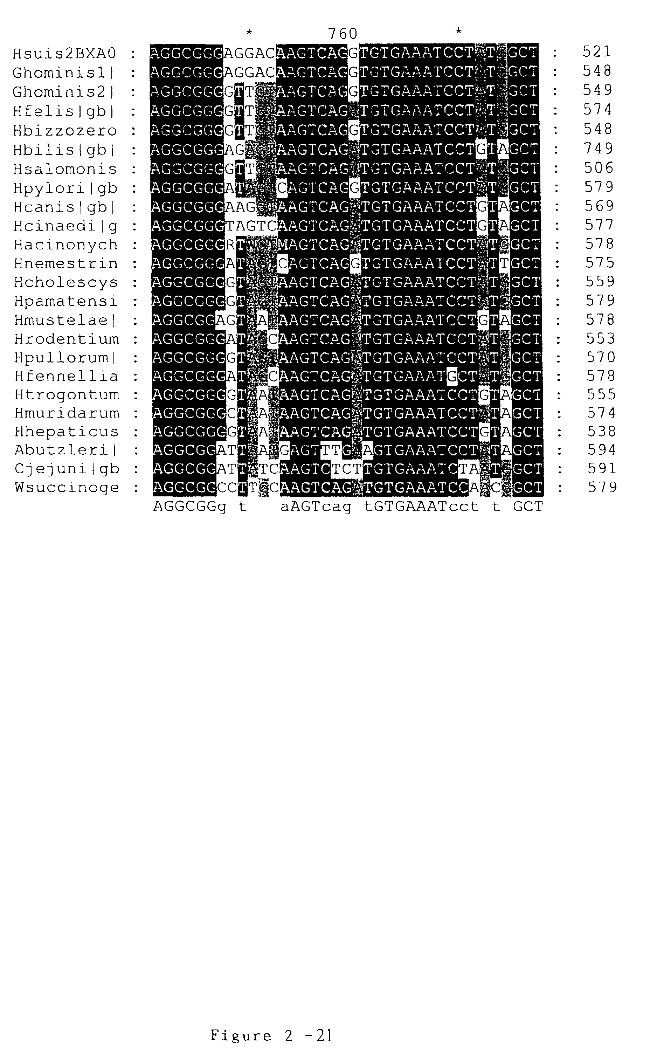

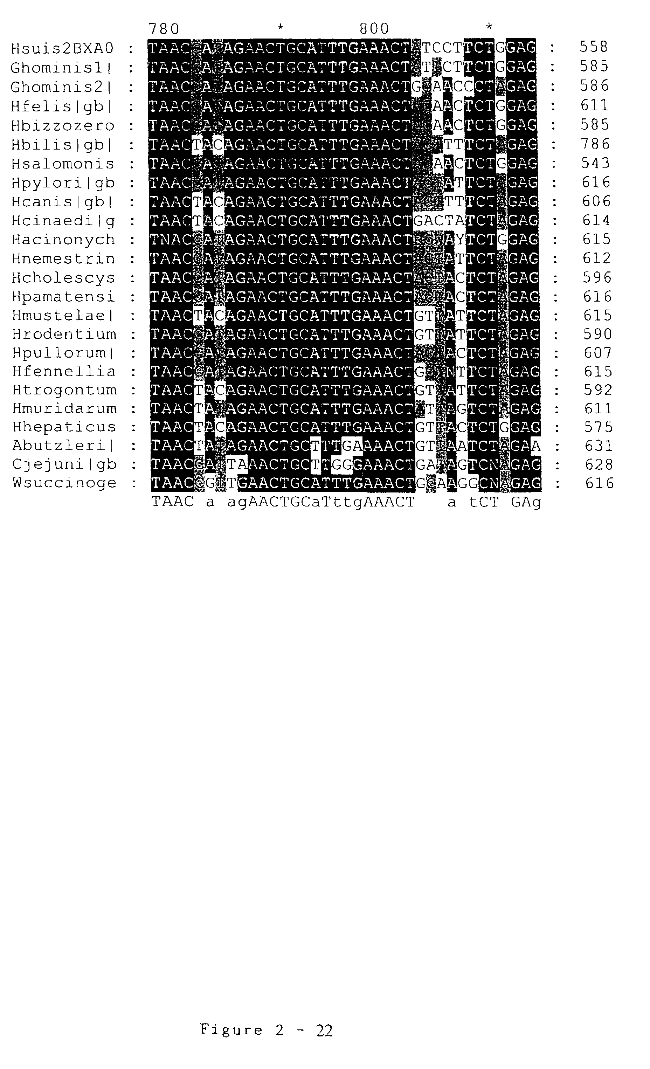

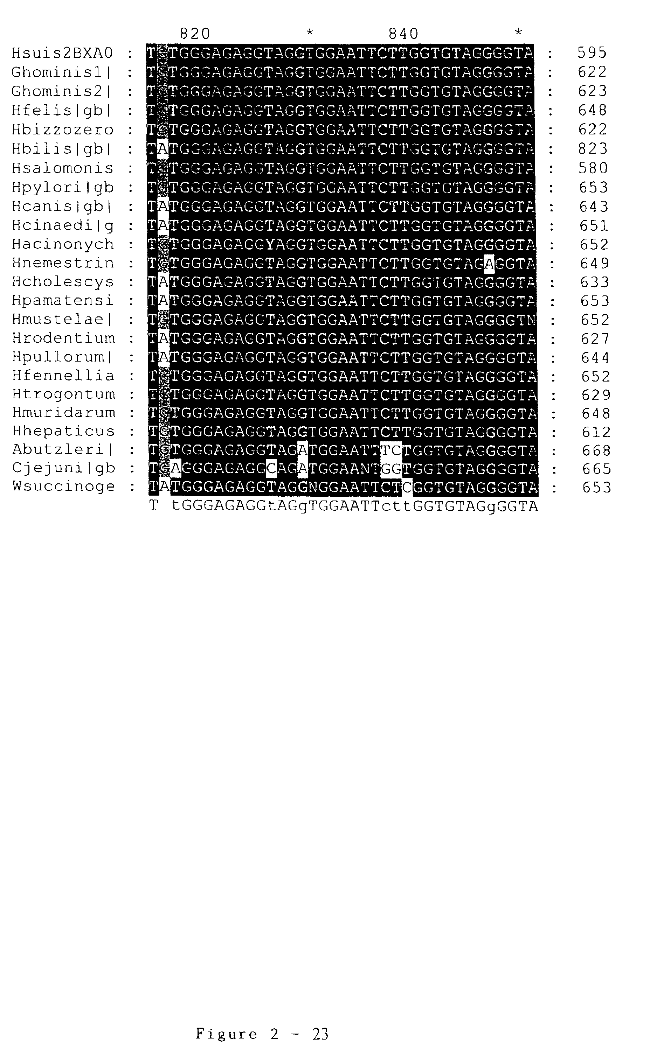

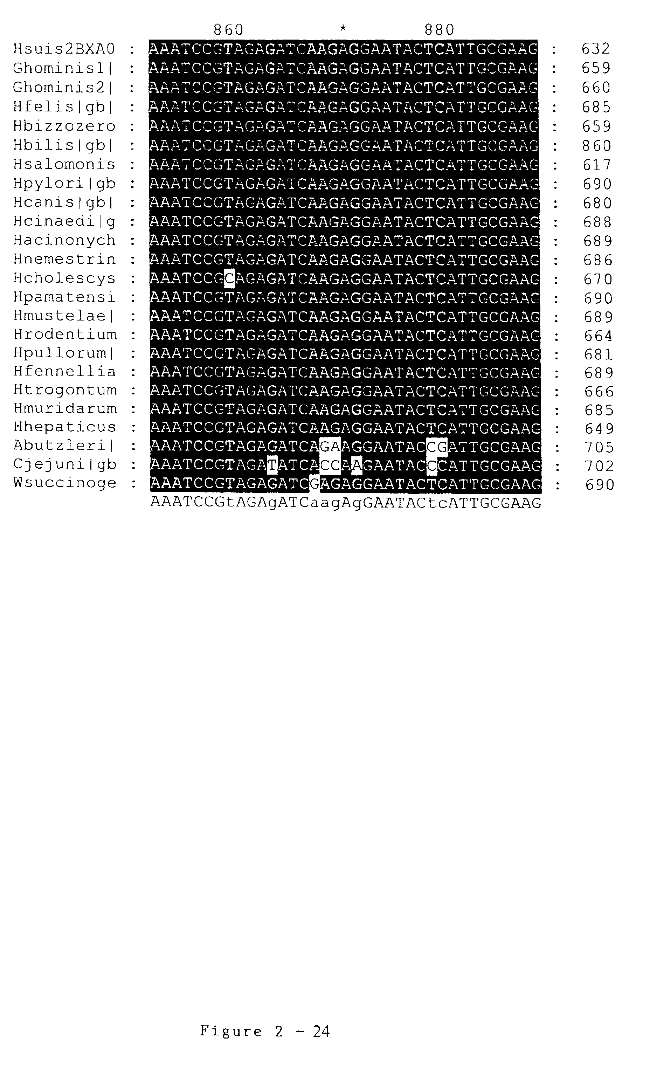

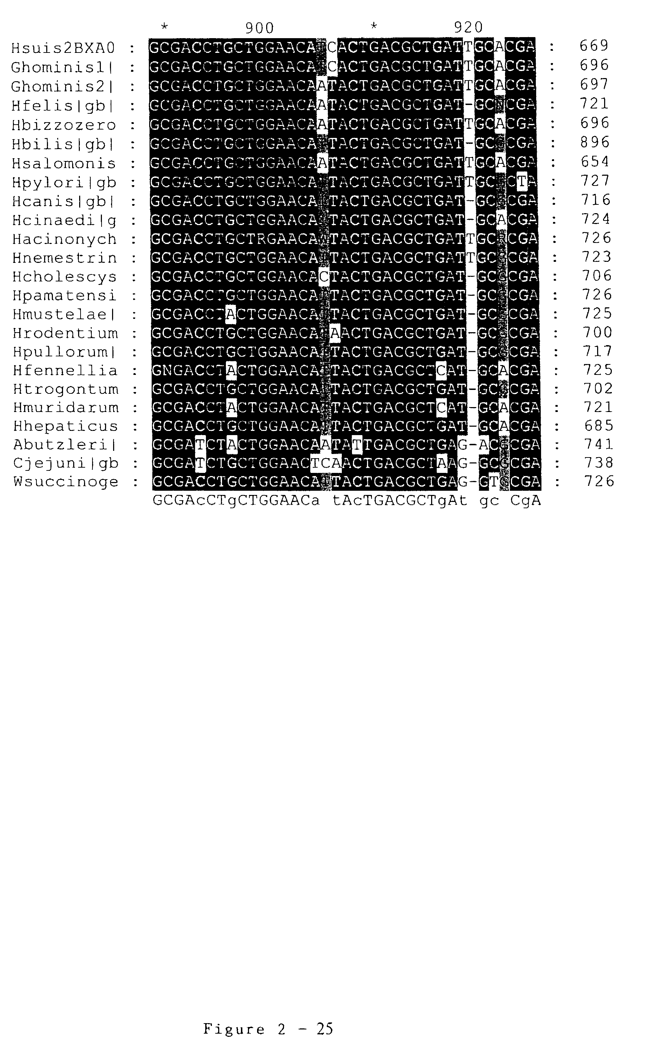

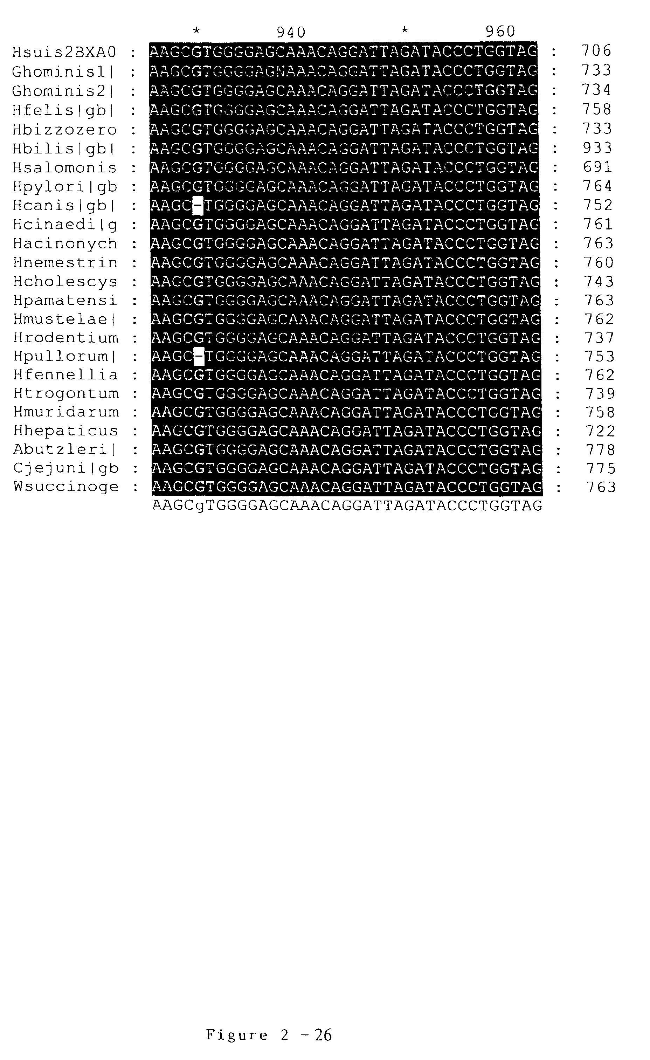

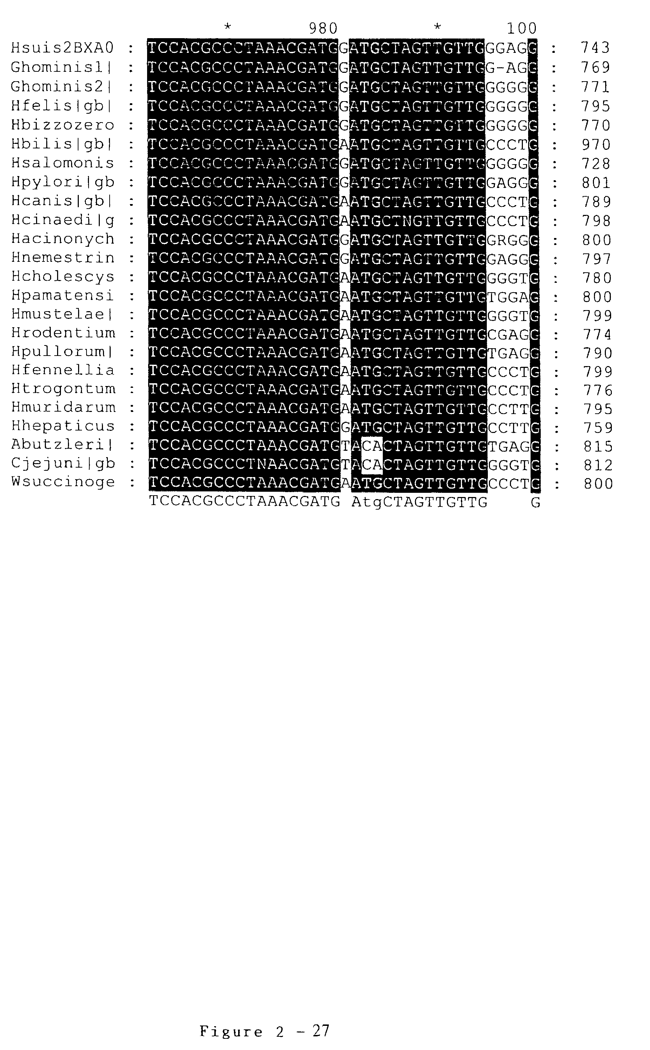

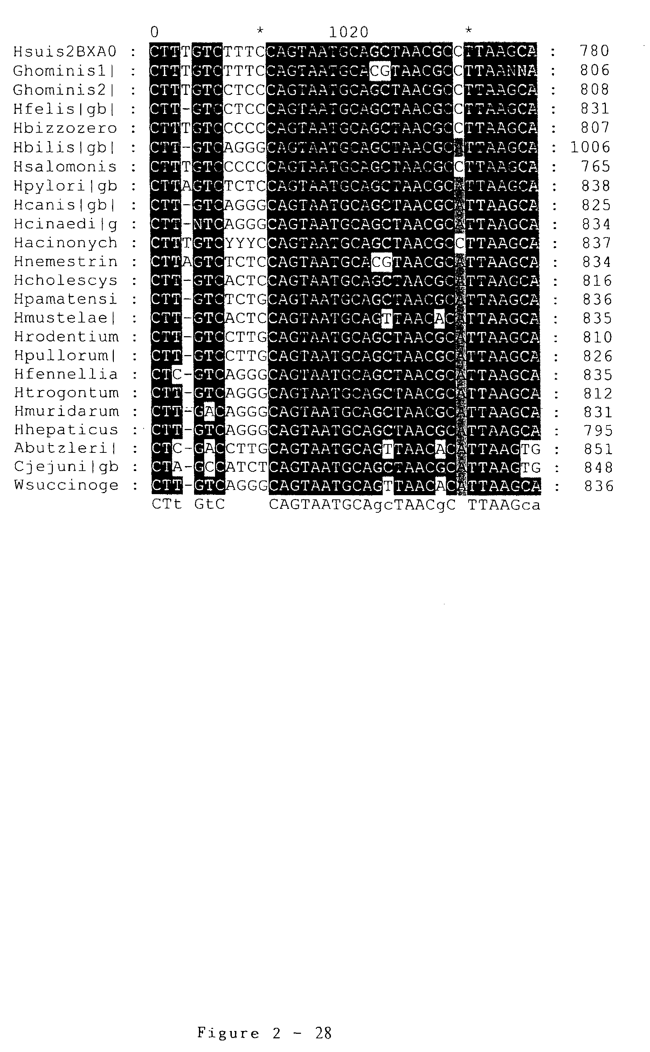

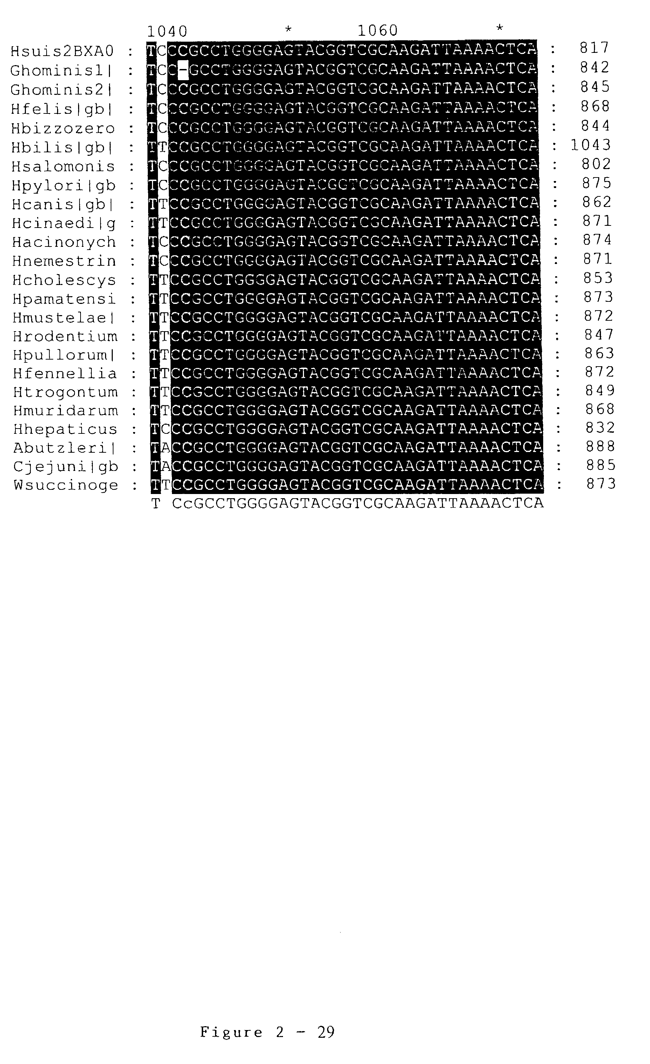

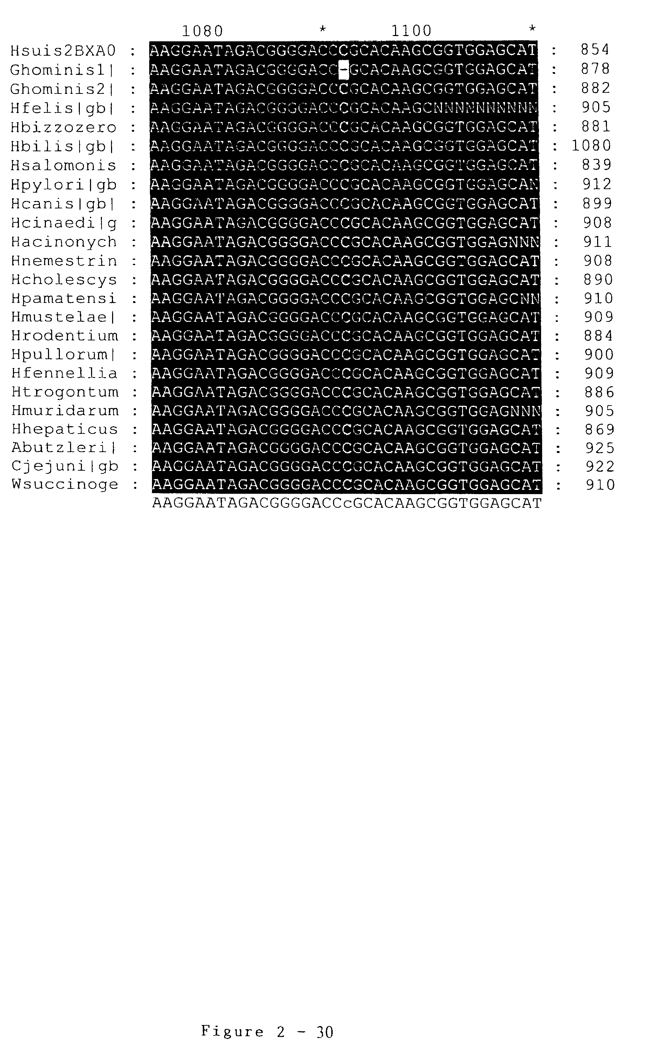

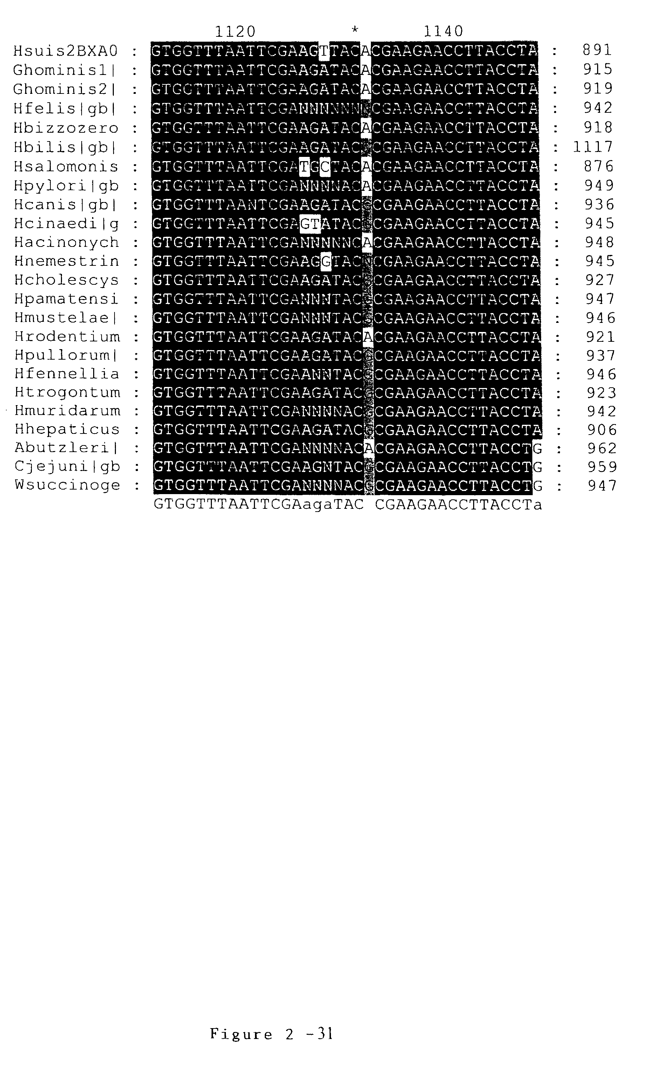

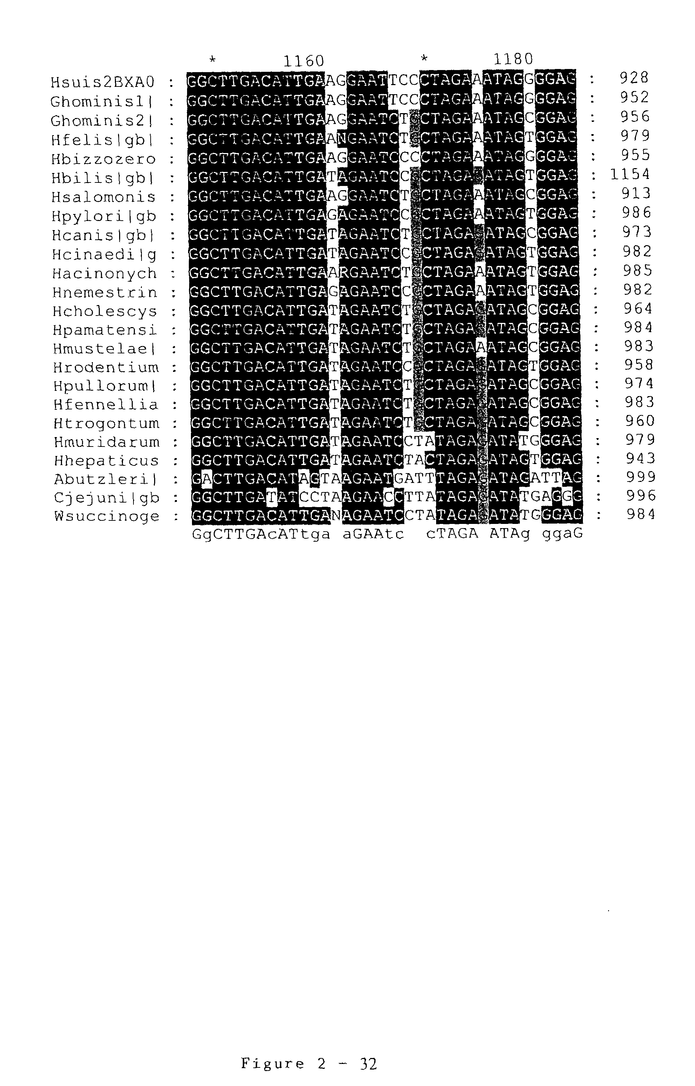

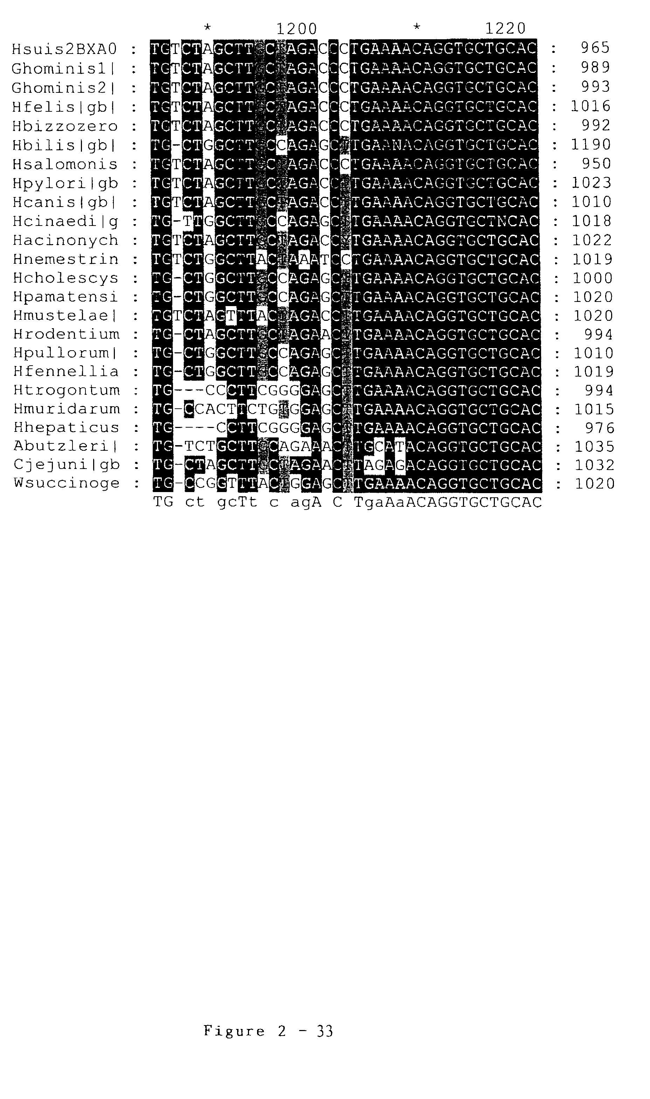

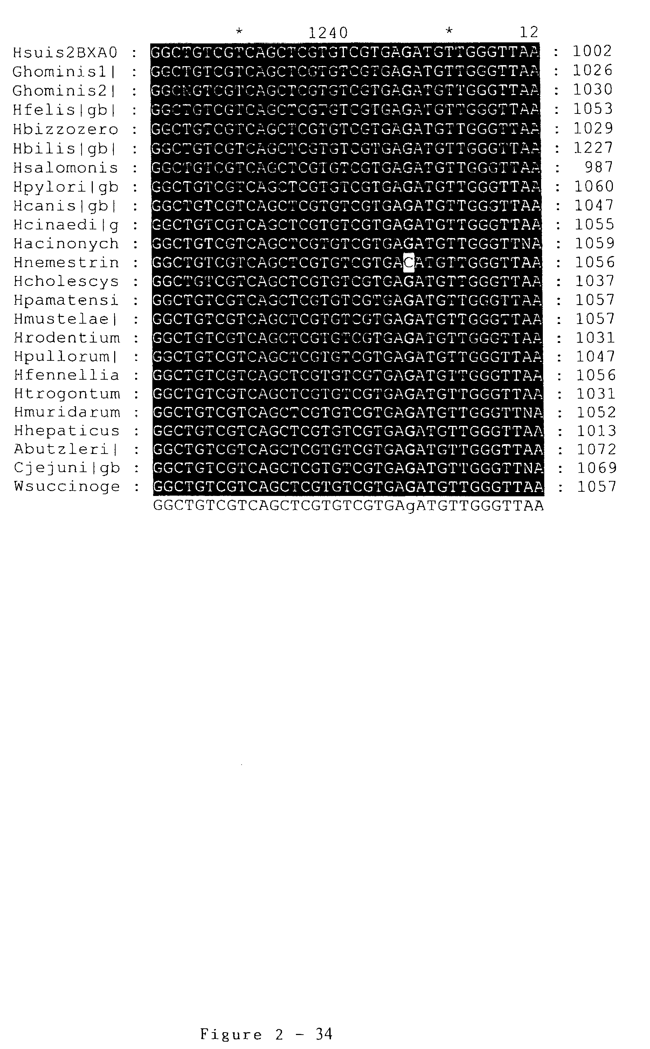

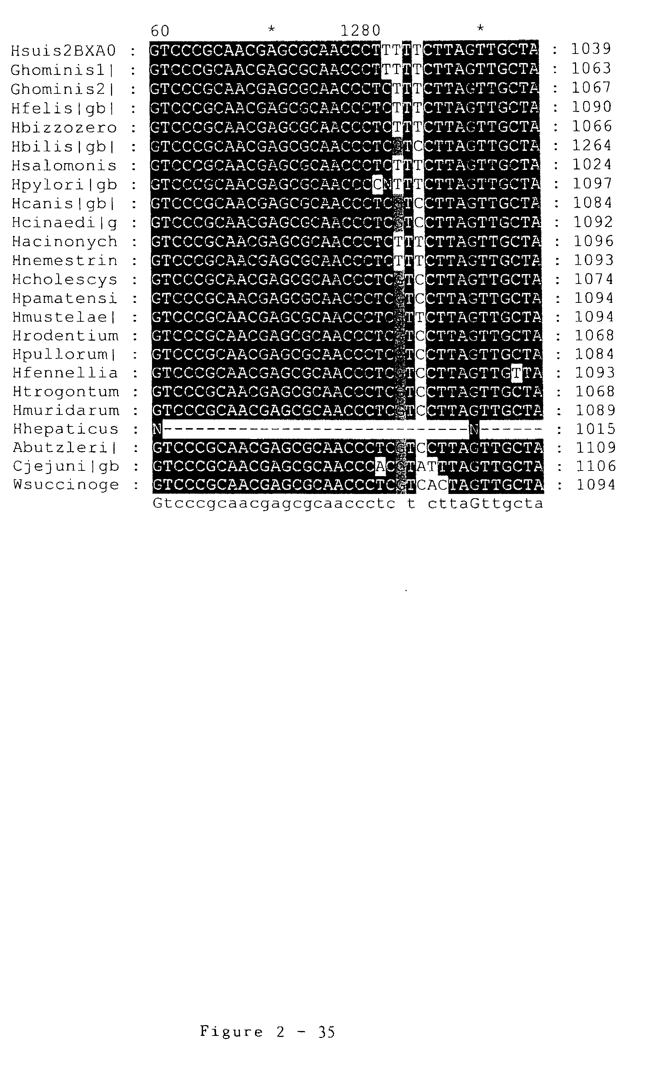

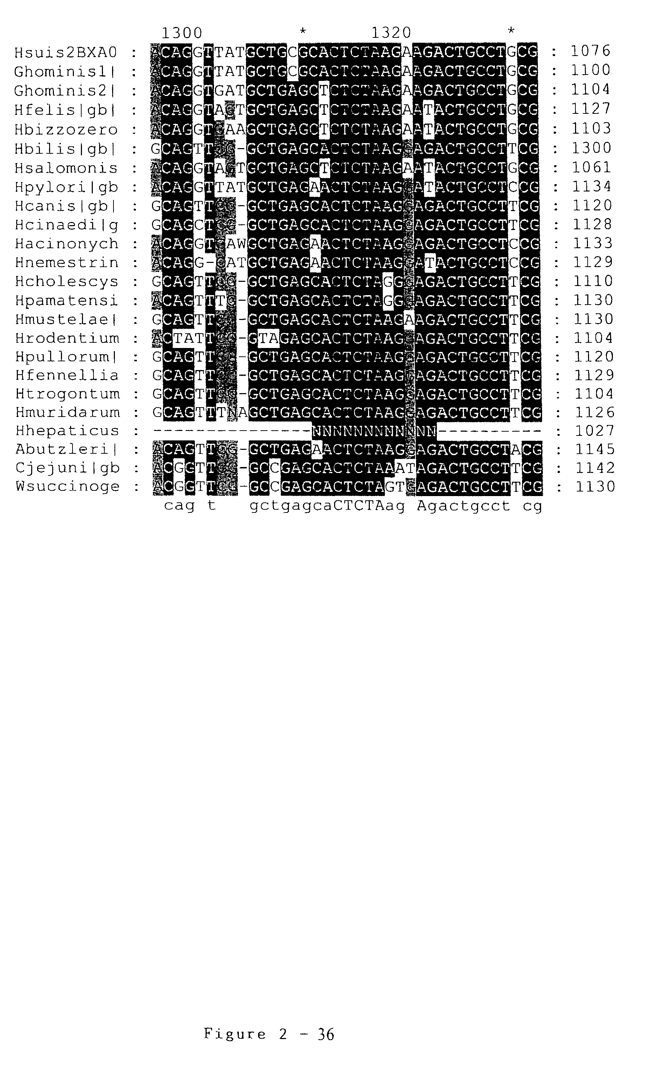

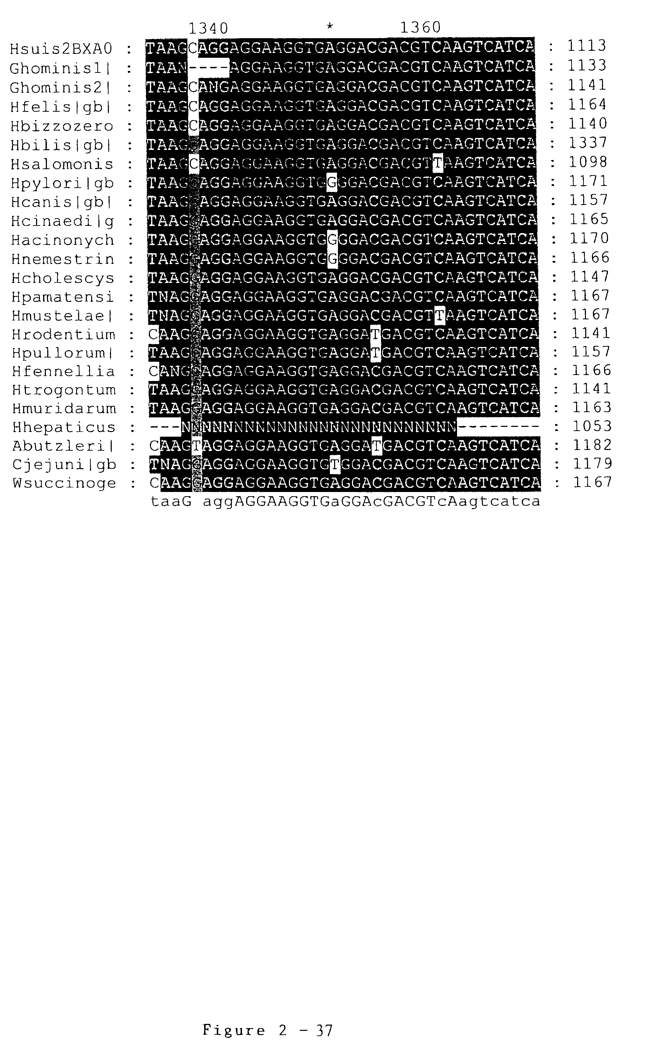

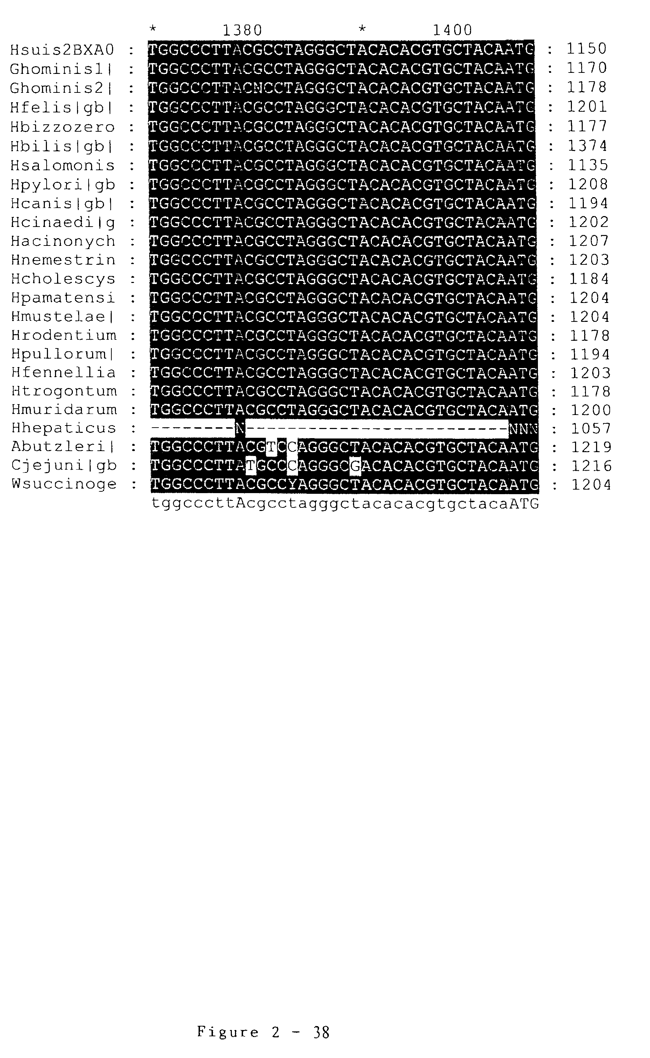

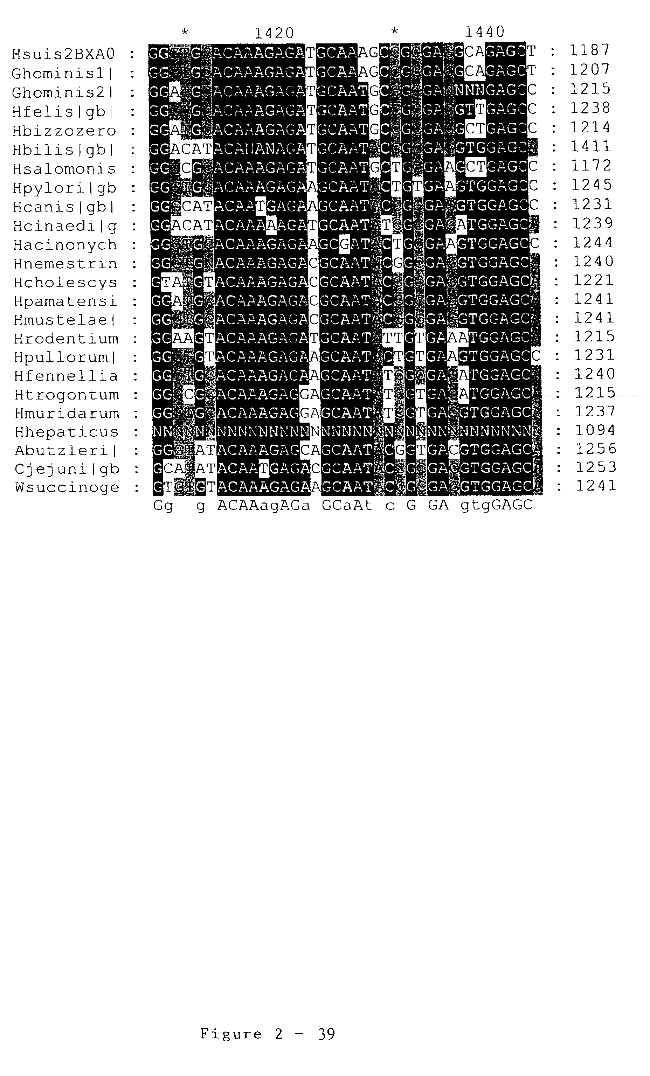

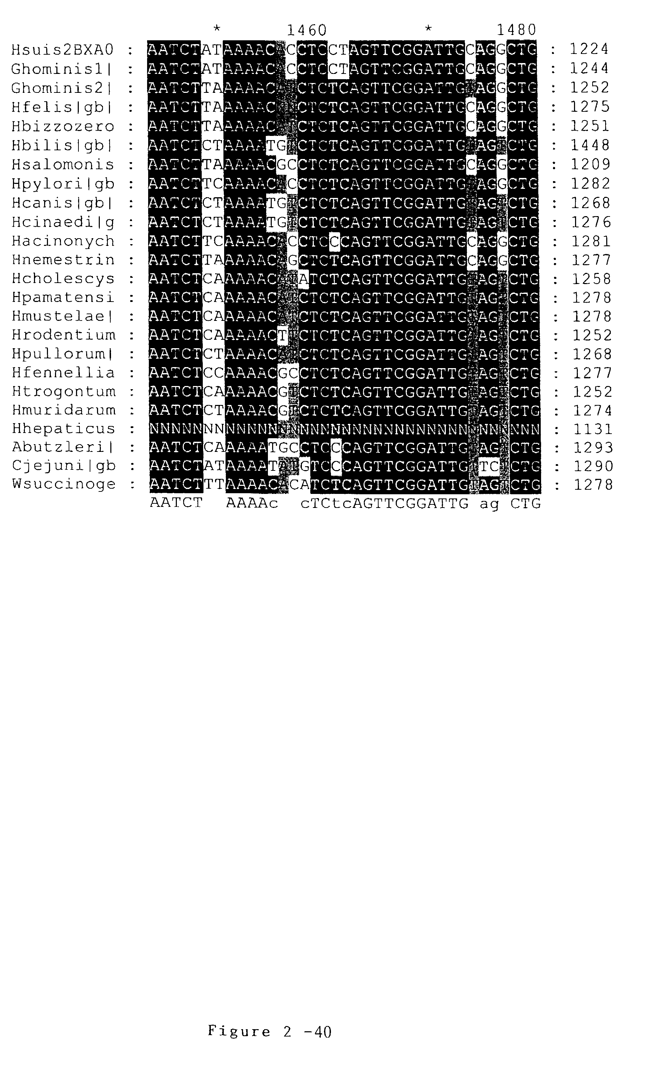

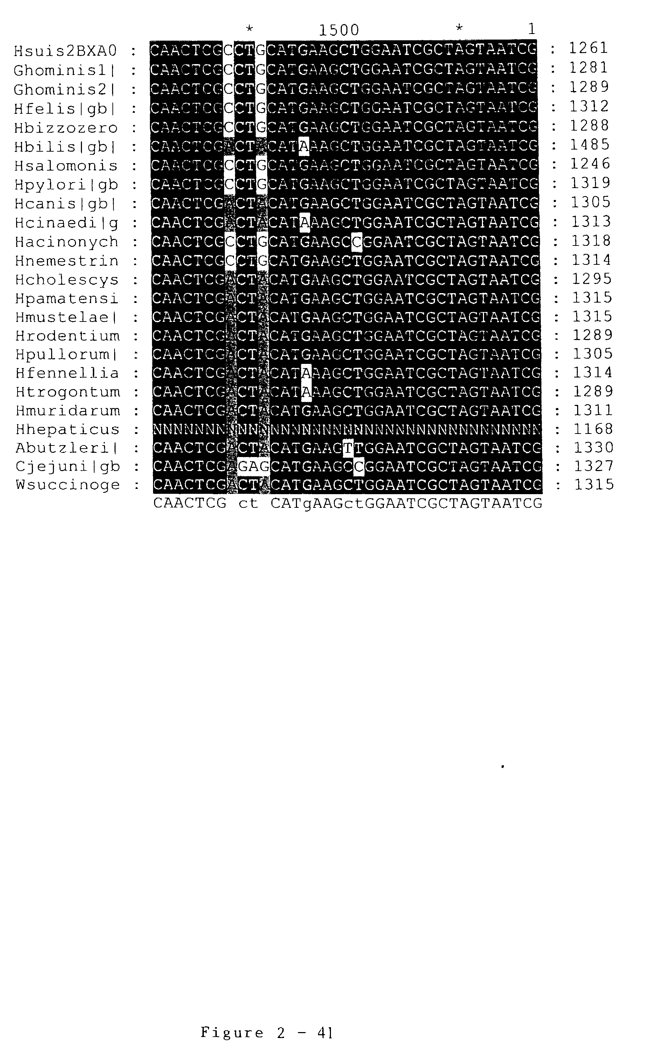

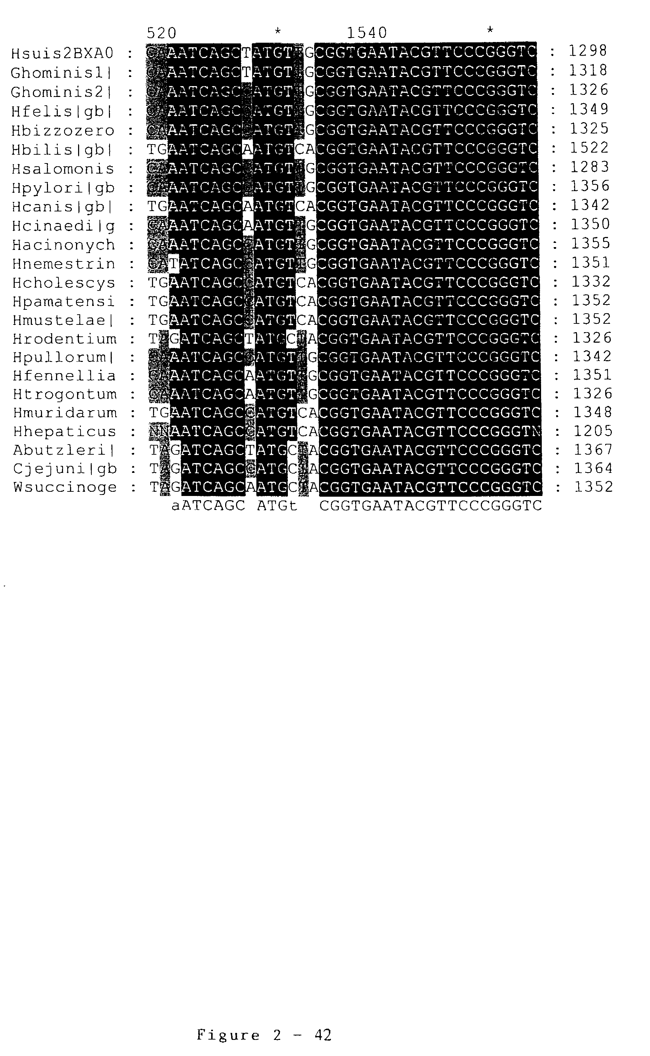

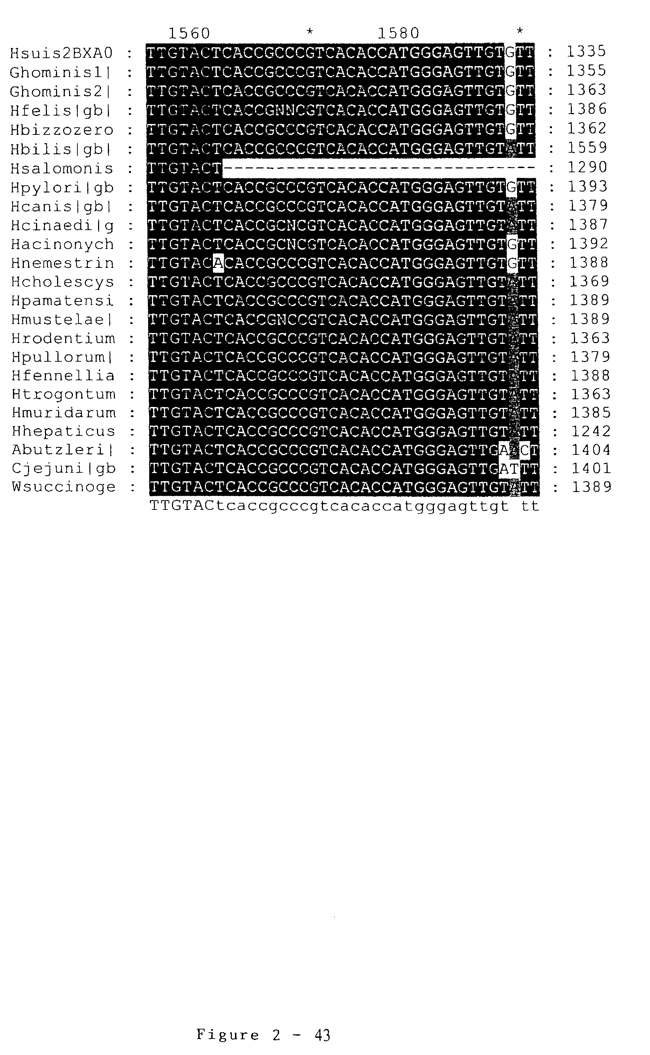

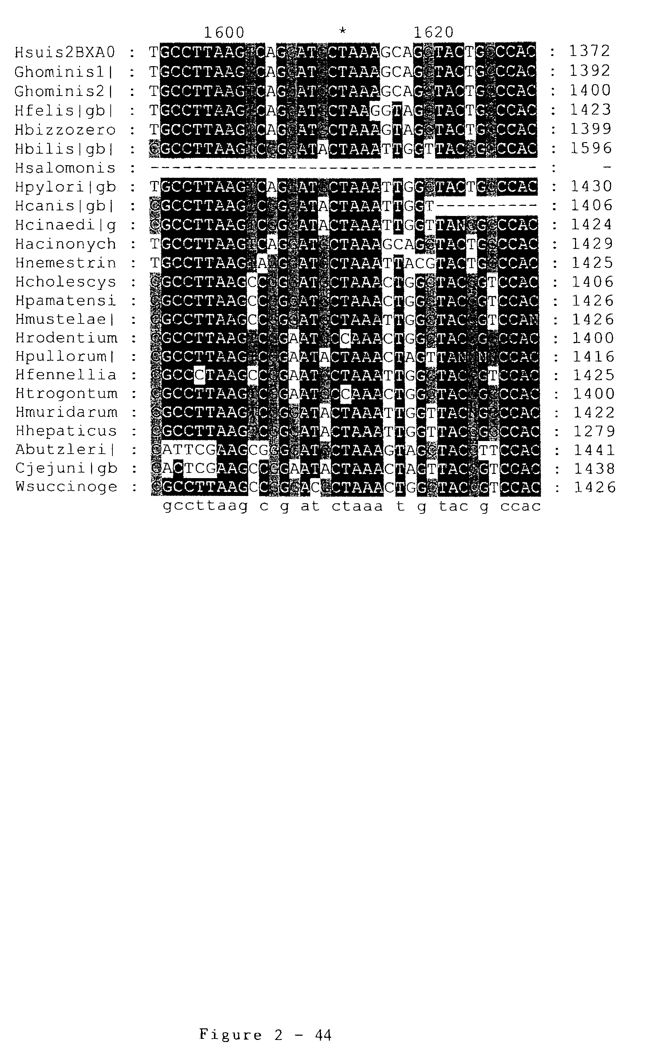

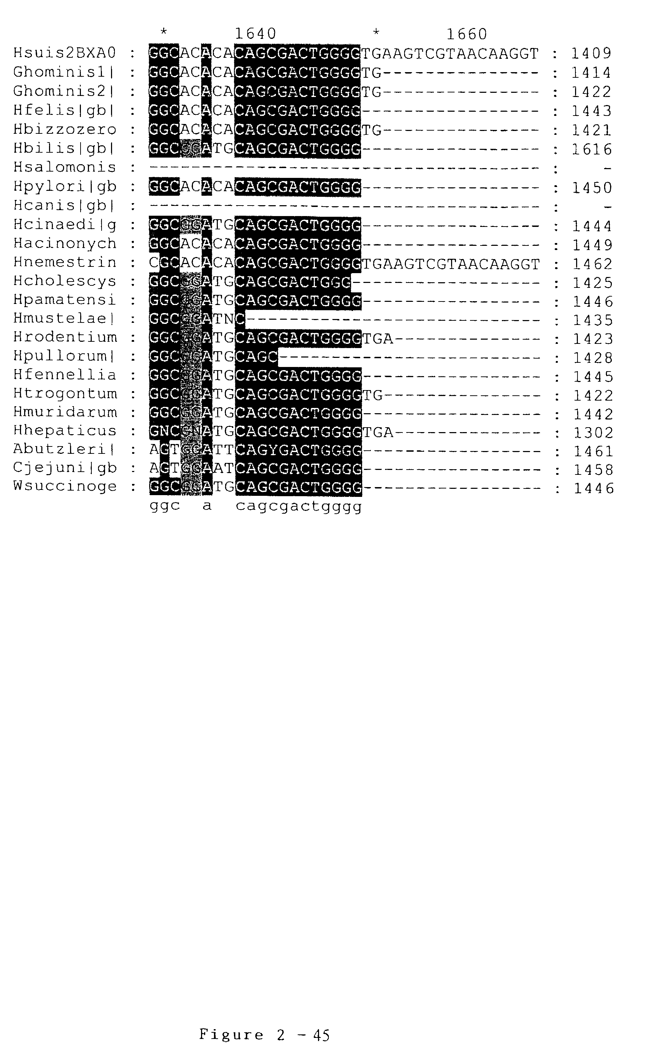

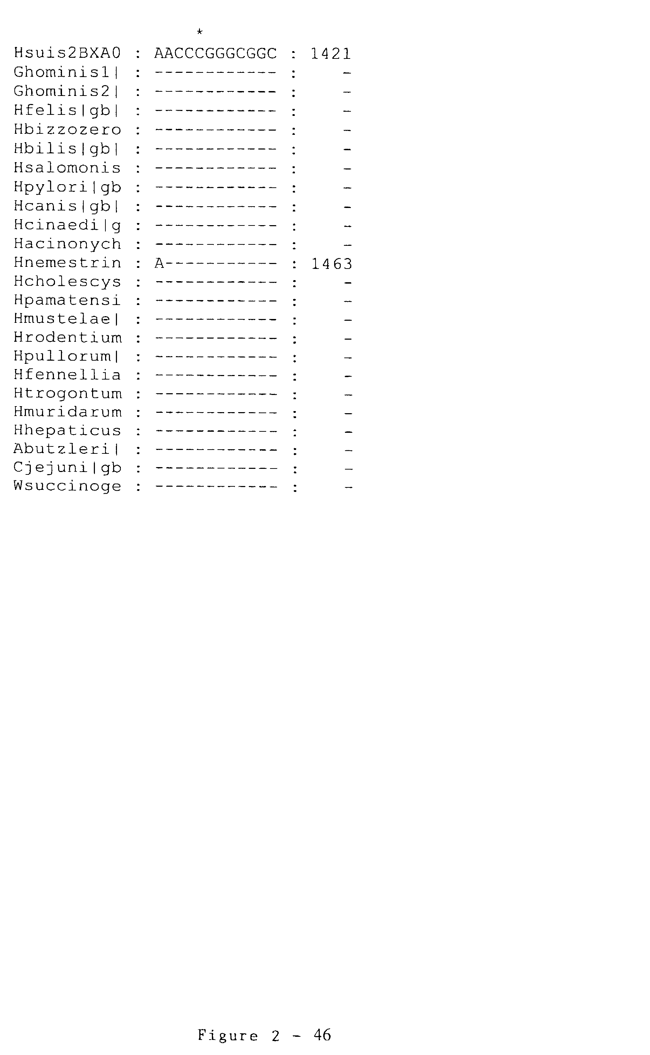

Preferred probes are given in Table 2. Probe *R628f* is a preferred "*Candidatus* Helicobacter bovis" specific probe. Probe *V100f* is a preferred "*Candidatus* Helicobacter suis" specific probe. Other suitable probes may be derived from a visual inspection of the alignment shown in Figure 1 or 2.

According to another embodiment, the present invention relates to a primer which specifically amplifies a polynucleic acid sequence as defined above.

Preferred primers according to the present invention are given in Table 2. Primers *R574f and R832r* are preferred "*Candidatus* Helicobacter bovis" specific primers and are suited for a specific PCR and in situ hybridisation assays. Primers *V832f* and *V1621r* are preferred "*Candidatus* Helicobacter suis" specific primers for a specific PCR and in situ hybridisation assays. Other suitable primers according to the present invention may be derived from a visual inspection of the alignment shown in Figure 1 or 2.

Relying on the principles well kown in the art, the skilled man will be able to select primers that allow specific amplification of SEQ ID NO 1 or 2 or the claimed variants thereof under given or experimental conditions, such as temperature, buffer composition, polymerase chain reaction cycle etc. Likewise the skilled man will be able to select probes that specifically hybridize to either SEQ ID NO 1 or 2 or the claimed variants under given experimental conditions such as temperature, buffer composition etc. Having chosen primers and/or probes, the skilled man will furthermore be able to assess the efficacy of these primers or probes without undue experimentation. It is also obvious that the skilled man may chose to combine more than one primer pair or more than one probe to carry out the method defined above.

In some cases, one may not wish to detect all SEQ ID NO 1 or 2 variants as specified above, for instance if one intends to detect alleles found in a certain geographic region.

According to another embodiment, the present invention relates to a method for detection and/or typing of *Helicobacter* strains present in a biological sample comprising hybridizing the 16S rRNA or 16S rDNA target region polynucleotides of said *Helicobacter* strains present in said biological sample with at least one probe as defined above.

Preferably said method may be used to study and detect the occurrence of pathogenic *Helicobacter* strains.

According to another embodiment, the present invention relates to a method for detection and/or typing of *Helicobacter* strains present in a biological sample comprising specifically amplifying the 16S rRNA or 16S rDNA target region polynucleotides of said *Helicobacter* strains present in said biological sample with at least one primer as defined above.

Preferably said method may be used to study and detect the occurrence of pathogenic *Helicobacter* strains.

A preferred embodiment according to the present invention involves a method for detection and/or typing of *Helicobacter* strains present in a biological sample comprising first amplifying a specifc target region encompassed in or comprising the 16S rRNA region of said *Helicobacter* strains present in said biological sample and subsequently hybridizing the 16S rRNA or 16S rDNA target region polynucleotides of said *Helicobacter* strains present in said biological sample with at least one (or more than one) probe as defined above.

Different techniques can be applied to perform the methods of the present invention. These techniques may comprise immobilizing the target polynucleic acids, possibly after amplification, on a solid support and performing a hybridization with labelled oligonucleotide probes of the present invention. Alternatively, said probes may be immobilized on a solid support and hybridization may be performed with labelled target polynucleic acids, possibly after amplification (i.e. a reverse hybridization).

A preferred method according to the present invention is an *in situ* hybridisation assay (see Examples section).

The well-known technique of Southern blotting is one example of a hybridization assay that can be used to perform the methods of the present invention. Another example of a hybridization technique is the DNA enzyme immuno assay (DEIA). According to this method, PCR products are generated by a primer set, of which either the forward or the reverse primer contain biotin at the 5' end. This allows binding of the biotinylated amplimers to streptavidin-coated microtiter wells. PCR products are denatured by sodium hydroxide, which allows removal of the non-biotinylated strand. Specific digoxigenin (DIG)-labelled oligonucleotide probes are hybridized to the single-stranded immobilized PCR product and hybrids are detected by enzyme-labelled conjugate and colorimetric methods.

A convenient reverse hybridization technique is the LiPA assay. The LiPA uses oligonucleotide probes immobilized as parallel lines on a solid support strip (Stuyver et al. 1993; international patent application WO 94/12670). This approach is particularly advantageous since it is fast and simple to perform.

It is to be understood that any other type of hybridization assay or hybridization format using any of the selected probes as described further in the invention, is also covered by the present invention.

According to another embodiment , the present invention relates to a diagnostic kit for detection and/or typing of *Helicobacter* strains comprising:
- at least one probe as defined above and/or,
- at least one primer as defined above.

According to another embodiment, the present invention relates to a medicament comprising a polynucleic acid sequence as defined above.

According to another embodiment, the present invention relates to a polynucleic acid sequence as defined above for use as a medicament.

The following definitions and explanations will permit a better understanding of the present invention.

The target material in the samples to be analysed may either be DNA or RNA, e.g. genomic DNA, messenger RNA, viral RNA or amplified versions thereof. These molecules are in this application also termed "polynucleic acids" or "polynucleotides". More particularly, the target material according to the present invention will be 16S ribosomal RNA or DNA or amplified versions thereof.

Well-known extraction and purification procedures are available for the isolation of RNA or DNA from a sample (e.g. in Sambrook et al.,1989).

The term "probe" according to the present invention refers to a single-stranded oligonucleotide which is designed to specifically hybridize to "*Candidatus* Helicobacter bovis or suis" polynucleic acids.

The term "primer" refers to a single stranded oligonucleotide sequence capable of acting as a point of initiation for synthesis of a primer extension product which is complementary to the nucleic acid strand to be copied. The length and the sequence of the primer must be such that they allow to prime the synthesis of the extension products. Preferably the primer is about 5-50 nucleotides long. Specific length and sequence will depend on the complexity of the required DNA or RNA targets, as well as on the conditions at which the primer is used, such as temperature and ionic strength. It is to be understood that the primers of the present invention may be used as probes and *vice versa,* provided that the experimental conditions are adapted.

The expression "suitable primer pair" in this invention refers to a pair of primers allowing specific amplification of a "*Candidatus* Helicobacter bovis or suis" polynucleic acid fragment.

The term "target region" of a probe or a primer according to the present invention is a sequence within the *"Candidatus* Helicobacter bovis or suis" polynucleic acids to which the probe or the primer is completely complementary or partially complementary (i.e. with some degree of mismatch). It is to be understood that the complement of said target sequence is also a suitable target sequence in some cases.

"Specific hybridization" of a probe to a target region of respectively the "*Candidatus* Helicobacter bovis" or *Candidatus* Helicobacter suis" polynucleic acids means that said probe forms a duplex with part of this region or with the entire region under the experimental conditions used, and that under those conditions said probe does not form a duplex with other regions of the polynucleic acids present in the sample to be analysed.

"Specific hybridization" of a primer to a target region of respectively the "*Candidatus* Helicobacter bovis" or "*Candidatus* Helicobacter suis" polynucleic acids means that, during the amplification step, said primer forms a duplex with part of this region or with the entire region under the experimental conditions used, and that under those conditions said primer does not form a duplex with other regions of the polynucleic acids present in the sample to be analysed. It is to be understood that "duplex" as used hereby, means a duplex that will lead to specific amplification.

"Specific amplification" of a fragment of respectively the "*Candidatus* Helicobacter bovis" or *Candidatus* Helicobacter suis" polynucleic acids means amplification of the fragment for which the primers were designed, and not of any other fragment of the polynucleic acids present in a sample.

The fact that amplification primers do not have to match exactly with the corresponding target sequence in the template to warrant proper amplification is amply documented in the literature (Kwok et al., 1990). However, when the primers are not completely complementary to their target sequence, it should be taken into account that the amplified fragments will have the sequence of the primers and not of the target sequence. Primers may be labelled with a label of choice (e.g. biotine). The amplification method used can be either polymerase chain reaction (PCR; Saiki et al., 1988), ligase chain reaction (LCR; Landgren et al., 1988; Wu & Wallace, 1989; Barany, 1991), nucleic acid sequence-based amplification (NASBA; Guatelli et al., 1990; Compton, 1991), transcription-based amplification system (TAS; Kwoh et al., 1989), strand displacement amplification (SDA; Duck, 1990) or amplification by means of Qß replicase (Lomeli et al., 1989) or any other suitable method to amplify nucleic acid molecules known in the art.

Preferably, the probes of the invention are about 5 to 50 nucleotides long, more preferably from about 10 to 25 nucleotides. Particularly preferred lengths of probes include 10, 11, 12, 13, 14, 15, 16, 17, 18, 19, 20, 21, 22, 23, 24 or 25 nucleotides. The nucleotides as used in the present invention may be ribonucleotides, deoxyribonucleotides and modified nucleotides such as inosine or nucleotides containing modified groups which do not essentially alter their hybridization characteristics.

Probe and primer sequences are represented throughout the specification as single stranded DNA oligonucleotides from the 5' to the 3' end. It is obvious to the man skilled in the art that any of the below-specified probes can be used as such, or in their complementary form, or in their RNA form (wherein T is replaced by U).

The probes according to the invention can be prepared by cloning of recombinant plasmids containing inserts including the corresponding nucleotide sequences, if need be by excision of the latter from the cloned plasmids by use of the adequate nucleases and recovering them, e.g. by fractionation according to molecular weight. The probes according to the present invention can also be synthesized chemically, for instance by the conventional phospho-triester method.

The oligonucleotides used as primers or probes may also comprise nucleotide analogues such as phosphorothiates (Matsukura et al., 1987), alkylphosphorothiates (Miller et al., 1979) or peptide nucleic acids (Nielsen et al., 1991; Nielsen et al., 1993) or may contain intercalating agents (Asseline et al., 1984). As most other variations or modifications introduced into the original DNA sequences of the invention these variations will necessitate adaptions with respect to the conditions under which the oligonucleotide should be used to obtain the required specificity and sensitivity. However, the eventual results of hybridization will be essentially the same as those obtained with the unmodified oligonucleotides. The introduction of these modifications may be advantageous in order to positively influence characteristics such as hybridization kinetics, reversibility of the hybrid-formation, biological stability of the oligonucleotide molecules, etc.

The term "solid support" can refer to any substrate to which an oligonucleotide probe can be coupled, provided that it retains its hybridization characteristics and provided that the background level of hybridization remains low. Usually the solid substrate will be a microtiter plate, a membrane (e.g. nylon or nitrocellulose) or a microsphere (bead) or a chip. Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, NH₂ groups, SH groups, carboxylic groups, or coupling with biotin, haptens or proteins.

The term "labelled" refers to the use of labelled nucleic acids. Labelling may be carried out by the use of labelled nucleotides incorporated during the polymerase step of the amplification such as illustrated by Saiki et al. (1988) or Bej et al. (1990) or labelled primers, or by any other method known to the person skilled in the art. The nature of the label may be isotopic (³²P, ³⁵S, etc.) or non-isotopic (biotin, digoxigenin, etc.).

The "biological sample" may be for instance cultured *Helicobacter* strains, gastric, abomasal stomachs, omasal stomachs, reticulum and rumen, or duodenal biopsies (fresh or parafine material), faeces, saliva, mouth mucosa, gastric juice or urine. Preferably these samples may be taken from piglets, pigs, humans, calves, cattle, etc.

For designing probes with desired characteristics, the following useful guidelines known to the person skilled in the art can be applied.

Because the extent and specificity of hybridization reactions such as those described herein are affected by a number of factors, manipulation of one or more of those factors will determine the exact sensitivity and specificity of a particular probe, whether perfectly complementary to its target or not. The importance and effect of various assay conditions are explained further herein.

∗∗The stability of the [probe : target] nucleic acid hybrid should be chosen to be compatible with the assay conditions. This may be accomplished by avoiding long AT-rich sequences, by terminating the hybrids with G:C base pairs, and by designing the probe with an appropriate Tm. The beginning and end points of the probe should be chosen so that the length and %GC result in a Tm about 2-10EC higher than the temperature at which the final assay will be performed. The base composition of the probe is significant because G-C base pairs exhibit greater thermal stability as compared to A-T base pairs due to additional hydrogen bonding. Thus, hybridization involving complementary nucleic acids of higher G-C content will be more stable at higher temperatures.

∗∗Conditions such as ionic strength and incubation temperature under which a probe will be used should also be taken into account when designing a probe. It is known that the degree of hybridization will increase as the ionic strength of the reaction mixture increases, and that the thermal stability of the hybrids will increase with increasing ionic strength. On the other hand, chemical reagents, such as formamide, urea, DMSO and alcohols, which disrupt hydrogen bonds, will increase the stringency of hybridization. Destabilization of the hydrogen bonds by such reagents can greatly reduce the Tm. In general, optimal hybridization for synthetic oligonucleotide probes of about 10-50 bases in length occurs approximately SEC below the melting temperature for a given duplex. Incubation at temperatures below the optimum may allow mismatched base sequences to hybridize and can therefore result in reduced specificity.

∗∗It is desirable to have probes which hybridize only under conditions of high stringency. Under high stringency conditions only highly complementary nucleic acid hybrids will form; hybrids without a sufficient degree of complementarity will not form. Accordingly, the stringency of the assay conditions determines the amount of complementarity needed between two nucleic acid strands forming a hybrid. The degree of stringency is chosen such as to maximize the difference in stability between the hybrid formed with the target and the non-target nucleic acid.

∗∗Regions in the target DNA or RNA which are known to form strong internal structures inhibitory to hybridization are less preferred. Likewise, probes with extensive self-complementarity should be avoided. As explained above, hybridization is the association of two single strands of complementary nucleic acids to form a hydrogen bonded double strand. It is implicit that if one of the two strands is wholly or partially involved in a hybrid that it will be less able to participate in formation of a new hybrid. There can be intramolecular and intermolecular hybrids formed within the molecules of one type of probe if there is sufficient self complementarity. Such structures can be avoided through careful probe design. By designing a probe so that a substantial portion of the sequence of interest is single stranded, the rate and extent of hybridization may be greatly increased. Computer programs are available to search for this type of interaction. However, in certain instances, it may not be possible to avoid this type of interaction.

∗∗Standard hybridization and wash conditions are disclosed in the Examples section. Other conditions are for instance 3X SSC (Sodium Saline Citrate), 20% deionized FA (Formamide) at 50EC. Other solutions (SSPE (Sodium saline phosphate EDTA), TMAC (Tetramethyl ammonium Chloride), etc.) and temperatures can also be used provided that the specificity and sensitivity of the probes is maintained. When needed, slight modifications of the probes in length or in sequence have to be carried out to maintain the specificity and sensitivity required under the given circumstances.

The term "hybridization buffer" means a buffer allowing a hybridization reaction between the probes and the polynucleic acids present in the sample, or the amplified products, under the appropriate stringency conditions.

The term "wash solution" means a solution enabling washing of the hybrids formed under the appropriate stringency conditions.

The Examples as set out below only serve to illustrate the present invention. The contents of all references referred to in this text are hereby incorporated by reference.

### FIGURE AND TABLE LEGENDS

**Figure 1** represents an alignment of the reference sequence R2XA of "*Candidatus* Helicobacter bovis" (SEQ ID NO 1) with sequences of strains belonging to the epsilon subdivision (Table 3) which were retrieved from the EMBL data library and were aligned with reference sequence R2XA
**Figure 2** represents an alignment of the reference sequence V2BXA of "*Candidatus* Helicobacter suis" (SEQ ID NO 2) sequence with the sequences of strains belonging to the same phylogenetic lineage (Table 3).
**Figure 3A** represents a phylogenetic tree based on the phylogenetic analysis of "*Candidates* Helicobacter bovis" as set out in the Examples section. The scale bar represents a one % difference in nucleotide sequences as determined by measuring the length of horizontal lines connecting any two species.
**Figure 3B** represents a a phylogenetic tree based on the phylogenetic analysis of "*Candidatus* Helicobacter suis" as set out in the Examples section. The scale bar represents a one % difference in nucleotide sequences as determined by measuring the length of horizontal lines connecting any two species.
**Figure 4** represents an alignment of the different "*Candidatus* Helicobacter bovis" sequences. The reference sequence is R2XA001 (SEQ ID NO 1). The other sequences are R5XE001 (SEQ ID NO 15), R3XA001 (SEQ ID NO 16), R6XA001 (SEQ ID NO 17), R13D001INV (SEQ ID NO 18), R27TOTAAL (SEQ ID NO 19) and R28TOTAAL (SEQ ID NO 20).
**Figure 5** represents an alignment of the different "*Candidatus* Helicobacter suis" sequences. The reference sequence is RBXA001 (SEQ ID NO 2). The other sequences are 4AXA001 (SEQ ID NO 21), 6W06001 (SEQ ID NO 22), V14D001 (SEQ ID NO 23), V19DINV001 (SEQ ID NO 24).

### EXAMPLES

### Material and Methods

### Samples from cattle

Seven abomasal stomachs from clinically healthy slaughterhouse cattle originating from different Belgian and Dutch farms, were selected. The stomachs were opened longitudinally along the greater curvatura and rinsed gently with tap water. Two small mucosal fragments were taken from each stomach, one near the torus pyloricus and one in the fundic region, and were tested for urease activity (CUTest, Temmler Pharma) for h at 37 °C. Three mucosal biopsies from the pyloric region were taken for immunohistochemistry and *in situ* hybridisation and placed into 4 % buffered formaline for 24 hours. For electronmicroscopy, a pyloric sample was taken from the same region and fixed in cacodylate buffer (0.1 M, pH 7.0) containing 5 % glutaraldehyde and 0.15 % (wt/vol) ruthenium red. From each stomach a mucosal fragment was also taken for PCR analysis, placed into sterile PBS and frozen in liquid nitrogen. Special care was taken during sampling to avoid cross-contamination.

### Samples from pigs

Stomachs from 5 healthy slaughterhouse pigs were selected, all originating from different farms in Belgium and the Netherlands. The stomachs were opened longitudinally along the greater curvatura and rinsed gently with tap water. A small mucosal fragment was taken from each stomach near the torus pyloricus and placed into an urease test tube (CUTest, Temmler Pharma) for 2 hours at 37 °C. Mucosal biopsies from the antral part of the stomach were taken along the curvatura major (*n*=2) and the curvatura minor (*n*=2) for immunohistochemical evaluation and placed into 4 % buffered formalin. For electronmicroscopy, samples were taken from the same places and fixed in 0.1 M cacodylate buffer (pH 7.0) containing 5% glutaraldehyde and 0.15 % (wt/vol) ruthenium red. Of each stomach a mucosal fragment was also taken for PCR, placed into sterile PBS and frozen in liquid nitrogen. Special care was taken during sampling to avoid cross-contamination.

### Reference strains for "Candidatus Helicobacter bovis"

A total of 15 *Helicobacter* strains and 1 *Wolinella succinogenes* strain were used to test the specificity of the "*Candidatus* Helicobacter bovis" specific PCR (Table 1). Strains were grown on a 5 % Mueller-Hinton blood agar and incubated at 37 °C in a microaerophilic atmosphere containing approximately 5 % O₂, 3.5 % CO₂, 7.5 % H₂ and 84 % N2. Bacteriological purity was checked by plating and Gram-staining.

### Reference strains for "Candidatus Helicobacter suis"

A total of 15 *Helicobacter* strains were used to test the specificity of the "*Candidatus* Helicobacter suis"-specific PCR assay (Table 1). Strains were grown on a 5% Mueller-Hinton blood agar and incubated at 37 °C in a microaerobic atmosphere containing approximately 5 % O₂, 3.5 % CO₂, 7.5 % H₂ and 84 % N₂. Bacteriological purity was checked by plating and Gram-staining.

### Immunohistochemistry

Immunohistochemical staining was performed to assess the presence of *Helicobacter*-like organisms. Formalin-fixed samples were dehydrated and paraffin-embedded. Sections of 4 µm were made of the paraffin-embedded tissues and were placed on 3-aminopropyltriethoxysilane-coated slides (APES, Sigma-Aldrich) and dried overnight at 60 °C. After dewaxing with xylene and rehydration in graded series with ethanol and distilled water, sections were placed in citrate buffer (0.1M with 2% ureum) and were boiled (1 x 3 min, 2 x 5 min) in a 800 Watt microwave oven (Whirlpool M611) to elicit antigen retrieval. Slides were then incubated with 12 % hydrogenperoxide in methanol (30 min) in order to block endogenous peroxidase activity. Thereafter the slides were pre-incubated with 30 % normal goat serum in PBS for 30 min to reduce non-specific antibody binding. A mouse polyclonal antibody directed against *H. pylori* (DAKO), diluted 1/20 in PBS, was incubated overnight at 21 °C in a moist chamber. The sections were washed and incubated with biotinylated swine anti-rabbit immunoglobulins at 21 °C for 30 min and after rinsing covered with peroxidase conjugated streptavidin-biotin-complex (ABC). Peroxidase activity was developed using H₂O₂ with diaminobenzidine (DAB) as a chromogen (Fast DAB Tablet Set, Sigma-Aldrich). Subsequently, the sections were counterstained with Mayer's hematoxylin and mounted. As a negative control, the primary antibody was replaced with fetal calf serum in Tris-HCl buffer (pH 7.6). As a positive control, a section of a mouse stomach experimentally infected with *Helicobacter pylori* LMG 7539^{T} was used.

### Transmission electronmicroscopy

For "*Candidatus* Helicobacter bovis", three different pyloric samples were selected for electronmicroscopic evaluation based upon the high presence of *Helicobacter*-like organisms in the corresponding immunostained sections.

For "*Candidatus* Helicobacter suis", two different antral biopsies were selected for electronmicroscopic evaluation based on the high presence of gastrospirillum-like organisms in the corresponding immunostained slides.

After dehydration in a graded series of acetone washes, the samples were embedded in Spurr low-viscosity resin. Ultrathin sections were poststained with uranyl acetate and lead citrate and examined with an electronmicroscope (Phillips 201 TEM) at an accelerating voltage of 60 kV.

### DNA-extraction

DNA was isolated from the scrapings of the gastric biopsies and from the reference strains by lysis with guanidinium isothiocyanate and DNA was bound to silica particles according to the method of Boom et al. (1990).

### Primers and PCR amplification of 16S rDNA

Broadrange primers *H33f, H61f* and *H1368r* were selected from rRNA superfamily VI *(Helicobacter, Campylobacter, Arcobacter, Wolinella)* specific regions of the 16S rRNA gene (Table 2).

The use of broad range primer *1492RPL* was suggested by Weissburg et al. (1991). A genus *Helicobacter*-specific primer *H274f* was adapted from primer *274r* described by Dewhirst et al. (1994) (Table 2). Primer combinations *H33F-H1368r, H274f-1492RPL* and *H61f-1492RPL* were used to amplify a ~ 1.3-Kb, ~ 1.2-Kb and a ~1.4-Kb fragment of *"Candidatus* Helicobacter suis" respectively.

PCR reactions were performed in a volume of 50 pl containing 10mM Tris HCl (pH 8.3), 50 mM KCl, 3.5 mM MgCl_{2,} 200 pM of each deoxynucleoside triphosphate, 1.5 U of AmpliTaq Gold (Perkin-Elmer, Roche Molecular Systems) and 25 pmol of both forward and reverse primer (Eurogentec). Reactions were covered with mineral oil and PCR was performed in a Biomed-60 thermocycler under the following conditions: 9 min preincubation at 94 °C to activate AmpliTaq Gold, followed by 50 cycles of 30 s at 94 °C, 45 s at 55 °C and 45 s at 72 °C. Final extension was performed for 5 min at 72 °C. DNA-extractions of *Helicobacter acinonychis* LMG 12684^{T} and *Helicobacter mustelae* LMG 8776 were used as positive controls.

### Analysis of amplified samples

PCR products were separated on 1 % agarose gels and stained with ethidium bromide.

DNA-extractions of *H. acinonychis* LMG 12684^{T} and *H. mustelae* LMG 8776 were used as positive controls.

In order to determine whether PCR products were derived from *Helicobacter*-like organisms, the desired DNA-bands were cut from the gels, diluted 1/2 in distilled water and sequenced using the *H33f* and *H1368r* 5'-Indocarbocyanin (Cy5) for "*Candidatus* Helicobacter bovis" and respectively *H61f* and *1492RPL* Indocarbocyanin (Cy5) labeled for "*Candidatus* Helicobacter suis". Partial sequences were screened for homologous sequences using the NCBI GENINFO ® BLAST Network service (http://www.ncbi.nlm.nih.gov/BLAST/) (Altshul et al., 1997).

### DNA cloning and sequence analysis for "Candidatus Helicobacter bovis"

PCR amplimers comprising the 16S rDNA-sequences derived from four different stomach samples (R2, R3, R5, R6) were each cloned into plasmid vector pGEM-T (Promega Biotech) according to the manufacturer's instructions and transformed into *Escherichia coli* JM109 using standard procedures. Plasmids were purified using the Easy Prep Plasmid Preparation Kit (Pharmacia Biotech). Sequences were determined by the T7-sequencing system (Pharmacia Biotech). Two primers flanking the multiple cloning sites (T7, SP6) as well as internal primers *H390f* and *H1053r* were used (Table 2). The sequence derived from the clone of the R2 sample (R2XA) was used as reference sequence. This sequence was has been asigned Genbank Accession No. AF127028. Sequence analysis was performed with the PCGene software (Intelligenetics)

PCR amplicons of three other gastric samples (R13, R27, R28) were sequenced without prior cloning (referred to below as direct sequence analysis).

### DNA cloning and sequencing for "Candidatus Helicobacter suis"

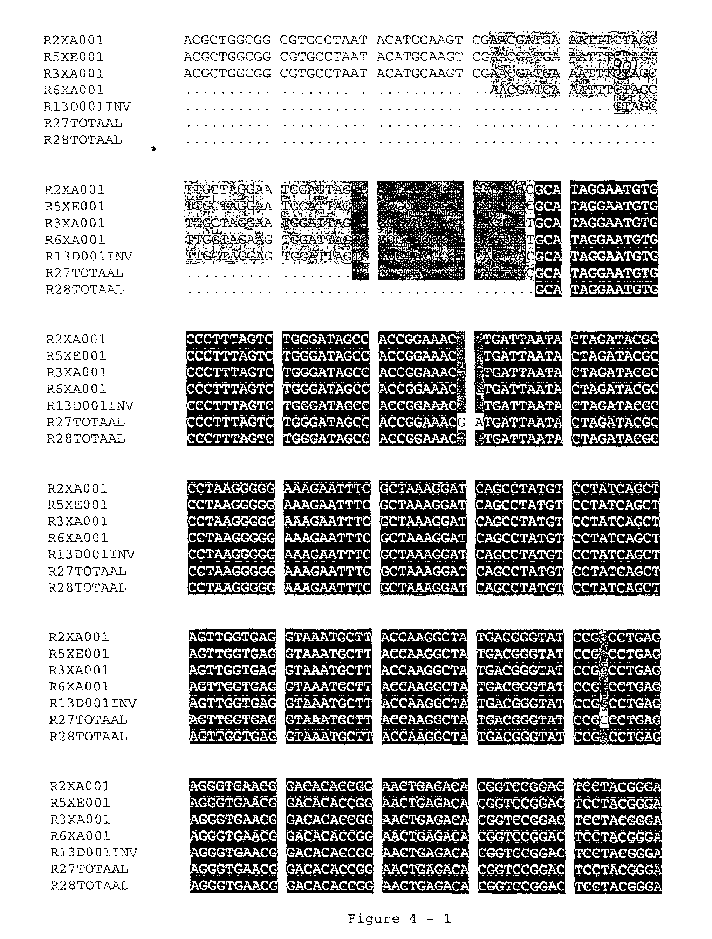

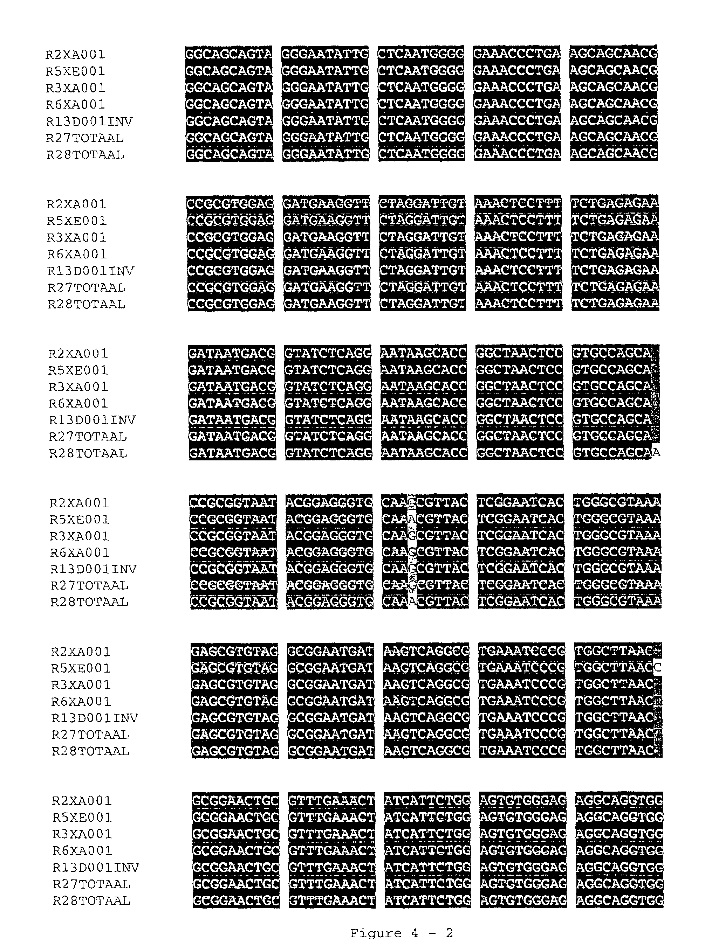

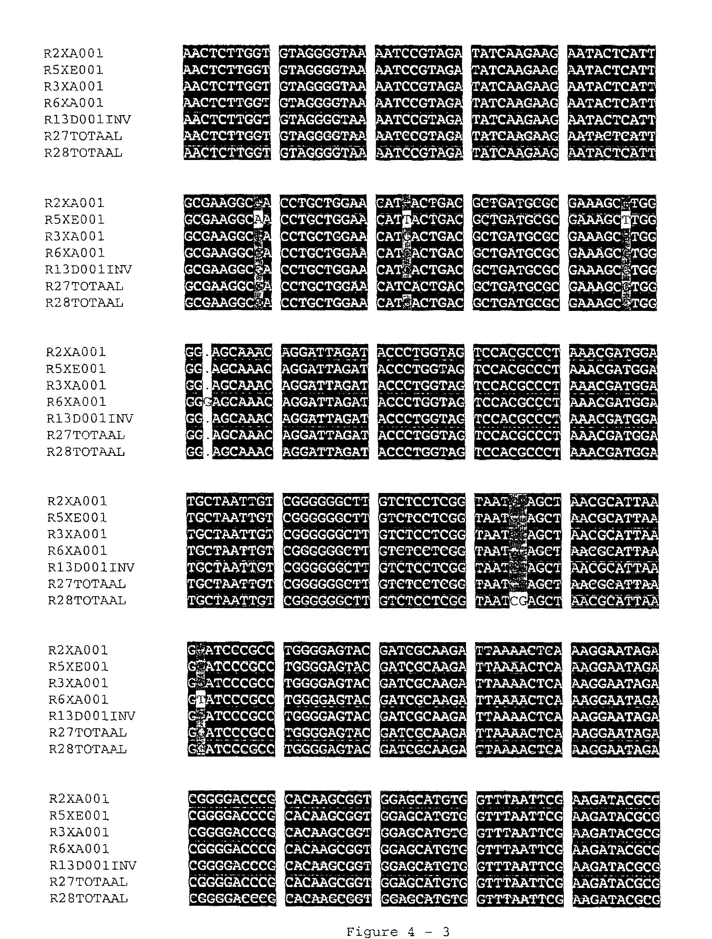

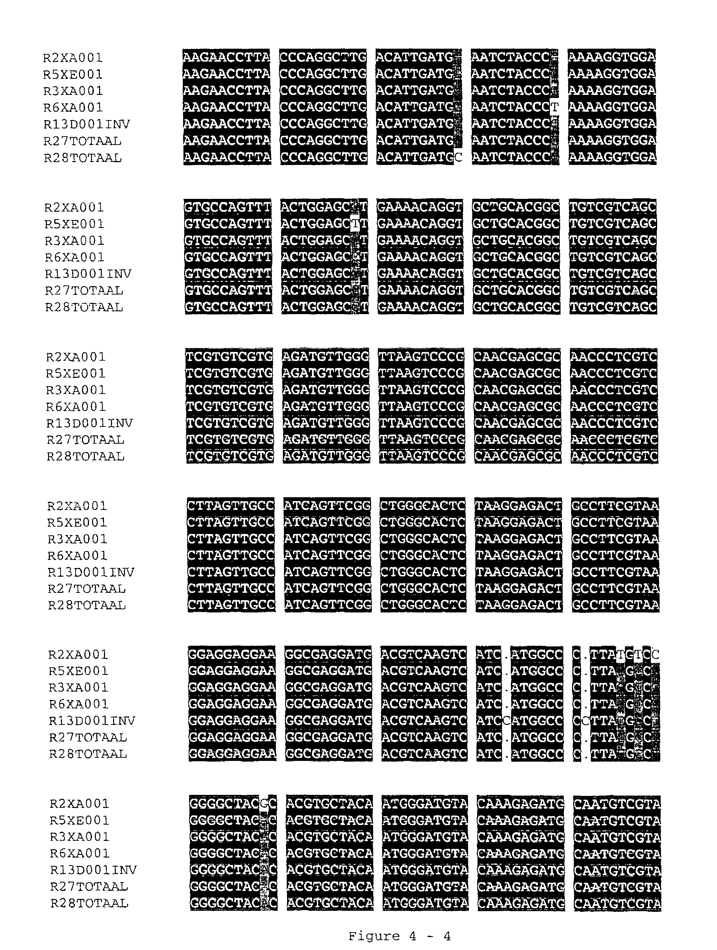

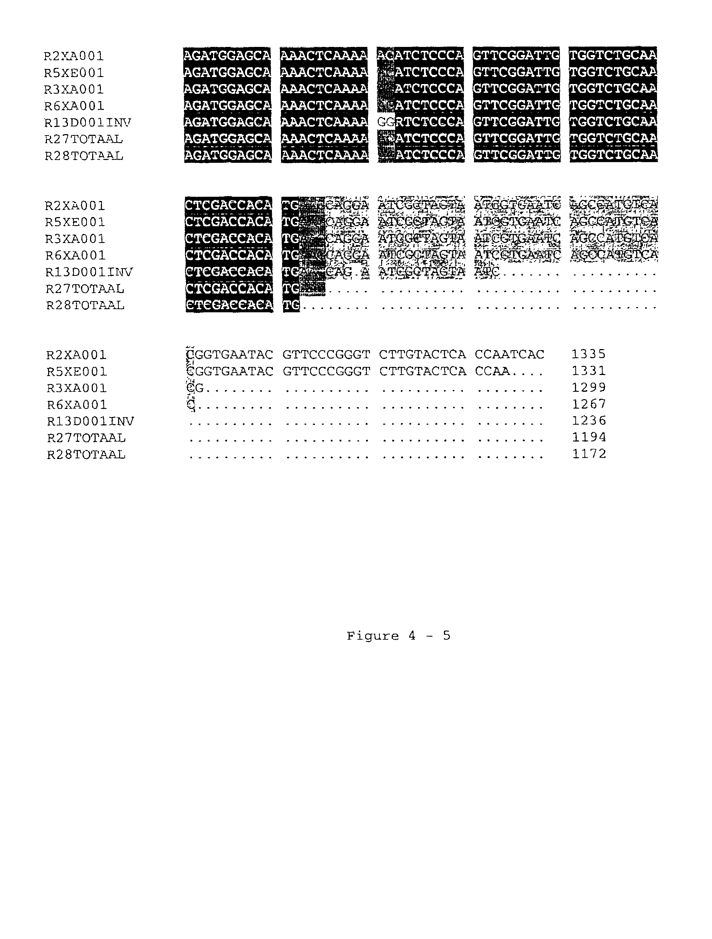

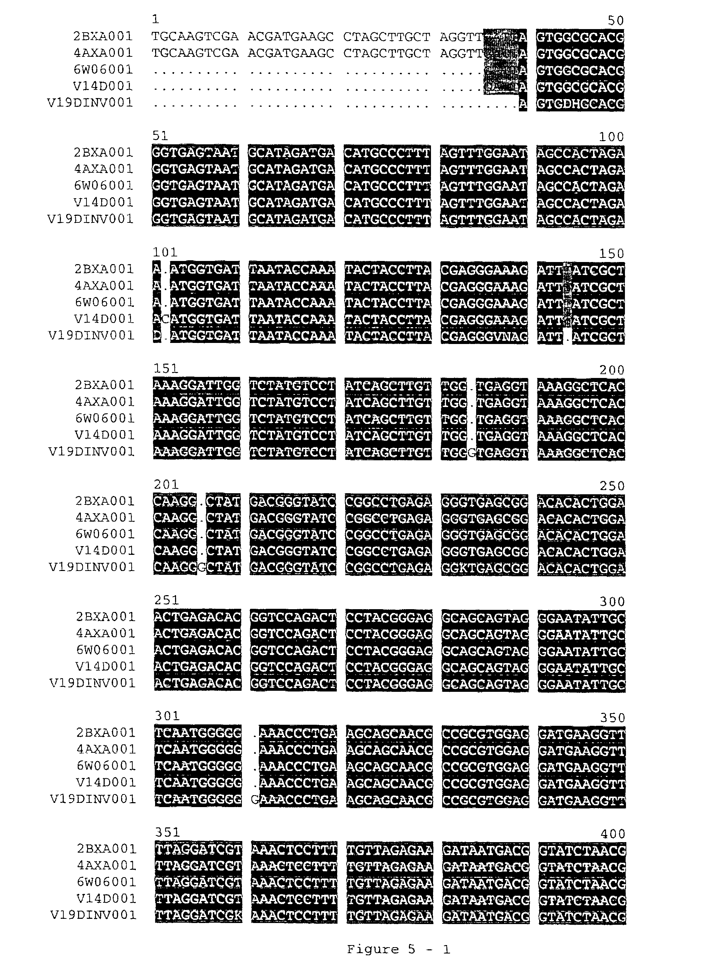

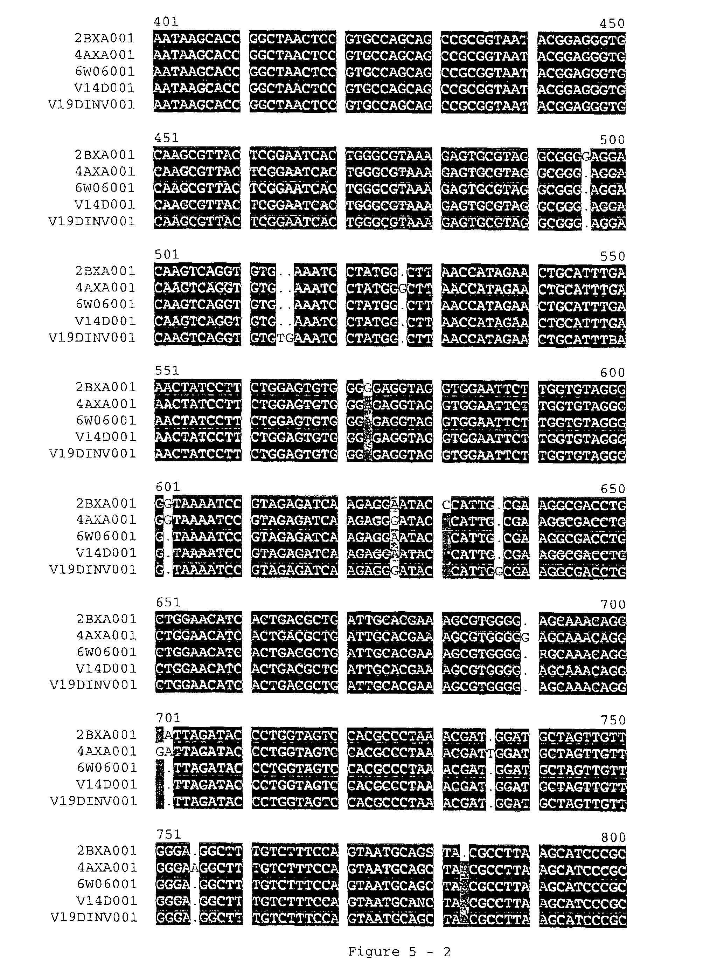

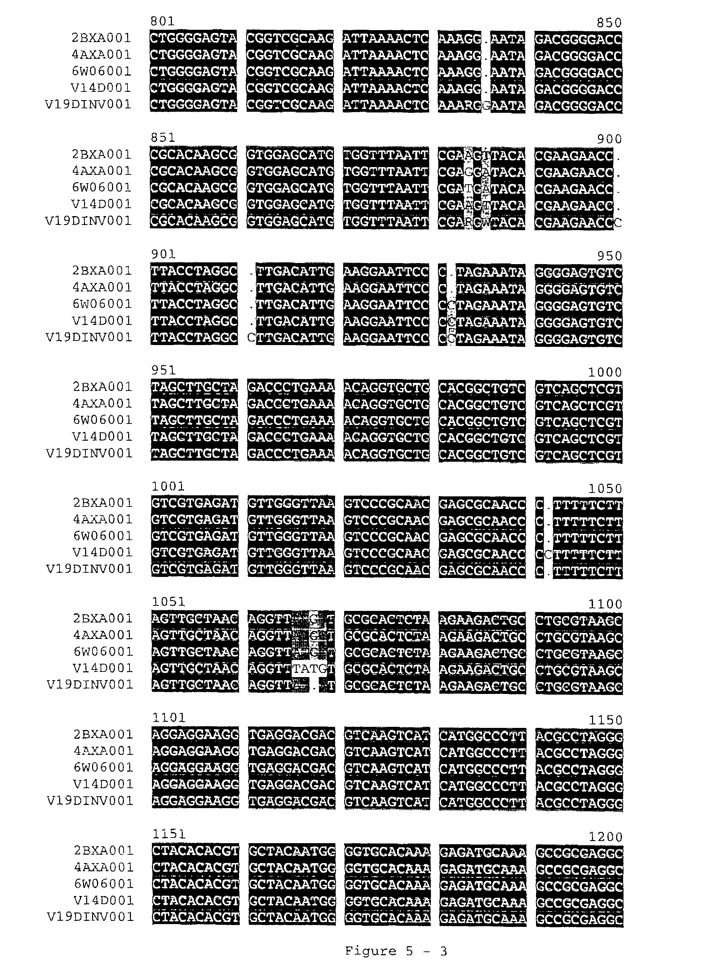

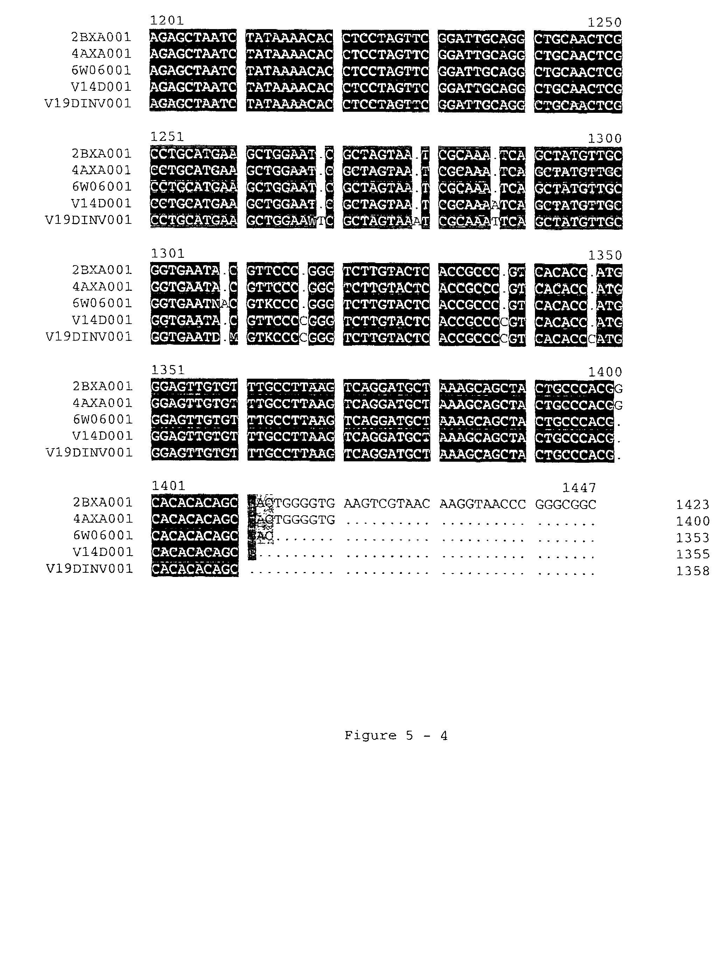

PCR amplimers comprising the 16S rDNA-sequences from 2 different stomachs (V2B, V4A) were cloned into plasmid vector pGEM-T (Promega Biotech) according to the manufacturer's instructions and transformed into *Escherichia coli* JM109 using standard procedures. Plasmids were purified using the Easy Prep Plasmid Prep Kit (Pharmacia Biotech). Sequences were determined by the T7-sequencing system (Pharmacia Biotech). Two primers flanking the multiple cloning sites *(T7* and *SP6)* as well as internal primers *H390f* and *H1053r* were used (Table 2). Sequence analysis was performed with the PCGene software (Intelligenetics). A reference sequence was determined based on its high length and was compared to the new sequencve and the other derived sequences, to check its integrity (see Figures 4 and 5). The reference sequence V2BXA was assigned Genbank Accession No. AF127028.

PCR amplicons of three other gastric samples (V5, V14, V19) were sequenced without prior cloning (referred to below as direct sequence analysis).

### Phylogenetic analysis for "Candidatus Helicobacter bovis"

Phylogenetic analysis was performed using the GeneCompar 2.0 software package (Applied Maths). Sequences of strains belonging to the epsilon subdivision (Table 3) were retrieved from the EMBL data library and were aligned with reference sequence R2XA. A similarity matrix was constructed from the aligned sequences and was corrected for multiple base changes by the method of Jukes & Cantor (1969). Unknown bases and gaps were not considered in the numerical analysis. A phylogenetic tree was constructed using the neighbour-joining method of Saitou & Nei (1987).

### Phylogenetic analysis for "Candidatus Helicobacter suis"

Phylogenetic analysis was performed using the GeneCompar 2.0 software package (Applied Maths). All five "*Candidatus* Helicobacter suis" sequences and the sequences of strains belonging to the same phylogenetic lineage (Table 3) were aligned. Using the neighbour-joining method, a phylogenetic tree and corresponding similarity matrix was constructed. Unknown bases and gaps were not considered in the numerical analysis

### "Candidatus Helicobacter bovis" specific PCR-assay

"*Candidatus* Helicobacter bovis" specific oligonucleotides *R574f and* R832r (Table 2), were selected from variable rDNA regions of the sequences determined by direct and indirect sequence analysis. These primers comprised a 259 bp 16S rDNA-fragment and were used to develop a specific PCR and an *in situ* hybridisation procedure. Within this fragment an internal "*Candidatus* Helicobacter bovis" specific probe R628f (Table 2) was selected for southern blot hybridisation purposes.

PCR reactions were performed in a volume of 50 µl containing 10 mM Tris HCl (pH 8.3), 50 mM KCl, 2.5 mM MgCl_{2,} 200 µM of each deoxynucleoside triphosphate, 1.5 U of AmpliTaq Gold, and 25 pmol of both forward and reverse primer. PCR amplification was performed under the following conditions: 9 min preincubation at 94 °C to activate AmpliTaq Gold, followed by 40 cycles of 30 s at 94 °C, 45 s at 60 °C and 90 s at 72 °C. Final extension was performed for 5 min at 72 °C. All gastric DNA-extracts were tested with this PCR. For positive controls, plasmid DNA was used from the cloned 16S rDNA fragments (R2XA). As a negative control a DNA-extract was used from an abomasum lacking of *Helicobacter-like* organisms.

Specificity of the "*Candidatus* Helicobacter bovis" specific oligonucleotides *R574f* and *R832r* was tested by PCR using DNA-extracts of 15 different *Helicobacter* strains and a *Wolinella succinogenes* strain (Table 1).

PCR products were separated on 2 % agarose gels, stained with ethidium bromide and transferred to Hybond N+ (Amersham) by electro-elution. Southern blot hybridisation was performed with the [γ^{32P}] ATP labelled probe *R628f* (Table 2) according to standard procedures (Amersham Pharmacia Biotech). In order to ensure the specificity of the probe hybridisation, blots were washed twice with 0.1 x SSC + 0.1 % SDS at 55°C.

### "Candidatus Helicobacter suis" specific diagnostic PCR-assay and Southern blot hybridisation

"*Candidatus* Helicobacter suis"- specific primers (V832f and *V1261r)* were selected from variable rDNA regions of the sequences determined by direct and indirect sequence analysis, comprising a ~0.4-Kb 16S rDNA-fragment. Within this fragment a "*Candidatus* Helicobacter suis"-specific probe *V1000f* (Table 2) was selected for hybridisation purposes. PCR reactions were performed in a volume of 50 pl containing 10 mM Tris HCl (pH 8.3), 50 mM KCl, 2.5 mM MgCl_{2,} 200 µM of each deoxynucleoside triphosphate, 1.5 U of AmpliTaq Gold (Perkin-Elmer), and 25 pmol of both forward and reverse primer (Eurogentec). PCR amplification was performed under the following conditions: 9 min preincubation at 94 °C to activate AmpliTaq Gold, followed by 40 cycles of 30 s at 94 °C, 45 s at 60 °C and 90 s at 72 °C. Final extension was performed for 5 min at 72 °C. As a positive control, plasmid DNA was used from the cloned 16S rDNA fragments (V2B, V4A). As a negative control DNA extracted from the stomach of a gnotobiotic piglet was used.

To test the specificity of the primers, PCR was also performed on DNA-extracts of 15 different *Helicobacter* species. (Table 1).

PCR products were separated on 2% agarose gels, stained with ethidium bromide and transferred to Hybond N+ (Amersham) by electro-blotting. Southern blot hybridisation was performed with the [γ^{32P}] ATP-labelled probe *V1000f* according to standard procedures (Amersham Pharmacia Biotech). In order to ensure the specificity of the probe hybridisation, blots were washed twice with 0.1 x SSC + 0.1 % SDS at 55°C.

### In situ hybridisation for "Candidatus Helicobacter bovis"

In order to make the link between the "*Candidatus* Helicobacter bovis" specific probe and the bacterial spiral cells observed in the tissue sections, an *in situ* hybridisation procedure was performed on the formalin fixed and paraffin embedded pyloric samples of each animal. A 259-base digoxigenin-labeled probe was synthezised using the "PCR Dig Probe Synthesis Kit" (Boehringer Mannheim) in combination with the "*Candidatus* Helicobacter bovis" specific primers *R574f* and *R832r* (Table 2). PCR conditions were identical to those described in the diagnostic PCR assay. The resulting PCR product was purified using the "High Pure PCR Product Purification Kit" (Boehringer Mannheim) following manufacturer's instructions.

To avoid RNA'se activity, all glassware was heated at 180°C for 3 hours. Further precautions included the use of RNA'se-free water, and the use of sterile disposable materials whenever possible. Sections of the paraffin-embedded tissues (4 µm thick) were mounted on RNA'se-free, APES-coated slides (Sigma-Aldrich) and fixed by heating for 1 hour at 60 °C. The sections were deparaffinized in xylene (2x5 min), rehydrated through graded ethanol, and washed twice in PBS for 5 min each. Sections were then treated with proteinase K (DAKO) for 15 min each at 37 °C in a humidified chamber. The enzyme was inactivated by treatment with 0.2 % glycine in PBS for 3 min. Sections were washed twice in PBS for 5 min each, dehydrated in graded ethanol and air dried. Tissues were circumlined with a DAKO Pen (DAKO) to avoid liquid spillage during further processing and to ensure an efficient sealing of the coverslip. For the hybridisation step, sections were covered with 5 to 15 pl solution, containing 5 ng/µl labeled probe in 50 % deionized formamide, 2x SCC, 10 % dextran sulfate, 0.25 µg/µl yeast t-RNA, 0.5 µg/µl heat denatured salmon sperm DNA, and lx Denhart's solution. Sections were covered with a piece of coverslip to avoid evaporation. To denature the probe, sections were heated for 10 min at 95 °C and chilled on ice for 10 min. Slides were then hybridised overnight at 37 °C in a humidified chamber. To remove the unbound probe, the coverslips were removed and the sections were washed in 2x SCC and 1x SCC at room temperature for 10 min each followed by two washes of 0.3x SCC at 40 °C for 10 min and at room temperature for 10 min, respectively.

All steps involving the immunological detection of the hybridised probe were performed at room temperature. The sections were treated first for 30 minutes in Buffer 1 (100mM Tris HCl, 150 mM NaCI, pH 7.5) containing 2 % normal goat serum and 0.3 % Triton X-100. An incubation step followed for 3 hours with diluted (1:30 in the same solution) anti-digoxigenin antibodies conjugated to horse-radish peroxidase (DAKO). Unbound antibodies were washed gently on a shaker with Buffer 1 followed by Buffer 2 (100 mM Tris HCl, 100 mM NaCI, 50 mM MgCl₂, pH 9.5) for 15 min each. To optimize the detection level, the "Tyramid Signal Amplification System" (NEN Life Science Products) was applied on each section, following manufacturer's instructions. The hybridised probe was then visualized, using H₂O₂ with diaminobenzidine as a chromogen (Fast DAB Tablet Set, Sigma-Aldrich). Thereafter the sections were counterstained with Mayer's hematoxylin and mounted.

### In situ hybridisation for "Candidatus Helicobacter suis"

To link the derived sequence to the corresponding organism, an *in situ* hybridisation procedure was performed on the formalin fixed and paraffin embedded pyloric samples of each animal. A ~0.4 Kb digoxigenin-labeled probe was synthezised using the "PCR Dig Probe Synthesis Kit" (Boehringer Mannheim) in combination with the "Candidatus Helicobacter suis"-specific primers *V832f* and *V1261r* (Table 2). The rest of the method was performed as mentioned above for "Candidatus Helicobacter bovis".

### Nucleotide sequence accession numbers

Accession numbers of the 16S rDNA gene sequences used for the phylogenetic analysis are listed in table 3.

The 16S rDNA nucleotide sequence of "*Candidatus* Helicobacter bovis" has been deposited in the Genbank database under accession number AF127027.

The 16S rDNA nucleotide sequence of "*Candidatus* Helicobacter suis" has been deposited in the Genbank database under accession number AF127028.

### Results

### Urease activity and immunohistochemical evaluation for the cattle samples

Urease activity was observed in all pyloric samples (7/7). In the fundic samples, urease activity was absent (0/7). Spiral immunostained organisms were observed in the pyloric samples of all animals. The highest concentration was seen in the most distal pyloric samples. They were mostly situated in the mucus layer and in the lumen of the proximal part of the gastric crypts where they formed small clusters. In some samples, coccoid organisms, were observed between the spiral bacteria, which also crossreacted with the *H. pylori* polyclonal antibodies. In the positive control only *Helicobacter pylori -* like bacteria were stained while in the negative controls no staining was observed.

### Urease activity and Immunohistochemical evaluation for the pig samples

Tightly coiled immunostained spiral organisms, morphologically similar to "*Gastrospirillum suis*" (Queiroz et al., 1990) were observed in all stomachs (5/5), which was consistent with the presence of urease activity (5/5). The gastrospirillum-like organisms were seen laying separately or in small clusters with a patchy distribution over the sample, and were found mostly in the superficial part of the gastric crypts. Some bacteria revealed bipolar immunostained flagellae. Immunostained coccoid-like organisms were also observed in the pyloric crypts. In the positive control, only *Helicobacter pylori-like* bacteria were stained while in the negative controls no labeling was observed.

### Transmission electronmicroscopy for "Candidatus Helicobacter bovis"

Large groups of multiple spiral bacteria were seen within the crypts of the gastric mucosa. There was no obvious cell association between the bacteria and the gastric cells, neither were there any intracellular bacterial inclusions. The bacteria were helical-shaped and had 1-3 complete spiral turns per cell with a wavelength of approximately 750 nm. Cells were 1 - 2.5 µm long and 0.3 µm wide. At least four flagelles were seen at one end. It was unclear whether these flagellae were uni- or bipolar, neither could the presence or absence of a flagella sheath be noted.

### Transmission electronmicroscopy for "Candidatus Helicobacter suis"

Within the gastric crypts of the antral region, longitudinal and transversal sections of spiral organisms could be seen. All bacteria had the same characteristic tightly coiled appearance, typical of *Helicobacters* with the gastrospirillum morphology. The length of cells varied from 2.5 to 3.5 pm and they were approximately 0.6 pm wide. Multiple complete spiral turns with a wavelength of ± 600 nm were seen in all longitudinal sections. As only few longitudinal sections of the bacteria were obtained, the number and implantation of the flagellae could not be studied although partial fragments were observed. The bacteria were not seen intracellularly nor was there any obvious cell association with the surrounding epithelial cells. The presence or absence of a flagella sheath could not be noted.

### Amplification, cloning and sequencing of Helicobacter-like 16S rDNA fragments from cattle samples

PCR amplification of the 16S rRNA gene using the *H33f and H1368r* primers, produced a fragment of the expected size range (± 1.3 Kbp) in all seven samples examined. Partial direct sequence analysis of four of these bands (R2, R3, R5, R6) and subsequent database comparison (BLAST) confirmed the PCR products to be *Helicobacter*-like 16S rDNA fragments. Four PCR products (R2, R3, R5, R6) were cloned followed by partial screening. In one clone a *Clostridium*-like 16S rDNA fragment was found. In all other clones *Helicobacter*-like fragments were inserted. The 16S rDNA sequences of four clones derived from different animals (R2XA, R3XA, R5XE, R6XA), were determined. Additional sequences of three other samples (R13, R27, R28) were characterized by direct sequence analysis using the primers *H33f, H1368r, H390f* and *H1053r.*

### Amplification, cloning and sequencing of Helicobacter-like 16S rDNA fragments from pig samples

Several combinations of PCR primers yielded sequences of the expected size. The length of these amplified fragments varied between 1.2 Kb *(H274f-1492RPL)* and 1.4 Kb *(H61f-1492RPL).* The latter primer combination was used to examine all samples. The 16S rDNA sequences of two different clones were determined (V2BXA, V4AXA). Additional sequences of 3 other samples (V5, V14, V19) were determined by direct sequence analysis.

### Sequences and phylogenetic analysis for cattle samples

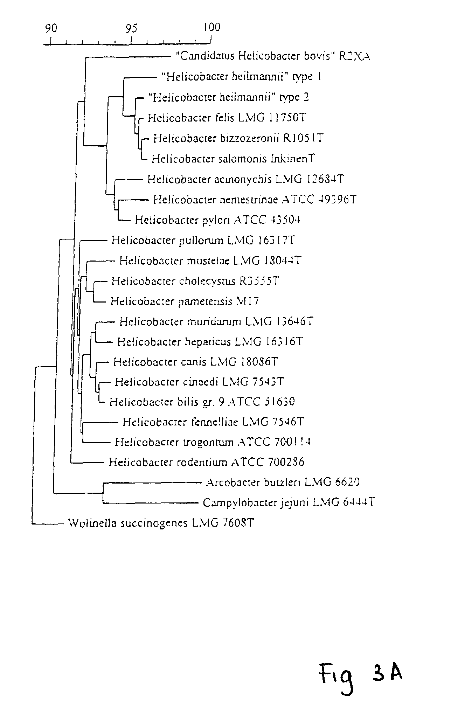

Sequence length varied from 1267 to 1335 basepairs. Pairwise comparisons between these 7 sequences revealed a sequence homology of more than 99 %. One reference sequence (R2XA) of 1335 bp (see Figure 1: SEQ ID NO 1) was selected for phylogenetic evaluation. A similarity matrix based on comparisons of 16S rRNA sequences of 23 strains representing all validly named *Helicobacter* species, "*Helicobacter heilmannii*" (type1, type2), *Campylobacter jejuni, Arcobacter cryaerophilus* and *Wollinella succinogenes* was calculated. By this analysis it was shown that the sequences of the bovine *Helicobacter*-like organisms form a distinct group within the genus *Helicobacter* with *Helicobacter bilis* as closest taxonomic relative (level of similarity 92.8 %). The reference sequence was clearly distinct from sequences belonging to other superfamily VI genera, as shown by a 85.6, 85.1 % and 89.7 % homology with *Campylobacter jejuni, Arcobacter butzleri* and *Wolinella succinogenes* respectively. A phylogenetic tree based on this analysis is shown in Fig. 3A.

### Sequences and phylogenetic analysis for pig samples

The 5 sequences that were determined had lengths varying from 1345 to 1421 basepairs. Pairwise comparisons between 1345 bp consensus fragments of these sequences, revealed a minimum homology of 97.7 %. One reference sequence of 1421 bp, obtained from PCR product 2BXA (see Figure 2: SEQ ID NO 2), was used for phylogenetic analysis. A similarity matrix was calculated based on comparisons of 16S rDNA sequences of all *Helicobacter* species, "*Helicobacter heilmannii*" type 1 and type 2, *Campylobacter jejuni, Arcobacter butzleri* and *Wollinella succinogenes* (Table 3). In this analysis, the sequence of the porcine gastrospirillum-like organism formed a distinct subgroup within the *Helicobacter* lineage together with other gastrospirilla: *Helicobacter felis,* H. *bizzozeronii, H. salomonis,* "*H. heilmannii*" type 1 and type 2. The sequence was highly similar to that of "*H. heilmannii*" type 1 (level of similarity 99.5 %). The similarity level of other gastrospirillum-like bacteria, H. *felis*, *H. bizzozeronii, H. salomonis* and H. *heilmanni* type 2 was 96.4 %, 96.5 %, 96.6 % and 96.8 % respectively. The reference sequence was clearly distinct from sequences belonging to other superfamily VI-genera, as shown by a 86.2 %, 84.7 % and 89.6 % homology with *Campylobacter jejuni, Arcobacter butzleri* and *Wolinella succinogenes* respectively.

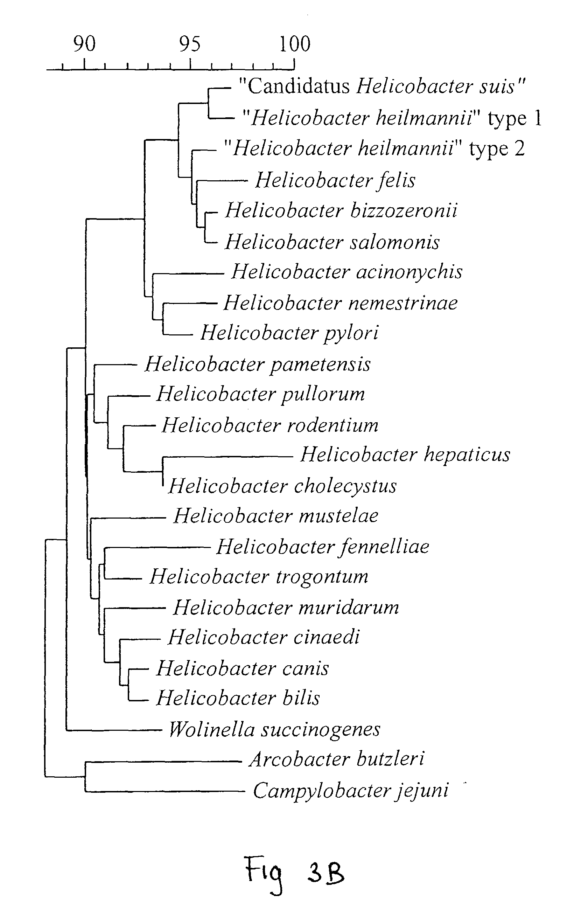

A phylogenetic tree based on this analysis is shown in Figure 3B.

### Diagnostic PCR-assay for "Candidatus Helicobacter bovis"

A 259 base fragment was produced for all seven stomach samples with primer pair *R574f-R832r.* All PCR products crosshybridised with the *R628f* probe after southern blot hybridisation. No amplification product was obtained using DNA preparations from any of the *Helicobacter* strains, nor from the bovine *Wolinella succinogenes* strain (Table 1). The positive control yielded a ~0.3 Kb product as expected. There was no DNA-amplification using the negative control material.

### "Candidatus Helicobacter suis"-specific PCR and Southern blot hybridisation

Amplification of *Helicobacter* DNA using the primers *V832f* and *V1261r* produced a 433-base fragment from all five stomach samples. All PCR products hybridised with the *V1000f* probe after Southern blot hybridisation. No amplification product was obtained using DNA preparations from any of the *Helicobacter* strains including *H. felis*, *H. bizzozeronii* and *H. salomonis* (Table 1), nor from the negative control. PCR with the cloned reference material (2BXA) yielded a ~0.4 Kb product as expected.

### In situ hybridisation for "Candidatus Helicobacter bovis"

*In situ* hybridisation of the bovine *Helicobacter-like* bacteria with the *"Candidatus* Helicobacter bovis"- specific probe was seen in sections from all (7/7) stomachs. These bacteria were observed as darkbrown spiral organisms, organised in small clusters, situated in the gastric crypts of the pyloric part of the abomasal stomach. Not all spiral bacteria were stained. Sometimes a faint background, seen as fine stained strings, was observed in the surrounding cells. This background staining was also observed in the *H. pylori*-infected mouse stomach which was used as a negative control. The *H*. *pylori* cells in this control though did not hybridise with the "*Candidatus Helicobacter bovis*"- specific probe.

### In situ hybridisation for "Candidatus Helicobacter suis"

*In situ* hybridisation of "*Gastrospirillum suis*"-like bacteria with the "*Candidatus* Helicobacter suis"- specific probe was seen in sections from all (5/5) stomachs. Bacteria were observed as darkbrown spiral organisms in the superficial mucus layer and the gastric crypts. In some cases, helical organisms located deeply in the crypts, were weakly labeled or were negative. Sometimes a faint background, seen as fine stained strings, was observed in the surrounding cells. This background staining was also observed in the *H.* pylori-infected mouse stomach which was used as a negative control. The *H. pylori* cells in this control though did not hybridise with the *"Candidatus* Helicobacter suis"- specific probe.

### REFERENCES

**Rappin, J. (1881).** Contribution à l'étude de bactéries de la bouche à l'état normal. Ph.D. thesis. Collège de France, Nantes.

**Bizzozero, G. (1893).** Ueber die schlauchformigen Drüsen des Magendarmkanals und die Beziehungen ihres Epithels zu dem Oberflächenepithel der Schleimhaut. *Arch Mikrosk Anat* **42**, 82.

**Salomon, H. (1896).** Ueber das Spirillum des Säugentiermagens und sein Verhalten zu den Belegzellen. *Zentralbl Bakt* **19***,* 433.

**Marshall, B.J. & Warren, J.R. (1984)**. Unidentified curved bacilli in the stomach of patients with gastritis and peptic ulceration. *Lancet* **1(8390)**, 1311-1315.

**Cover, T.L. & Blaser M.J. (1992)**. *Helicobacter pylori* and gastroduodenal disease. *Annu Rev Med* **43**, 135-145.

**Blaser, M.J., Perez-Perez, G.I., Lindenbaum, J., Schneidman, D., Van Deventer, G., Marin-Sorensen, M. & Weinstein, W.M. (1991).** Association of infection due to *Helicobacter pylori* with specific upper gastrointestinal pathology. *Rev Infect Dis* **13 Suppl 8**, S704-708.

**Parsonnet, J., Friedman, G.D., Vandersteen, D.P., Chang, Y., Vogelman, J.H., Orentreich, N. & Sibley, R.K. (1991)**. *Helicobacter pylori* infection and the risk of gastric carcinoma [see comments]. *N Engl J Med* **325,** 1127-1131.

**On, S.L. (1996)**. Identification methods for campylobacters, *Helicobacters,* and related organisms. *Clin Microbiol Rev* **9**, 405-422.

**Franklin, C.L., Beckwith, C.S., Livingston, R.S., Riley, L.K., Gibson, S.V., Besch-Williford, C.L. & Hook, R.R., Jr. (1996)**. Isolation of a novel *Helicobacter* species, *Helicobacter cholecystus* sp. nov., from the gallbladders of Syrian hamsters with cholangiofibrosis and centrilobular pancreatitis. *J Clin Microbiol* **34**, 2952-2958.

**Mendes, E.N., Queiroz, D.M., Dewhirst, F.E., Paster, B.J., Moura, S.B. & Fox, J.G. (1996).** *Helicobacter trogontum* sp. nov., isolated from the rat intestine. *Int J Syst Bacterial* **46**, 916-921.

**Jalava, K., Kaartinen, M., Utriainen, M., Happonen, I. & Hanninen, M.L. (1997).** *Helicobacter salomonis* sp. nov., a canine gastric *Helicobacter* sp. related to *Helicobacter felis* and *Helicobacter bizzozeronii. hit J Syst Bacteriol* **47**, 975-982.

**Trivett-Moore, N.L., Rawlinson, W.D., Yuen, M. & Gilbert, G.L. (1997).** *Helicobacter westmeadii* sp. nov., a new species isolated from blood cultures of two AIDS patients. *J Clin Microbiol* **35,** 1144-1150.

**Shen, Z., Fox, J.G., Dewhirst, F.E., Paster, B.J., Foltz, C.J., Yan, L., Shames, B. & Perry, L**. (1997). *Helicobacter rodentium* sp. nov., an urease-negative *Helicobacter* species isolated from laboratory mice. *Int J Syst Bacteriol* **47**, 627-634.

**Vandamme, P., Falsen, E., Rossau, R., Hoste, B., Segers, P., Tytgat, R. & De Ley, J. (1991).** Revision of *Campylobacter, Helicobacter,* and *Wolinella* taxonomy: emendation of generic descriptions and proposal of *Arcobacter* gen. nov. *Int J Syst Bacteriol* **41**, 88-103.

**Dent, J.C., McNulty, C.A.M., Uff, J.C., Wilkinson, S.P. & Gear, M.W.L. (1987).** Spiral organisms in the gastric antrum [Letter]. *Lancet* **2 (8550)**, 96.

**Günther, H. & Schulze, F. (1992).** Histologische Untersuchungen zum Vorkommen von *Campylobacter-ahnlich* geformten Keimen im Labmagen von Kälbern. *Zentralbl Veterinarmed B* **39,** 737-745.

**Haringsma, P.C. & Mouwen, J.M. (1992).** Mogelijke betekenis van spirilvormige bacteriën bij het ontstaan van lebmaagzweren bij het volwassen rund. *Tijdschr Diergeneeskd* **117**, 485-487.

**Seidel, K.E., Kampschulte, J., Lehn, N. & Bauer, J. (1996). *Helicobacter*** *pylori:* Antikörper in Seren von Schweinen und Kälbern. *Berl Munch Tierarztl Wschr* 109, 434-439.

**Korhonen, H., Syväoja, E.-L., Ahola-Lutila, H., Sivelä, S., Kopola, S., Husu,** J. & Kosunen, T.U. (1995). Bactericidal effect of bovine normal and immune serum, colostrum and milk against *Helicobacter pylori. J Appl Bacteriol* **78**, 655-662.

**Jelinski, M.D., Ribble, C.S., Chirino-Trejo, M., Clark, E.G. & Janzen, E.D. (1995).** The relationship between the presence of *Helicobacter pylori, Clostridium perfringens* type A, *Campylobacter* spp., or fungi and fatal abomasal ulcers in unweaned beef calves. *Can Vet J* **36**, 379-382.

**Braun, U., Anliker, H., Corboz, L. & Ossent, P. (1997)**. Untersuchungen über das Vorkommen von spiralformigen Bakterien im Labmagen das Rindes. *Schweiz Arch Tierheilkd* **139**, 507-516.

**Al-Mashat, R.R. & Taylor, D.J. (1980).** *Campylobacter* spp. in enteric lesions in cattle. *Vet Rec* **107,** 31-34.

**Mills, K.W., Johnson, J.L., Jensen, R.L., Woodard, L.F. & Doster, A.F. (1990).** Laboratory findings associated with abomasal ulcers/tympany in range calves. *J Vet Diagn Invest* **2**, 208-212.

**Boom, R., Sol, C.J.A., Salimans, M.M.M., Jansen, C.L., Wertheim van Dillen, P.M.E. & van der Noorda J. (1990).** Rapid and simple method for purification of nucleic acids. *J Clin Microbiol* **28**, 495-503.

**Altschul, S.F., Madden, T.L., Schäffer, A.A., Zheng Zhang, J.Z., Miller W. & Lipman D.J. (1997).** "Gapped BLAST and PSI-BLAST: a new generation of protein database search programs". *Nucleic Acids Res* **25,** 3389-3402.

**McNulty, C.A., Dent, J.C., Curry, A., Uff, J.S., Ford, G.A., Gear, M.W. & Wilkinson, S.P. (1989).** New spiral bacterium in gastric mucosa [see comments]. J *Clin Pathol* **42**, 585-591.

**Solnick, J.V., O'Rourke, J., Lee, A., Paster, B.J., Dewhirst F.E. & Tompkins L.S. (1993).** An uncultured gastric spiral organism is a newly identified *Helicobacter* in humans. *J Infect Dis* **168**, 379-385.

**Andersen, L.P., Norgaard, A., Holck, S., Blom, J. & Elsborg, L. (1996).** Isolation of a *"Helicobacter heilmanii"*-like organism from the human stomach [letter]. *Fur J Clin Microbiol Infect Dis* **15**, 95-96.

**Jukes, T. & Cantor, C. (1969)**. Evolution of protein molecules. In *Mammalian protein metabolism,* vol 3. Edited by H. Munro. New York: Academic Press.

**Saitou, N. & Nei, M. (1987).** The neighbor-joining method: a new method for reconstructing phylogenetic trees. *Mol Biol Evol* **4**, 406-425.

**Wolin, M.J. & Jacobs, N.J. (1961).** Cytochrome-producing anaerobic vibrio, *Vibrio succinogenes,* sp. nov. *J Bacteriol* **81**, 911-917.

**Paster, B.J. & Canale-Parola, E. (1985)**. *Treponema saccharophilum* sp. nov., a large pectinolytic spirochete from the bovine rumen. *Appl Environ Microbiol* **50**, 212-219.

**Stanton, T.B. & Canale-Parola, E. (1980).** *Treponema bryantii sp.nov.,* a rumen spirochete that interacts with cellulolytic bacteria. *Archiv Microbiol* **127**, 145-156.

**Lee, A., Hazell, S.L., O'Rourke, J. & Kouprach, S. (1988).** Isolation of a spiral-shaped bacterium from the cat stomach. *Infect Immun* **56**, 2843-2850.

**O'Rourke, J., Solnick, J., Lee, A. & Tompkins, L. (1992).** *Helicobacter heilmanii* (previously *Gastrospirillum),* a new species of *Helicobacter* in humans and animals [abstract]. *J Med Science* **101**, 31.

**Hänninen, M.-L., Happonen, I., Saari, S. & Jalava, K. (1996).** Culture and characteristics of *Helicobacter bizzozeronii,* a new canine gastric *Helicobacter sp* [published erratum appears in Int J Syst Bacteriol 1996 Jul;46(3):839 ]. *Int J Syst Bacteriol* **46**, 160-166.

**Queiroz, D.M.M., Rocha, G.A., Mendes, E.N., Lage, A.P., Carvalho, A.C.T. & Barbosa, A.J.A. (1990)**. A spiral microorganism in the stomach of pigs. *Vet Microbiol* **24**, 199-204.

**Mendes, E.N., Queiroz, D.M.M., Rocha, G.A., Moura, S.B., Leite, V.H.R. & Fonseca, M.E.F. (1990).** Ultrastructure of a spiral micro-organism from pig gastric mucosa *("Gastrospirillum suis"). J Med Microbiol* **33**, 61-66.

**Mendes, E.N., Queiroz, D.M.M., Rocha, G.A., Nogueira, A.M., Carvalho, A.C., Lage, A.P. & Barbosa, A.J. (1991).** Histopathological study of porcine gastric mucosa with and without a spiral bacterium ("*Gastrospirillum suis*"). *J Med Microbiol* **35**, 345-348.

**Barbosa, A.J., Silva, J.C., Nogueira, A.M., Paulino, Junior, E. & Miranda, C.R. (1995).** Higher incidence of *Gastrospirillum* sp. in swine with gastric ulcer of the pars oesophagea. *Vet Pathol* **32**, 134-139.

**Queiroz, D.M.M., Rocha, G.A., Mendes, E.N., De Moura, S.B., De Oliveira, A.M. & Miranda, D. (1996).** Association between *Helicobacter* and gastric ulcer disease of the pars esophagea in swine [see comments]. *Gastroenterology* **111**, 19-27.

**Moura, S.B., Queiroz, D.M., Mendes, E.N., Nogueira, A.M. & Rocha, G.A. (1993).** The inflammatory response of the gastric mucosa of mice experimentally infected with *"Gastrospirillum suis". J Med Microbiol* **39**, 64-68.

**Mendes, E.N., Queiroz, D.M.M., Coimbra, R.S., Moura, S.B., Barbosa, A.J. & Rocha G.A. (1996).** Experimental infection of Wistar rats with *"Gastrospirillum suis". J Med Microbiol* **44**, 105-109.

**Seymour, C., Lewis, R.G., Kim, M., Gagnon, D.F., Fox, J.G., Dewhirst, F.E. & Paster, B.J. (1994).** Isolation of *Helicobacter* strains from wild bird and swine feces. *Appl Env Microbiol* **60**, 1025-1028.

**Dewhirst, F.E., Seymour, C., Fraser, G.J., Paster, B.J. & Fox, J.G. (1994).** Phylogeny of *Helicobacter* isolates from bird and swine feces and description of *Helicobacter pametensis* sp.nov. *Int J Syst Bacteriol* **44**, 553-560.

**On, S.L.W., Bloch, B., Holmes, B., Hoste, B. & Vandamme P. (1995).** *Campylobacter hyointestinalis* subsp. *Lawsonii* subsp. nov. isolated from the porcine stomach, and an emended description of *Campylobacter hyointestinalis*, *Int J Syst Bacteriol 45,* 767-774.

**Suarez, D.L., Wesley, I.V. & Larson, D.J. (1997).** Detection of *Arcobacter* species in gastric samples from swine. *Vet Microbiol* **57**, 325-336

**Eaton, K.A., Radin, M.J., Kramer, L., Wack, R., Sherdino, R., Krakowka, S., Fox, J.G. & Morgan, D.R. (1993).** Epizootic gastritis associated with gastric spiral bacilli in cheetas *(Acinonyx jubatus*). *Vet Pathol* **30**, 55-63.

**Jakob, W., Stolte, M., Valentin, A. & Schroder, H.D. (1997).** Demonstration of *Helicobacter pylori-like* organisms in the gastric mucosa of captive exotic carnivores. *J Comp Pathol* **116**, 21-33.

**Stackebrandt, E. & Goebel, B.M. (1994)**. Taxonomic note: a place for DNA-DNA reassociation and 16S rRNA sequence analysis in the present species definition in bacteriology. *Int J Sys Bacter* **44**, 846-849.

**Murray, R.G., Stackebrandt, E. (1995)** Taxonomic note: implementation of the provisional status Candidatus for incompletely described procaryotes. *Int J Syst Bacteriol* 45, 186-187.

**Murray, R.G.E. & Schleifer, K.H. (1994)**. *Taxonomic notes: a proposal for recording* the properties of putative taxa of procaryotes. *Int J Syst Bacteriol* 44, 174-176.

**Stuyver L, Rossau R, Wyseur A, et al. (1993)** *Typing of hepatitis C virus isolates and characterization of new subtypes using a line probe assay. J. Gen. Virol. 74 : 1093-1102.*

**Sambrook J, Fritsch E, Maniatis T (1989)** *Molecular cloning: a laboratory manual. Cold Spring Harbor Laboratory Press, Cold Spring Harbor, NY.*

**Matsukura M, Shinozuka K, Zon G, Mitsuya H, Reitz M, Cohen J, Broder S (1987)** Phosphorothioate analogs of oligodeoxynucleotides : inhibitors of replication and cytopathic effects of human immunodeficiency virus. Proc. Natl. Acad. Sci. USA 84(21):7706-10.

**Miller P, Yano J, Yano E, Carroll C, Jayaram K, Ts'o P (1979)** *Nonionic nucleic acid analogues. Synthesis and characterization of dideoxyribonucleoside methylphosphonates. Biochemistry 18(23):513-43.*

**Nielsen P, Egholm M, Berg R, Buchardt O (1991)** *Sequence-selective recognition of DNA by strand displacement with a thymine-substituted polyamide. Science 254(5037):1497-500.*

**Nielsen P, Egholm M, Berg R, Buchardt O (1993)** *Sequence specific inhibition of DNA restriction enzyme cleavage by PNA. Nucleic-Acids-Res. 21(2):197-200.*

**Asseline U, Delarue M, Lancelot G, Toulme F, Thuong N (1984)** *Nucleic acid-binding molecules with high affinity and base sequence specificity : intercalating agents covalently linked to oligodeoxynucleotides. Proc. Natl. Acad. Sci. USA 81(11):3297-301.*

**Table 1:**

| Bacterial strains used for the evaluation of the *"Candidatus* Helicobacter bovis" specific PCR | | |
|---|---|---|
| **Taxon** | **Source** | **Collection N° or Strain** |
| *Helicobacter acinonychis* | Cheetah gastric mucosa | LMG 12684^{T} |
| *Helicobacter cinaedi* | Human feces | LMG 7543 ^{T} |
| *Helicobacter* sp. strain CLO-3 | Human rectal swab | LMG 7792 |
| *Helicobacter fennelliae* | Human feces | LMG 11759 |
| *Helicobacter pametensis* | Tern feces | LMG 12678 ^{T} |
| *Helicobacter* sp. strain Bird B | Bird feces | LMG 12679 |
| *Helicobacter* sp. strain Bird C | Bird feces | LMG 13642 |
| *Helicobacter hepaticus* | Murine liver | LMG 16316^{T} |
| *Helicobacter pullorum* | Chicken lower bowel | LMG 16318 |
| *Helicobacter mustelae* | Ferret gastric mucosa | LMG 18044 ^{T} |
| *Helicobacter canis* | Canine feces | LMG 18086 ^{T} |
| *Helicobacter muridarum* | Murine intestinal mucosa | LMG 14378 ^{T} |
| *Helicobacter bizzozeronii* | Canine gastric mucosa | Strain 12A |
| *Helicobacter salonionis* | Canine gastric mucosa | CCUG 37845 T |
| *Helicobacter felis* | Feline gastric mucosa | CCUG 28539 T |

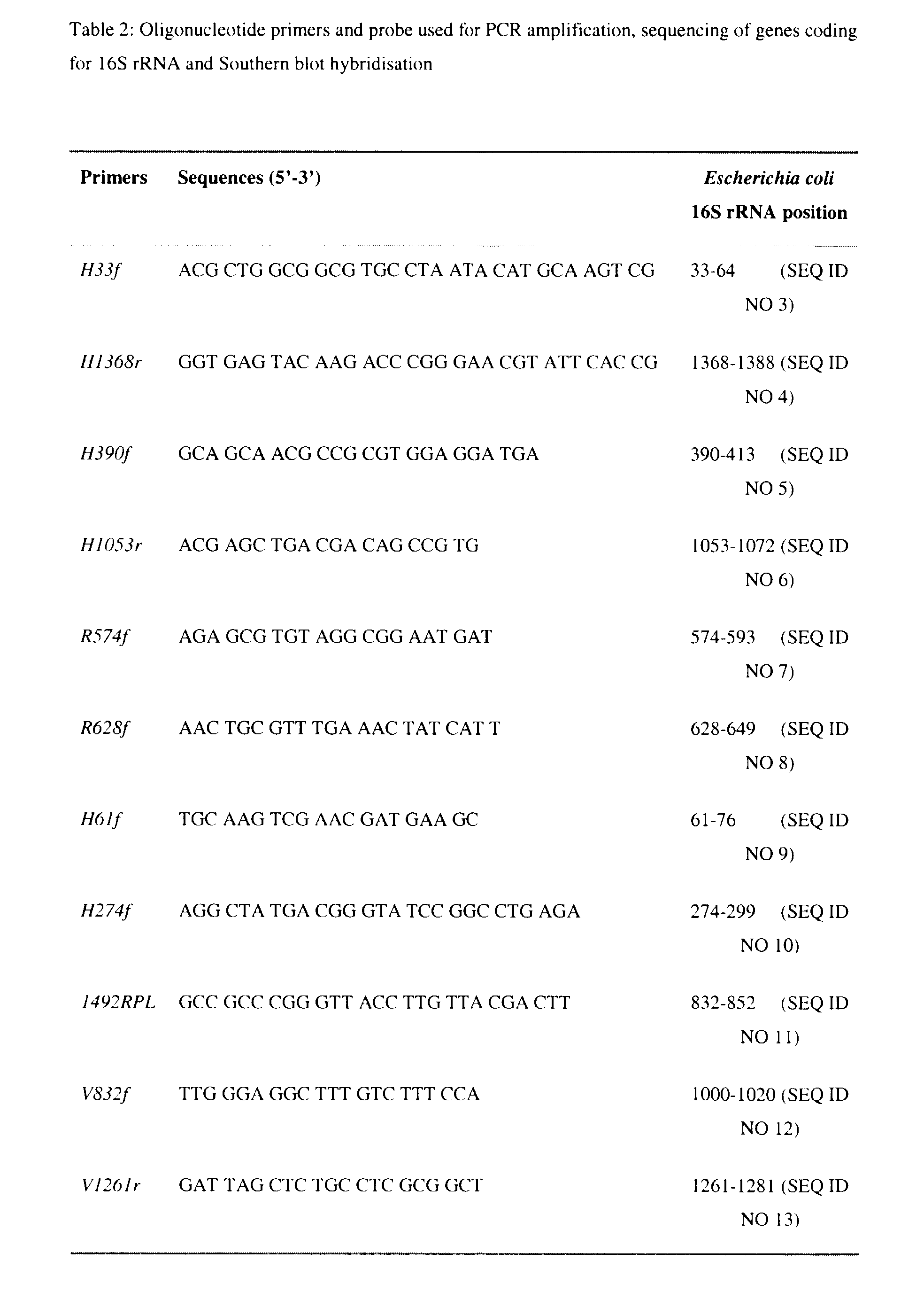

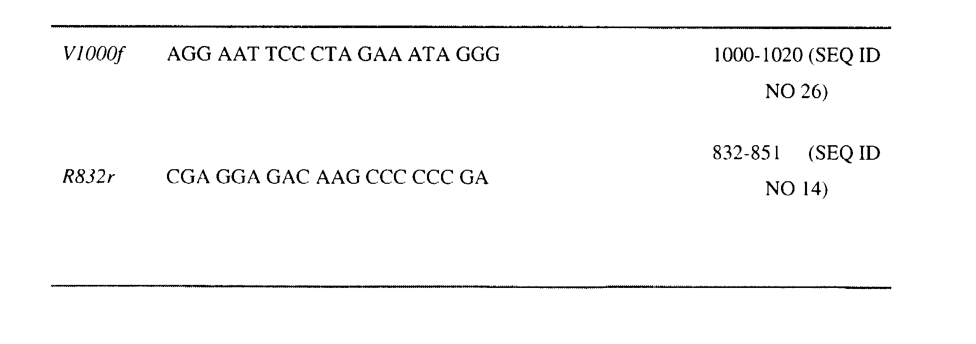

**Table 3:**

| Sources and accession numbers of strains used for phylogenetic analysis. | | |
|---|---|---|
| **Taxon** | **Source** | **Genbank Accession N°** |
| *"Gastrospirillum hominis"* type 1 | Human gastric mucosa | L10079 |
| *"Gastrospirillum hominis"* type 2 | Human gastric mucosa | L10080 |
| *Helicobacter acinonychis* | Cheetahgastric mucosa | M88148 |
| *Helicobacter bilis* | Murine liver | U18766 |
| *Helicobacter bizzozeronii* | Canine gastric mucosa | Y09404 |
| *Helicobacter canis* | Canine feces | L13464 |
| *Helicobacter cholecystus* | Murine liver | U46129 |
| *Helicobacter cinaedi* | Human feces | M88150 |
| *Helicobacter felis* | Feline gastric mucosa | M57398 |
| *Helicobacter fennelliae* | Human feces | M88154 |
| *Helicobacter hepaticus* | Murine liver | U07574 |
| *Helicobacter muridarum* | Murine intestinal mucosa | M80205 |
| *Helicobacter mustelae* | Ferret gastric mucosa | M35048 |
| *Helicobacter nemestrinae* | Macaque gastric mucosa | X67854 |
| *Helicobacter pametensis* | Swine feces | M88155 |
| *Helicobacter pullorum* | Broiler chicken cecum | L36141 |
| *Helicobacter pylori* | Human gastric mucosa | M88157 |
| *Helicobacter salomonis* | Canine gastric mucosa | Y09405 |
| *Helicobacter trogontum* | Rat colon mucosa | U65103 |
| *Helicobacter rodentium* | Murine intestinal mucosa | U96297 |
| *Arcobacter butzleri* | Human | L14626 |
| *Campylobacter jejuni* | Human feces | L14630 |
| *Wolinella succinogenes* | Cattle abomasal mucosa | M88159 |

## Claims

1. An isolated 16S rDNA *Helicobacter* polynucleic acid sequence selected from any of the following
(a) a sequence represented in any of SEQ ID NO 1 to 2, or, the 16S rRNA sequence encoded thereby,
(b) a sequence which hybridizes under stringent conditions to any of the sequences defined in (a).

2. An isolated polynucleid acid sequence according to claim 1 represented by any of SEQ ID NO 1 to 2 or 15 to 24.

3. An isolated polynucleic acid sequence according to claim 1 which is more than 92.8%, preferably more than 93.5%, more preferably more than 95% and most preferably more than 97.5% homologous to SEQ ID NO 1.

4. A part of an isolated polynucleic acid according to any of claims 1 to 3, wherein said part is unique to the polynucleic acid sequence it is derived from.

5. A probe which specifically hybridizes to a polynucleic acid sequence according to any of claims 1 to 4.

6. A primer which specifically amplifies a polynucleic acid sequence according to any of claims 1 to 4.

7. A method for detection and/or typing of *Helicobacter* strains present in a biological sample comprising hybridizing the 16S rRNA gene target region polynucleotides of said *Helicobacter* strains present in said biological sample with at least one probe according to claim 5.

8. A method for detection and/or typing of *Helicobacter* strains present in a biological sample comprising specifically amplifying the 16S rRNA gene target region polynucleotides of said *Helicobacter* strains present in said biological sample with at least one primer according to claim 6.

9. A method for detection and/or typing of *Helicobacter* strains present in a biological sample comprising specifically hybridizing or specifically amplifying the 16S rRNA gene target region polynucleotides of said *Helicobacter* strains present in said biological sample with at least one sequence which is more than 96.6% homologous to SEQ ID NO 2, or a sequence specific primer or a sequence specific probe derived thereof.

10. A diagnostic kit for detection and/or typing of *Helicobacter* strains comprising:
- at least one probe according to claim 5 or 9, and/or,
- at least one primer according to claim 6 or 9.

11. A medicament comprising a polynucleic acid sequence according to any of claims 1 to 6.

12. A polynucleic acid sequence according to any of claims 1 to 6 for use as a medicament.
